# EUROPEAN PATENT APPLICATION

(11) **EP 3 510 861 A2**
(43) Date of publication of application: **17.07.2019**
(21) Application number: 18215595.2
(22) Date of filing: 21.12.2018
(51) Int. Cl.: A01K 67/027

(54) **HYPOALLERGENIC FOOD AND MEDICAL PRODUCTS FROM GENOME EDITED LIVESTOCK**

(30) Priority: 22.12.2017 IT 201700149385
(71) Applicant: Avantea SRL, 26100 Cremona (IT)
(72) Inventor: PEROTA, Andrea, 26100 Cremona (IT); GALLI, Cesare, 26100 Cremona (IT); LAGUTINA, Irina, 26100 Cremona (IT); DUCHI, Roberto, 26100 Cremona (IT); LAZZARI, Giovanna, 26100 Cremona (IT); COZZI, Emanuele, 35129 Padova (IT); CONCHON, Sophie, 44300 Nantes (FR); SOULILLOU, Jean-Paul, 44100 Nantes (FR)
(74) Representative: Zaccaro, Elisabetta

(57) **Abstract**

The present invention concerns the generation of low antigenic livestock as source of animal derivatives for prevention and treatment in humans of allergic syndromes and chronic degenerative and autoimmune diseases caused by consumption of foodstuffs of animal origin or by receiving medical treatments containing livestock derived products.

## Description

### FIELD OF THE INVENTION

The present invention concerns the field of low antigenic livestock animals as source of safer and healthier animal derived products for prevention and treatment of allergic syndromes and long term chronic degenerative diseases caused by their consumption as well as the use of animal derived products used for medical treatments.

### STATE OF THE ART

Consumption of red meats and innards of non-primate mammals (for example bovine, pig and sheep) could provoke in some patients a 3-6 hours delayed allergy reaction characterized by anaphylaxis, angioedema, urticaria and gastrointestinal symptoms. This type of allergy (recognized as Meat Allergy or Mammalian Meat allergy) has a time course different from common food allergies and it was firstly described in 2009 by Commins and colleagues (Commins et al., J allergy Clin Immunol2009; 123:426-33) in US patients reporting delayed allergic systemic symptoms. In these patients they recognized high titer of IgE antibodies directed against the mammalian oligosaccharide epitope galactose-α-1,3-galactose (a-gal) demonstrating a strict correlation between red meat meal consumption and the allergic reaction. In the same period high serum level of IgE specific for the α-gal antigen was described also for oncologic patients that, after treatment with the monoclonal antibody Cetuxymab anaphylactic shocks (Chung et al., N Engl J Med 2008; 358: 1108-17). Instead, following characterization of new patients affected by the red meat allergy and extended population studies of the affected areas, confirmed the delayed allergic symptoms introducing a new correlation between the tick bites, the high titer of alpha-gal IgE and the delayed allergic reaction (Commins et al., J Allergy Clin Immunol. 2011May; 127(5): 1286-1293). These findings demonstrated that tick bites (*Amblyomma americanus*, *Ixodes ricinus, Ixodes holocyclus, Ixodes pacificus*) are the sensitizing event for the production of specific IgE (sIgE), leading to a reduction of tolerance to livestock products in some patients. A new study defined also that these patients developed a sensitization to a common non-human mammals derivatives ingredient, like gelatin that was recognised by the α-gal sIgE (Mullins et al., J Allergy Clin Immunol. 2012; 129: 1334-1342). Gelatin is an ingredient of some processed foods and gelatin colloids and it can be used also in vaccines production as stabilizing reagents or it could be used for capsules constitution of different drugs, indeed it is real the risk that many more hidden animal derivatives could provoke an anaphylactic shock in patients affected by the red meat allergy. Number of reported cases is still expanding in different countries like Australia, Germany, Spain, Sweden, Japan and Italy (Commins et al., Allergol Int. 2016;65: 16-20;Steinke et al., J Allergy Clin Immunol. 2015; 135: 589-597; Apostolovich et al., Allergo J Int 2016; 25:49-54; Takahashi et al. Allergy 2014; 69: 199-207; Takahashi et al. Allergy 2014; 69: 199-207; Calamari et al., Eur Ann Allergy Clin Immunol 2015, 47:161-162) recognizing higher the sensitization risk for people exposed to the tick bites, such as forest workers and hunters (Fisher J. et al, Allergy 2017; 72:1540-1547).

Alpha gal antigen is expressed in all mammals except for humans and non-human primates, because the gene coding for the enzyme alpha 1-3 galactosyltransferase was lost during evolution (Galili et al., J Biol Chem. 1988 Nov 25;263:17755-62). For this reason anti-alpha gal antibodies (IgG and IgM) are the most abundant natural antibodies in humans (1%) and they were recognized as the main responsible for the hyperacute rejection of xenotransplanted organs in pig-to non-human primates experiments (Galili U, Glycobiology 2016 Nov;26(11):1140-1150.). Following xenotransplantation studies of sera of non-human primates transplanted with porcine Gal-KO organs led to the characterization of new non-alpha gal antibody populations responsible for the acute vascular rejection of transplanted grafts, identifying in pig new xeno-antigens, such as the Neu5GC, that is not expressed in humans due to gene mutations occurred in the CMAH gene (Byrne et al., Xenotransplantation 2008; 15:268-276) and the Sd^{a} expressed by the porcine B4GalNT2 gene (Byrne et al., Transplantation 2011; 91:287-292).

α(1,3) galactosyltransferase (aGal or αGal), and cytidine monophosphate-N-acetylneuraminic acid hydroxylase (CMAH) are expressed by non-human-mammals and not by human cells. Non-human mammal cells also express β1-4 N-acetylgalactosaminyltransferase (B4GalNT2 or β4GalNT2).. The aGal enzyme catalyzes the formation of galactose-α1,3-galactose (aGal) residues on glycoproteins. CMAH converts the sialic acid N-acetylneuraminic acid (Neu5Ac) to N-glycolylneuraminic acid (Neu5Gc). B4GalNT2 catalyzes the terminal addition of N-acetylgalactosamine to a sialic acid modified lactose amine to yield Sd^{a}-like antigens, including but not limited to Sd^{a} and CAD antigens. Porcine B4GalNT2 may catalyze the formation of additional glycans as well. Xenotransplantation studies in pig to non-human primates models demonstrated that pre-formed antibodies to the aGal, Neu5Gc and Sd^{a} antigens are present in primates blood and they are involved in the intense and immediate antibody mediated rejection of implanted tissues, justifying the inactivation of the porcine enzymes responsible of their synthesis (Estrada et al., Xenotransplantation 2015 22:194-202).

Generation of mutated pigs (GGTA1/CMAH/B4GalNT2-KO) were reported in patents WO2016065046 A1 and WO2016094679 A1, protecting their application as donors of organs, tissues, cells and derivatives especially for xenotransplantation purposes in human. However these patents never propose the use as source for healthier and safer food and medical products considering their low allergenic properties.

But today the need and importance is increasingly felt for the development of low antigenic animals as a source of animal derivatives for prevention and treatment of xeno-allergies and possibly chronic degenerative or autoimmune diseases caused by their use and consumption in humans.

It is therefore object of the present invention the development of modified livestock animals, obtained with the newly available genome editing technologies having the low antigenic qualities useful in many industrial productions for the food and medical chain.

### SUMMARY OF THE INVENTION

The present invention concerns a genetically modified non-human mammal having the combination of mutated or disrupted α(1,3)-galactosyltransferase, CMAH and B4GalNT2 genes in a said genetically modified non-human mammal, wherein expression of α(1,3)-galactosyltransferase, cytidine monophosphate-N-acetylneuraminic acid hydroxylase and β1,4-N-acetylgalactosaminyltransferase is abolished as compared to a wild-type genetically modified non-human mammal.

The purpose underlying the present invention is that of making available low antigenic animals as source of animal derivatives for prevention and treatment of xeno-allergies, chronic degenerative and autoimmune diseases caused by their consumption and treatments in humans.

This problem is resolved by the present finding by edited genetically modified non-human mammals combining the mutated or disrupted α(1,3)-galactosyltransferase, CMAH and B4GalNT2 gene, wherein expression of α(1,3)-galactosyltransferase, cytidine monophosphate-N-acetylneuraminic acid hydroxylase and β1,4-N-acetylgalactosaminyltransferase is abolished compared to a wild-type genetically modified non-human mammal, as identified in the attached claims.

In a second aspect, this invention moreover provides a product isolated from the edited genetically modified non-human mammal, wherein said product is chosen from the group consisting of an organ, tissue, carcass, meat, milk, milk derivatives transfusion product or cell(s).

As will be further described in the detailed description of the invention, the products isolated from the genetically modified non-human mammal s of the present invention have the advantages of being able to prevent specific diseases.

A further aspect of the present invention is the use of the product isolated from the genetically modified non-human mammals for the preparation of animal derived products to prevent specific xeno-allergies.

The alpha 1,3 galactosyltransferase (aGal, αGal, GGTA, GGT1, GT, aGT, GGTA1, GGTA-1) gene encodes an enzyme (GT, aGal, α1,3 galactosyltransferase). Functional a1,3 galactosyltransferase catalyzes formation of galactose-α-1,3-galactose residues on glycoproteins. The galactose-α-1,3-galactose (αGal) residue is an antigenic epitope or antigen recognized by the human immunological system. Removing aGal from edited organ material does not eliminate the human immunological response to transplant of foreign material, suggesting an involvement of additional antibodies in the rapid immunological response to xenotransplant. (Mohiudden et al (2014), Am J. Transplantation 14:488-489 and Mohiudden et al 2014 Xenotransplantation 21:35-45). Disruptions of the porcine GGTA1 gene that abolish the expression of functional a1,3 galactosyltransferase in pig and bovine may include substitutions, deletions and insertions. (see Table 2 and Table 3).

The cytidine monophosphate-N-acetylneuraminic acid hydroxylase (CMP-Neu5Ac hydroxylase gene, CMAH) gene encodes an enzyme (CMAH). Functional CMAH catalyzes conversion of sialic acid N-acetylneuraminic acid (Neu5Ac) to N-glycolylneuraminic acid (Neu5Gc). The Neu5Gc residue is an antigenic epitope or antigen recognized by the human immunological system. Disruptions of the CMAH gene that abolish the expression of functional CMAH in pig and in bovine may include but are not limited to substitution, deletion, insertions (see Table 2 and Table 3).

The β1,4-N-acetylgalactosaminyltransferase (B4GalNT2, B4GalNT2, B1,4GalNT2, 131,4GalNT2) gene encodes an enzyme (B4GalNT2). Functional B4GalNT2 produces Sd^{a}-like glycans (Dall'Olio et al (2014) Biochemica Biophysica Acta 1840:443-453 and Blanchard et al (1983) JBC 258:7691-7685). B4GalNT2 is thought to be expressed in most humans. Disruption of B4GalNT2 gene that abolish the expression of functional B4GalNT2 in pig may include but are not limited to substitution, deletion, insertions (see Table 3).

### BRIEF DESCRIPTION OF THE DRAWINGS

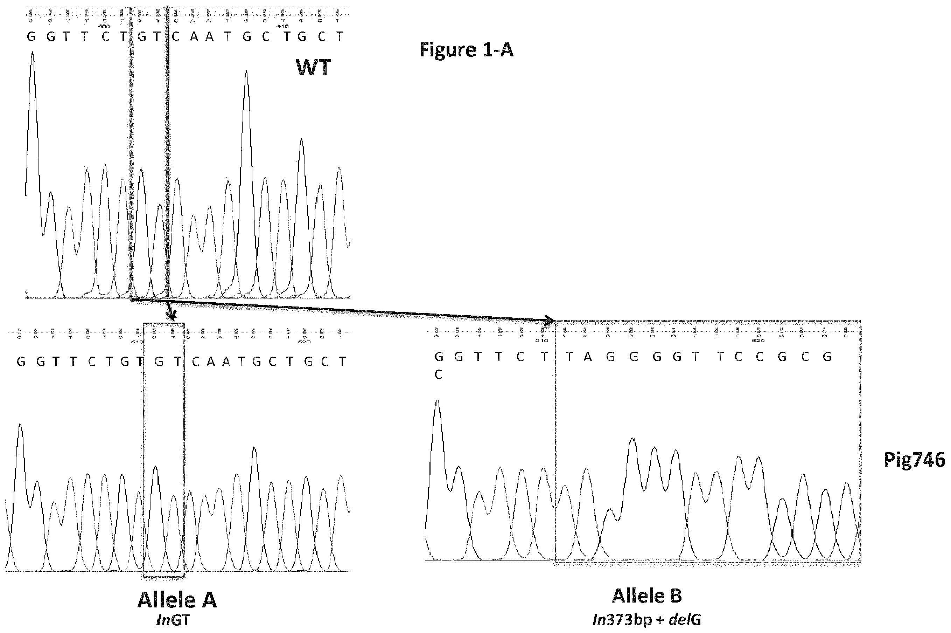

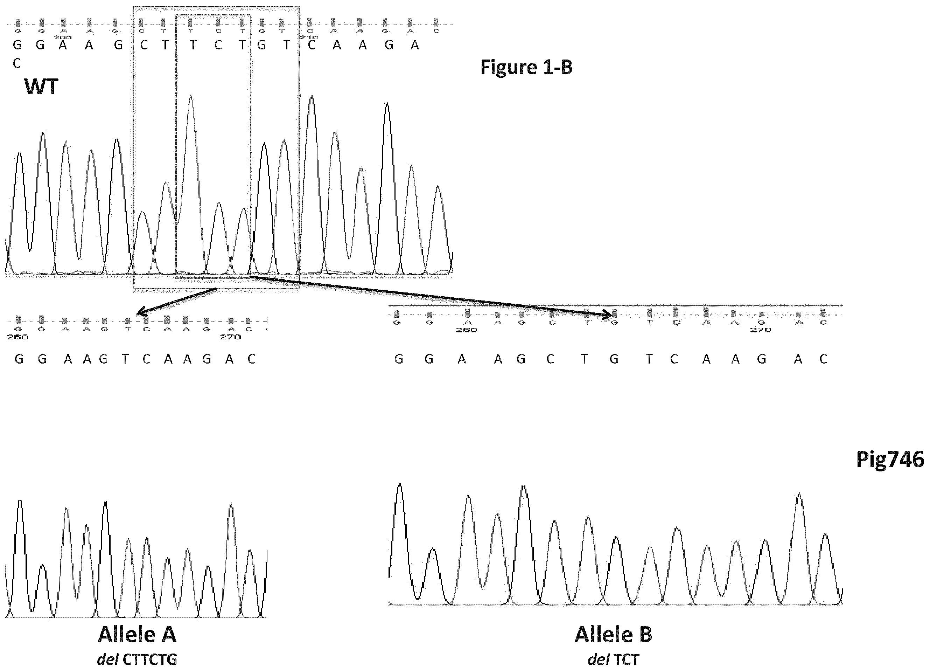

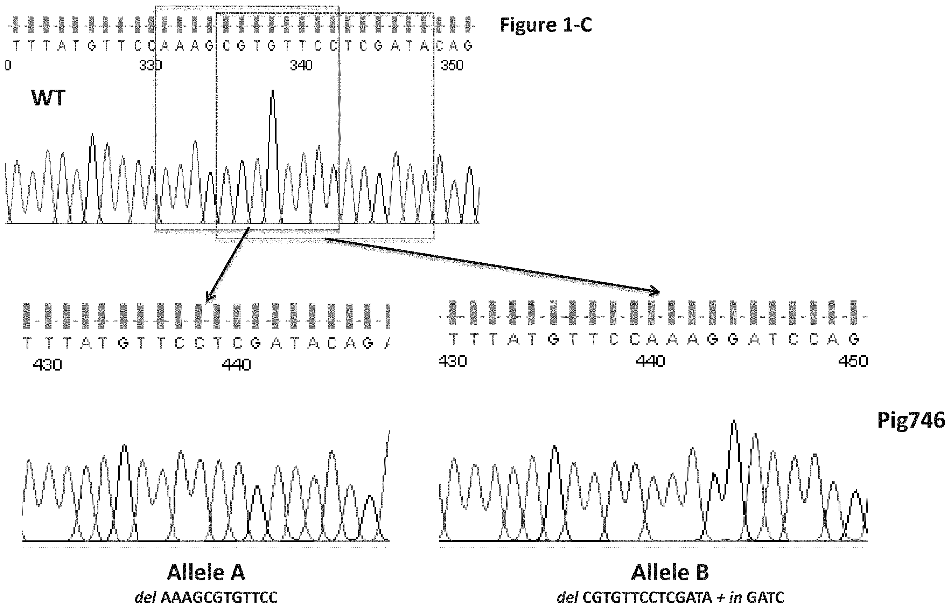

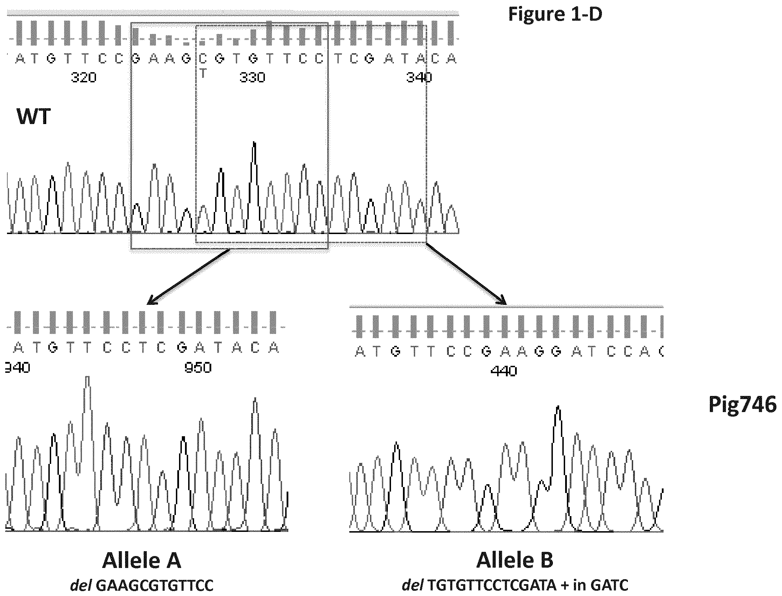

The characteristics and advantages of the present invention will be apparent from the detailed description reported below, from the Examples given for illustrative and non-limiting purposes, and from the annexed Figures 1-5, wherein:
Figure 1: shows the genotyping results describing the mutations (*InsDels*) detected for target genes (Figure 1-A = GGTA1; Figure 1-B = CMAH; Figure 1-C = B4GalNT2; Figure 1-D = B4GalNT2-like) of cloned Pig746.
Figure 1-A TKO pig - genome sequences. Sequences results for GGTA1 gene of Pig746.
   Wild type sequences (WT) and the mutated allelic sequences of GGTA1 gene characterising the edited Pig746 (in GT; in 373 + del G) are presented here. Genomic sequences where insertions occurred are separately highlighted (continue and dotted lines) in the WT sequence (UP) and described into the resulting sequencing data for the mutated allele (Allele A and Allele B) of Pig746 (DOWN).
Figure 1-B TKO pig - genome sequences.
   Sequences results for CMAH gene of Pig746.
   Wild type sequences (WT) and the mutated allelic sequences of CMAH gene characterising the edited Pig746 (del CTTCTG; del TCT) are presented here. Detected mutation are separatley highlighted (continue and dotted lines) in the WT sequence (UP) and confirmed by the resulting sequencing data for the Allele A and B of Pig746 (DOWN).
Figure 1-C TKO pig - genome sequences.
   Sequences results for B4GalNT2 gene of Pig746.
   Wild type sequences (WT) and the mutated allelic sequences of B4GalNT2 gene characterising the edited Pig746 (del AAAGCGTGTTCC; del CGTGTTCCTCGATA + in GATC) are presented here. Detected mutation are separatley highlighted (continue and dotted lines) in the WT sequence (UP) and confirmed by the resulting sequencing data for the Allele A and B of Pig746 (DOWN).
Figure 1-D TKO pig - genome sequences.
   Sequences results for B4GalNT2-like gene of Pig746.
   Wild type sequences (WT) and the mutated allelic sequences of B4GalNT2-like characterising the edited Pig746 (del GAAGCGTGTTCC; del TGTGTTCCTCGATA + in GATC) are presented here. Detected mutation are separately highlighted (continue and dotted lines) in the WT sequence (UP) and confirmed by the resulting sequencing data for the Allele A and B of Pig746 (DOWN).

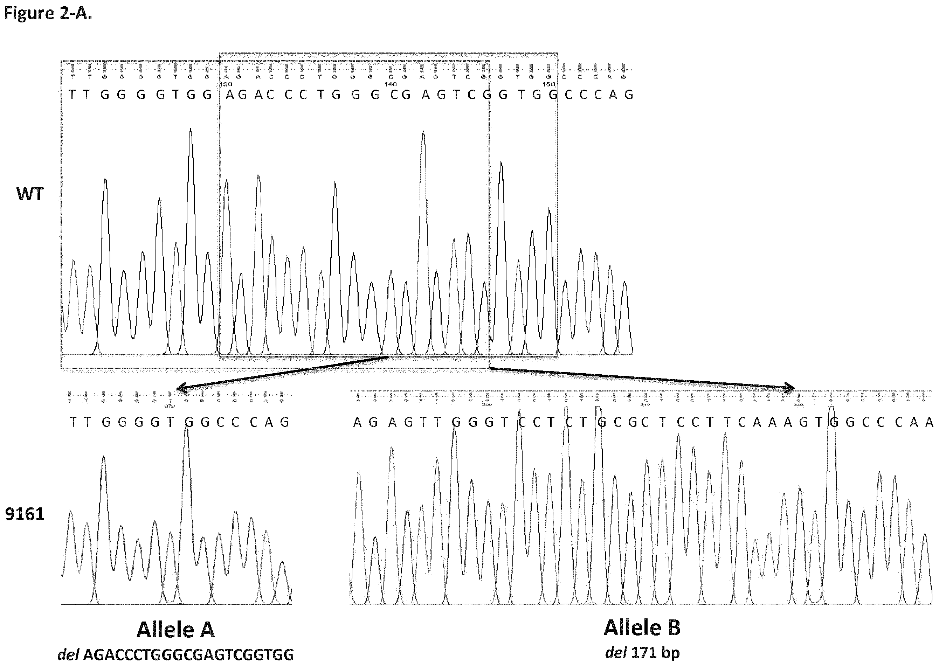

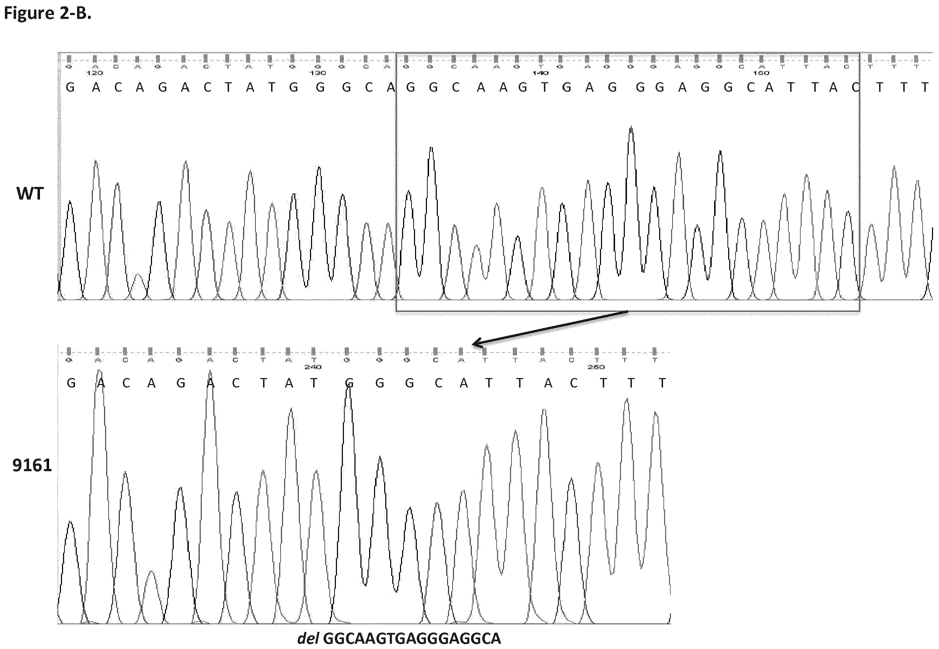

Figure 2: show the genotyping results describing the mutations (*InsDels*) detected for the two target genes (Figure 2-A = GGTA1; Figure 2-B = CMAH) of the cloned calf 9161.
Figure 2-A. DKO 9161 calf - genome sequences. Sequences results for GGTA1 gene of calf 9161.
   For the GGTA1 gene are presented the wild type sequences (WT - UP) and the two mutated allelic sequences characterising the edited calf 9161 (DOWN). Biallelic mutation of CMAH gene(del GAGCCCTGGGCGAGTCGGTGG; del 171 bp) are highlighted (continue or dotted line) in the WT sequence and the resulting mutated CMAH gene sequences are confirmed by the sequencing data for the calf 9161.
Figure 2-B. DKO 9161 calf - genome sequences. Sequences results for CMAH gene of calf 9161.
   For the CMAH gene are presented the wild type sequences (WT - UP) and the mutated allelelic sequences characterising the edited calf 9161 (DOWN). Biallelic mutation of CMAH gene(del GGCAAGTGAGGGAGGCA) is highlighted in the WT sequence and the resulting mutated CMAH gene sequence is confirmed by the sequencing data for the calf 9161.
Figure 3: shows the phenotypic characterization (FACS) of primary cells (fibroblasts and PBMC) derived from cloned Pig746. Fibroblasts resulted negative for the *α-Gal* (Figure 3-A) and *Neu5Gc* (Figure 3-B) epitopes. *Sd^{a}* expression (Figure 3-C) was investigated on PBMC cells resulted also negative.
Figure 3-A. Pig746 FACS analyses. Expression of α-Gal antigen in primary fibroblasts and PBMCs.
   Fibroblasts from wild type animal (WT) and from the edited Pig746 (746) were analysed by FACS. Shown results demonstrated that the α*-GAL epitope* was deleted from the cell surface of our cloned animal, finally confirming the genotype analyses (knock-out) for the GGTA1 gene.
Figure 3-B. Pig746 FACS analyses. Expression of Neu5Gc antigen in primary fibroblasts and PBMCs.
   Fibroblasts from wild type animal (WT) and from the edited Pig746 (746) were analysed by FACS. Shown results demonstrated that the *Nue5Gc* epitope was deleted from the cell surface of our cloned animal, finally confirming the genotype analyses (knock-out) for the CMAH gene.
Figure 3-C. Pig746 FACS analyses. Expression of Sd^{a} antigen in primary fibroblasts and PBMCs.
   PBMC from wild type animal (WT) and from the edited Pig746 (746) were analysed by FACS. Shown results demonstrated that the Sd^{a} epitope was deleted from the cell surface of our cloned animal, finally confirming the genotype analyses (knock-out) for the B4GalNT2 and B4GalNT2-like genes.
Figure 4: shows the phenotypic characterization (FACS) of primary fibroblasts derived from cloned bovine 9161. Fibroblasts resulted negative for the *αGal* (Figure 4-A) and *Neu5Gc* (Figure 4-B) epitopes.
Figure 4-A. FACS analyses on Gal expression - cloned DKO calf (9161) Fibroblasts from wild type animal (WT) and from the edited calf 9161 (9161) were analysed by FACS. Shown results demonstrated that the α*-GAL* antigen was deleted from the cell surface of our cloned animal, confirming the genotype analyses for the knocked-out gene (GGTA1).
Figure 4-B. FACS analyses on Neu5Gc expression - cloned DKO calf (9161)
   Fibroblasts from wild type animal (WT) and from the edited calf 9161 (9161) were analysed by FACS. Shown results demonstrated that the *Neu5Gc* antigen was deleted from the cell surface of our cloned animal, confirming the genotype analyses for the knocked-out gene (CMAH).
Figure 5: shows the Surveyor assay tests done for the selection of site specific nucleases to use for genome editing in pig, bovine (5-A) and equine (5-B).
Figure 5-A. Surveyor assays - Talens (pig) and CRISPR/Cas9 (bovine) selection.
   Cells transfected (+) and not-transfected (-) with the plasmids coding for the Site Specific Nucleases (Talens for pig; CRISPR/Cas9 for bovine) for the selected gene were used to test the endonucleases cutting efficiency using the Surveyor assay. Here are shown some examples or the results obtained justifying the Talens selection for the porcine GGTA1 and CMAH genes where could be detected bands(*) resulting from digestion of the PCR product.
   Also using the CRISPR/Cas9 system for the bovine CMAH gene we where able to select 2 different sgRNAs (cr5 and cr8) that produced the expected fragments
   (*) with different efficiency (cr5>cr8).

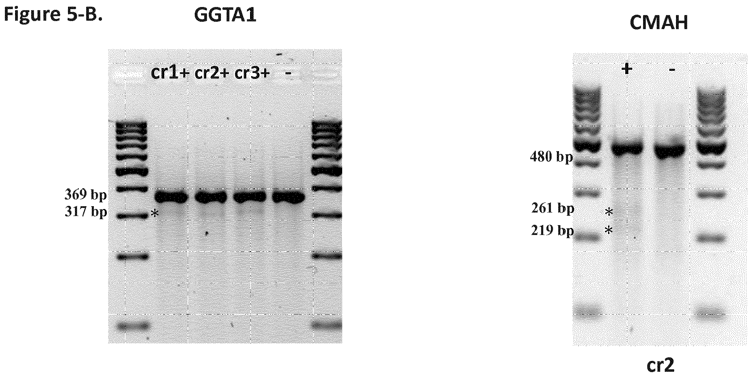

Figure 5-B. Surveyor assays - CRISPR/Cas9 selection (horse)
   Equine fibroblasts transfected (+) and not-transfected (-) with the plasmids coding for different CRISPR/Cas9 specific for the GGTA1 and CMAH genes were used to test the endonucleases cutting efficiency using the Surveyor assay. Here are shown some examples or the results obtained justifying the selection of 3 different sgRNAs (cr1, cr2, cr3) for the equine GGTA1 gene and one sgRNA(cr2) for the CMAH gene, where could be detected bands(*) resulting from digestion of the PCR product.
Figure 6.

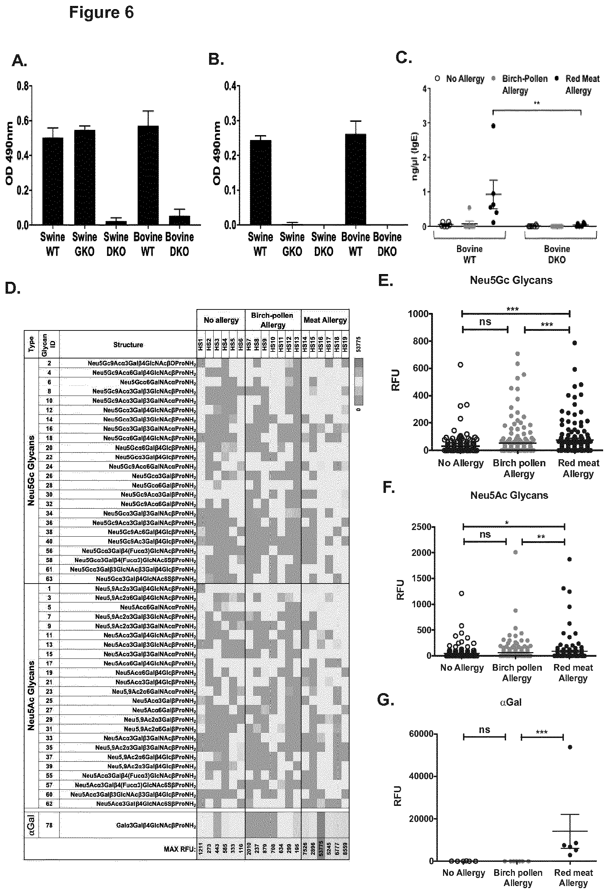

Figures 6A and 6B graphically show the biochemical characterization of Neu5Gc and α-Gal in meat samples. Meat homogenates were coated onto a 96-well plate in triplicates (1 µg/well) and analyzed with anti-Neu5Gc IgY (Neu5Gc) (Figure 6A), or monoclonal anti aGal IgM (Figure 6B), washed then detected with HRP-donkey-anti-chicken IgY or HRP-goat-anti-mouse IgM, respectively.
Figure 6C Human serum IgE reactivity against meat samples. Meat homogenates were coated onto a 96-well plate in duplicates (1µg/well), then reactivity of human serum IgE (1:100) was examined by ELISA in samples of donors with no allergy, birch-pollen allergy or red meat allergy.
Figures 6D, 6E and 6F: Reactivity of human serum IgE (1:100) was examined by glycan microarrays using samples of donors with no allergy, birch-pollen allergy or red meat allergy allergy. In Figure 6D, glycan microarray profiles of human sera samples donors are presented in Relative Fluorescent Units (RFU;
representing fluorescence at 532 nm after local background subtraction) as a heatmap. In Figure 6E reactivity of sera samples against Neu5Gc-glycans was compared between the groups showing that red meat allergy sera have a higher anti-Neu5Gc IgE reactivity (mean ± SEM; each spot is a different glycan per serum; Kruskal Wallis test with Dunn's multiple comparison test; P < 0.0001).
Figure 6F: the reactivity of sera samples against Neu5Ac-glycans was compared between the groups. Figure 6G shows the reactivity of sera samples against aGal glycan on the microarray compared between the groups.

### DETAILED DESCRIPTION OF THE INVENTION

The present invention concerns a multiplexed genetically modified non-human mammal comprising a mutated or disrupted α(1,3)-galactosyltransferase, CMAH and B4GalNT2 gene in at least one cell of said livestock animal, wherein expression of α(1,3)- galactosyltransferase, CMAH and B4GalNT2 is abolished compared to a wild-type livestock animal.

In particular said genetically modified non-human mammal is chosen from the group consisting of a pig, a bovine, an equine, a sheep, a goat, a buffalo, a camel and a rabbit, said non-human mammal comprising mutated α(1,3)-galactosyltransferase (GGTA1), cytidine monophosphate-N-acetylneuraminic acid hydroxylase (CMAH) and beta1-4N-acetylgalactosaminyltransferase (B4GalNT2) genes in at least one cell,
wherein:
when said non-human mammal is a pig:
   - the α(1,3)-galactosyltransferase gene has a sequence chosen from the group consisting of SEQ ID NO:91, SEQ ID NO:92, SEQ ID NO:93, SEQ ID NO:94, SEQ ID NO:95, SEQ ID NO:96, SEQ ID NO:97 and SEQ ID NO:98;
   - the CMAH gene has a sequence chosen from the group consisting of SEQ ID NO:99, SEQ ID NO:100, SEQ ID NO:101, SEQ ID NO:102, SEQ ID NO:103, SEQ ID NO:104 and SEQ ID NO:105; and
   - the B4GalNT2 gene has a sequence chosen from the group consisting of SEQ ID NO:106, SEQ ID NO:107, SEQ ID NO:108, SEQ ID NO:109, SEQ ID NO:110, SEQ ID NO:111, SEQ ID NO:112, SEQ ID NO:113, SEQ ID NO:114, SEQ ID NO:115, SEQ ID NO:116, SEQ ID NO:117, SEQ ID NO:30, SEQ ID NO:31, SEQ ID NO:118 and SEQ ID NO:119;
when said non-human mammal is a bovine:
   - the α(1,3)-galactosyltransferase gene has a sequence chosen from the group consisting of SEQ ID NO:120, SEQ ID NO:121, SEQ ID NO:122, SEQ ID NO:123, SEQ ID NO:124 and SEQ ID NO:125; and
   - the CMAH gene has a sequence chosen from the group consisting of SEQ ID NO:126, SEQ ID NO:127, SEQ ID NO:128, SEQ ID NO:129, SEQ ID NO:130, SEQ ID NO:131 and SEQ ID NO:132; and
when said non-human mammal is an equine:
   - the α(1,3)-galactosyltransferase gene having the sequence of SEQ ID NO:68;
   - the CMAH gene having a sequence chosen from the group consisting of SEQ ID NO:78, SEQ ID NO:79 and SEQ ID NO:80; and
   - the B4GalNT2 gene having the sequence of SEQ ID NO:90 are mutated.

The genetically modified non-human mammals, comprising a disrupted GGTA1, CMAH and B4GalNT2 genes, are not expressing the α(1,3)-galactosyltransferase, cytidine monophosphate-N-acetylneuraminic acid hydroxylase and β1,4-N-acetylgalactosaminyltransferase in their organs, tissues or cells and may be used as source animal derivatives for feeding and or treating patients affected by the red meat allergy or patients affected by chronic degenerative and autoimmune diseases.

The red meat allergy may be given also by the animal innards.

The term "edited" as used herein, is intended as "mutated" and thus containing one of more genes that have been altered, removed or disrupted. It is to be emphasized that the term is to be intended to include also the progeny (descendant) of such animals. Thus, the founder animal and all F1, F2, F3 and so on progeny thereof are included, regardless of whether progeny were generated by somatic cell nuclear transfer (SCNT) from the founder animal or a progeny animal or by traditional breeding. By "single edited" is meant a genetically modified non-human mammal wherein one gene has been altered, removed or disrupted. By "double edited" is meant a genetically modified non-human mammal wherein two genes have been altered, removed or disrupted. By "triple edited" is meant a genetically modified non-human mammal wherein three genes have been altered, removed or disrupted.

By the term "disrupted" as used herein, it is intended to include mutated by insertion, deletion or substitution of one or more bases. A "mutation" is a detectable change in the genetic material in the animal that is transmitted to the animal's progeny. A mutation is usually a change in one or more deoxyribonucleotides, such as, for example adding, inserting, deleting, inverting or substituting nucleotides.

In principle, edited animals may have both copies of the gene sequence of interest disrupted. In the case where only one copy or allele of the nucleic acid sequence of interest is disrupted, the knockout animal is termed a "heterozygous edited animal". The term "null" mutation encompasses both instances in which the two copies (alleles) of a nucleotide sequence of interest are disrupted differently but for which the disruptions overlap such that some genetic material has been removed from both alleles, and instances in which both alleles of the nucleotide sequence of interest share the same disruption.

In a preferred embodiment, the genetically modified non-human mammal according to the invention is chosen, but not limited to, from the group consisting of pig, bovine, equine, sheep, goat, buffalo, asses, camels and rabbit.

For the purposes of the present disclosure, each gene has a corresponding SEQ ID NO. as described herein.

Edited animals suitable for use in generation of low antigenicity foodstuffs and medical products or devices are provided.

Food allergy symptoms are very common and can appear at any age. Allergies to food can be developed to foods that have been eaten for years without any reaction.

Symptoms of an allergic reaction may involve the skin, the gastrointestinal tract, the cardiovascular system and the respiratory tract. The present invention wishes to solve the problem of food allergy to meat and related syndromes.

Specifically, the present application describes the production of cloned triple edited pigs (triple knock-out) that don't express the enzymes alpha 1,3 galactosyltransferase (aGal), cytidine monophosphate-N-acetylneuraminic acid hydroxylase (CMAH) and β 1,4 N-acetylgalactosaminyltransferase (B4GalNT2 and B4GalNT2-like) and the production of double edited bovines that don't express the enzymes alpha 1,3 galactosyltransferase (aGal) and cytidine monophosphate-N-acetylneuraminic acid hydroxylase (CMAH).

Expression of a gene product is abolished or suppressed when total expression of the gene product is undetectable (mRNA and protein), a gene product of an altered size is produced or when the gene product exhibits an altered functionality. Thus, if a gene is not expressed or if a gene expresses and altered (enzyme activity, size, cellular localization pattern, receptor-ligand binding or other interaction) product expression of that gene product is abolished. Expression may be analyzed by any means known in the art including, but not limited to, RT-PCR, Western blots, Northern blots, microarray analysis, immunoprecipitation, radiological assays, polypeptide purification, spectrophotometric analysis, Coomassie staining of acrylamide gels, ELISAs, 2-D gel electrophoresis, in situ hybridization, chemiluminescence, silver staining, enzymatic assays, ponceau S staining, multiplex RT-PCR, immunohistochemical assays, radioimmunoassay, colorimetric assays, immunoradiometric assays, positron emission tomography, fluorometric assays, fluorescence activated cell sorter staining of permeablized cells, radioimunnosorbent assays, real-time PCR, hybridization assays, sandwich immunoassays, flow cytometry, SAGE, differential amplification or electronic analysis. Expression may be analyzed directly or indirectly. Indirect expression analysis may include but is not limited to, analyzing levels of a product catalyzed by an enzyme to evaluate expression of the enzyme.

In a further embodiment, the genetically modified non-human mammal according to the invention is a pig and:
- the α(1,3)-galactosyltransferase gene having the sequence of SEQ ID NO:10;
- the CMAH gene having a sequence of SEQ ID NO:20; and
- the B4GalNT2 gene has a sequence of SEQ ID NO:30, SEQ ID NO:31 or SEQ ID NO:32 are mutated.

The edited pig according to the invention surprisingly has an expression of α(1,3)-galactosyltransferase, CMAH and B4GalNT2 which is abolished compared to a wild-type pig, and, for example, when using tissue from said edited pig any allergic reaction is prevented as compared to when tissue from a wild-type pig is used.

In embodiments of the present invention, pigs and porcine organs, tissues and cells therefrom are provided in which the αGal, CMAH, B4GalNT2 and B4GalNT2-like genes are not active, such that the resultant αGal, CMAH, B4GalNT2 products not generate wild-type of a1,3-galactosyl epitopes, Neu5Gc or Sd^{a}-like epitopes on a cell surface, glycoprotein or glycolipid.

In an alternative embodiment the αGal, B4GalNT2 and CMAH genes are inactivated in such a way that no transcription of the gene occurs. In various embodiments triple αGal/B4GalNT2/CMAH knockout pigs were made.

In a still more preferred embodiment, the edited pig according to the invention wherein
- the α(1,3)-galactosyltransferase gene has the sequence of SEQ ID NO:91;
- the CMAH gene has the sequence of SEQ ID NO:99; and
- the B4GalNT2 gene has the sequences of SEQ ID NO:106 in allele A and SEQ ID NO:107 in allele B or SEQ ID NO:114 in allele A and SEQ ID NO:31 in allele B;
   or
- the α(1,3)-galactosyltransferase gene has the sequences of SEQ ID NO:92 in allele A and SEQ ID NO:93 in allele B;
- the CMAH gene has the sequences of SEQ ID NO:100 in allele A and SEQ ID NO:101 in allele B; and
- the B4GalNT2 gene has the sequences of SEQ ID NO:108 in allele A and SEQ ID NO:109 in allele B or SEQ ID NO:115 in allele A and SEQ ID NO:116 in allele B;
   or
- the α(1,3)-galactosyltransferase gene has the sequence of SEQ ID NO:94;
- the CMAH gene has the sequence of SEQ ID NO:102; and
- the B4GalNT2 gene has the sequences of SEQ ID NO:110 in allele A and SEQ ID NO:111 in allele B or SEQ ID NO:117 in allele A and SEQ ID NO:31 in allele B;
   or
- the α(1,3)-galactosyltransferase gene has the sequence of SEQ ID NO:95 in allele A and SEQ ID NO: 96 in allele B;
- the CMAH gene has the sequence of SEQ ID NO:103 in allele A and SEQ ID NO: 104 in allele B; and
- the B4GalNT2 gene has the sequences of SEQ ID NO:112 in allele A and SEQ ID NO:30 in allele B or SEQ ID NO:118 in allele A and SEQ ID NO:118 in allele B;
   or
   Fetus-2.
- the α(1,3)-galactosyltransferase gene has the sequence of SEQ ID NO:97 in allele A and SEQ ID NO: 98 in allele B;
- the CMAH gene has the sequence of SEQ ID NO:105; and
- the B4GalNT2 gene has the sequences of SEQ ID NO:113 in allele A and SEQ ID NO:113 in allele B or SEQ ID NO:119 in allele A and SEQ ID NO:31 in allele B;
Edited animals suitable for use in xenotransplantation and methods of producing mammals suitable for use in generation of healthier food and medical products and methods of producing mammals suitable for use in food and medical productions are provided (Example 9 and Example 10). Specifically, the present invention describes the production of double edited cattle (double knock-out) with abolished expression of alpha 1,3 galactosyltransferase (aGal) and (β 1,4 N-acetylgalactosaminyltransferase (B4GalNT2).

In a further embodiment, the genetically modified non-human mammal according to the invention is a bovine and:
- the α(1,3)-galactosyltransferase gene having the sequence of SEQ ID NO:40 or SEQ ID NO:41;
- the CMAH gene having a sequence chosen from the group consisting of SEQ ID NO:46 are mutated; and optionally
- the B4GalNT2 gene has a sequence of SEQ ID NO:59 is also mutated.

The edited bovine according to the invention surprisingly has an expression of α(1,3)-galactosyltransferase and CMAH which is abolished compared to a wild-type bovine, and when using tissue from said edited bovine any allergic reaction is prevented as compared to when tissue from a wild-type bovine is used.

In a preferred embodiment, the genetically modified non-human mammal is a bovine and wherein,
- the α(1,3)-galactosyltransferase gene has the sequences of SEQ ID NO:120 in allele A and SEQ ID NO:121 in allele B;
- the CMAH gene has the sequence of SEQ ID NO:126
   or
- the α(1,3)-galactosyltransferase gene has the sequences of SEQ ID NO:122;
- the CMAH gene has the sequences of SEQ ID NO:127 in allele A and SEQ ID NO:128 in allele B
   or
   Fetus-2180
- the α(1,3)-galactosyltransferase gene has the sequences of SEQ ID NO: 123;
- the CMAH gene has the sequences of SEQ ID NO:129 in allele A and SEQ ID NO:130 in allele B
   or
   Fetus-2197.
   the α(1,3)-galactosyltransferase gene has the sequences of SEQ ID NO:124 in allele A and SEQ ID NO:125 in allele B;
- the CMAH gene has the sequences of SEQ ID NO:131 in allele A and SEQ ID NO:132 in allele B.

The edited equine according to the invention surprisingly has an expression of α(1,3)-galactosyltransferase, CMAH and B4GalNT2 which is abolished compared to a wild-type equine, and when using tissue from said edited equine any allergic reaction is prevented as compared to when tissue from a wild-type equine is used.

In a still further embodiment the present invention provides a product isolated from the genetically modified non-human mammal wherein said product is chosen from the group consisting of an carcass, meat, milk, organ, tissue, transfusion product or a cell.

The organ, tissue, transfusion product, cell or derivative could be used by patients affected by the red meat syndrome improving an allergy related symptom as compared to when an organ, tissue, transfusion product, cell or derivative from a wild-type livestock animals is used.

When the organ, tissue, transfusion product, cell or derivative from the genetically modified non-human mammals of the invention is used by humans, anaphylaxis, urticarial, alpha gal IgE antibodies and gastrointestinal diseases are abolished as compared to when an organ, tissue, transfusion product, cell or derivative from a wild-type livestock animal is used.

Furthermore, when the organ, tissue, transfusion product, cell or derivative from the genetically modified non-human mammals of the invention is used by humans, angioedema is decreased as compared to when an organ, tissue, transfusion product, cell or derivative from a wild-type livestock animal is used by humans.

Edited meat material can be isolated from edited animal. Any organ can be used including, but not limited to, the brain, heart, lungs, eye, muscle, stomach, pancreas, kidneys, liver, intestines, uterus, bladder, skin, hair, nails, ears, glands, nose, mouth, lips, spleen, gums, teeth, tongue, salivary glands, tonsils, pharynx, esophagus, large intestine, small intestine, small bowel, rectum, anus, thyroid gland, thymus gland, bones, cartilage, tendons, ligaments, suprarenal capsule, skeletal muscles, smooth muscles, blood vessels, blood, spinal cord, trachea, ureters, urethra, hypothalamus, pituitary, pylorus, adrenal glands, ovaries, oviducts, uterus, vagina, mammary glands, testes, seminal vesicles, penis, lymph, lymph nodes and lymph vessels.

In a preferred embodiment the present invention provides an organ isolated from the genetically modified non-human mammal, wherein said organ is selected from the group consisting of muscle, udder, skin, heart, liver, kidneys, lung, pancreas, thyroid, small bowel, blood and components thereof.

In a still further embodiment the present invention provides the use of the product isolated from the genetically modified non-human mammal of the invention for the preparation of animal derived products to prevent specific xeno-allergies and chronic degenerative and autoimmune diseases.

The products according to the invention surprisingly do not express the αGal, Neu5GC and Sd^{a}-like antigens, thus avoiding formation of new, or the reaction of specific human xeno-antibodies (IgE, IgG, IgM, etc).

The products according to the invention surprisingly prevent an allergy related symptom as compared to when an organ, tissue, transfusion product, cell or derivative from a wild-type livestock animals is used.

In a still further embodiment the present invention provides the use of the product isolated from the genetically modified non-human mammal for the preparation of animal derived products to prevent specific xeno-allergies, food born allergies and autoimmune and degenerative diseases.

In a still further embodiment the present invention provides the use of the product isolated from the genetically modified non-human mammal for the direct consumption or for the preparation of animal derived products (foodstuffs) for human consumption and for advanced therapy medicinal products.

In a still further embodiment the present invention provides the use of the product isolated from the genetically modified non-human mammal for the preparation of animal derived products for human drug production and for human medical devices production.

In a still further embodiment the present invention provides the use of the product isolated from the genetically modified non-human mammal for the preparation of animal derived products for antisera or antibody production and for products for human cosmetics and human detergents production.

The invention further relates to a method of preparing animal derived products to prevent specific xeno-allergies, comprising providing the genetically modified non-human mammal as a source of raw biological materials and wherein said raw biological material is selected from the group consisting of organs, tissues, transfusion products, cells or derivative and wherein said raw biological material do not express the αGal antigens, the Neu5GC antigens and the Sd^{a}-like antigens, avoiding formation of new specific human xeno-antibodies.

**Table 1. Oligonucleotides used for genotyping of wild type cell lines, edited colonies and cloned animals (piglets and calves).**

| **Oligo** | **Sequence (5'-3')** | **Amplicon (bp)** | **Gene** | **sgRNA target (Exon)** | **Specie** |
|---|---|---|---|---|---|
| PCR-ssGGTA1 FW | GGACCTTGGGATCAGTCAGG SEQ ID NO:1 | 492 | GGTA1 | 3 | *S.scrofa* |
| PCR-ssGGTA1 RV | CGGCAACTCTCTGGAATGC SEQ ID NO:2 | | | | |
| PCR-ssGGTA1 FW2 | GTAGACCTAGGAAACGGATGC SEQ ID NO:3 | 894 | GGTA1 | 3 | *S.scrofa* |
| PCR-ssGGTA1 RV2 | CGGTATTTAAGGGCTCAGG SEQ ID NO:4 | | | | |
| PCR-ssCMAH FW | CCTTTCATGAGTTTTGTGTTCAACC SEQ ID NO:11 | 327 | CMAH | 4 | *S.scrofa* |
| PCR-ssCMAH RV | AATCAGAATGCATCCAAGGCTTC SEQ ID NO:12 | | | | |
| PCR-ssCMAH FW2 | CCCTATCTCCAACTATCACATCC SEQ ID NO:13 | 2105 | CMAH | 4 | *S.scrofa* |
| PCR-ssCMAH RV2 | TGGTCAATTGTCCTCTCACCTACACA SEQ ID NO:14 | | | | |
| PCR-ssB4GNT2 FW | CGCAAGTGACCAGACATCGTTCCC SEQ ID NO:21 | 465 | B4GNT2 | 2 | *S.scrofa* |
| PCR-ssB4GNT2RV | CACCGTAGAAATCGGGTTTCTCAGG SEQ ID NO:22 | | | | |
| PCR-ssB4GNT2 FW2 | CGCAAGTGACCAGACATCGTTC SEQ ID NO:23 | 471 | B4GNT2-like | 2 | *S.scrofa* |
| PCR-ssB4GNT2 RV2 | AGGTGAAGAGGCTCCTGACATG SEQ ID NO:24 | | | | |
| PCR-btGGTA1 FW | GGATGCCTTTGATAGAGTTGG SEQ ID NO:33 | 440 | GGTA1 | 9 | *B. taurus* |
| PCR-btGGTA1 RV | GCTTTCATCATGCCATTGG SEQ ID NO:34 | | | | |
| PCR-btGGTA1 FW2 | AGCATCTTTCACAACTCAGG SEQ ID NO:35 | 739 | GGTA1 | 4 | *B. taurus* |
| PCR-btGGTA1 RV2 | TGAGACATTAGGAACATGGC SEQ ID NO:36 | | | | |
| PCR-btCMAH FW | TCAGGAGGAGACATCACCAACGG SEQ ID NO:42 | 225 | CMAH | 2 | *B. taurus* |
| PCR-btCMAH RV | TGCCCATCCTACTTGTCGAGGG SEQ ID NO:43 | | | | |

**Table 2. Summary of mutations (Insertions/deletions) detected in cloned T-KO (triple-knock out)/D-KO (double knock-out) piglets.**

| **ID N°** | **GGTA1** | **CMAH** | **B4GalNT2** | **B4GalNT2-like** |
|---|---|---|---|---|
| Pig745 (colony #U3) **T-KO** | del | del | del AAAGCGTGTTCC (-12bp) | del GAAGC + in AAA (-2bp) |
| | TATTCCACTCTG GGTGTATTTAAT CTAATTTTCCCT TCTCCTTTTCTT TTCCCAGGAGAA AATAATGAATGT CAAAGGAAGAGT GGTTCTGTCAAT GCTGCTTGTCTC A (-109 bp) SEQ ID NO:91 | TCCTCCTGGAAGCTTCTGTC AA (-22bp) SEQ ID NO:99 | | |
| | | | SEQ ID NO:106 | SEQ ID NO:114 |
| | | | | |
| | | | del AAAGCGTGTTCC TC + GAAG (-10bp) SEQ ID NO:107 | WT SEQ ID NO:31 |
| Pig746 Pig748 Pig750 (colony #O2) **T-KO** | in GT (+2 bp) SEQ ID NO:92 | del CTTCTG (-6bp) SEQ ID NO:100 | del AAAGCGTGTTCC (-12 bp) | del GAAGCGTGTTCC (-12 bp) |
| | | | SEQ ID NO:108 | SEQ ID NO:115 |
| | in | | | |
| | TAGGGGTTCCGC GCACATTTCCCC GAAAAGTGCCAC CTAAATTGTAAG CGTTAATATTTT GTTAAAATTCGC GTTAAATTTTTG TTAAATCAGCTC ATTTTTTAACCA ATAGGCCGAAAT CGGCAAAATCCC TTATAAATCAAA AGAATAGACCGA GATAGGGCTGGC CCCAGCGCTGCG ATGATACCGCGA GAACCACGCTCA CCGGCTCCGGAT TTATCAGCAATA AACCAGCCAGCC GGAAGGGCCGAG CGCAGAAGTGGT CCTGCGACTTTA TCCGCCTCCATC CAGTCTATTAAT TGTTGCCGGGAA GCTAGAGTAAGT AGTTCGCCAGTT AATAGTTTGCGC AACGTTGTTGCC ATCGCTACAGGC (+372) SEQ ID NO:93 | del TCT (-3bp) SEQ ID NO:101 | del CGTGTTCCTCGATA + in GATC (-10 bp) SEQ ID NO:109 | del TGTGTTCCTCGATA + in GATC (-10 bp) SEQ ID NO:116 |
| Pig747 | del T (-1 bp) | del | del AAAGCGTGTTCC | del GAAGCGTGTTCC |
| Pig749 (colony #N1) **D-KO** | SEQ ID NO:94 | TTAAAAAAAAAAACAAATTT TAAGGGCTGCACCCATAGCA TATAGAAGTTCCTAGGCTAG CGGTCAAATTAGAGCTGCAG CTGCCAGCCTATGCCACAGCC ACAGCAATGCCAGATCTGAG CTGCATCTGTGACCTACACT GCAGCTTGCAGCAATGCTGG ATCCTTAACCCATTGAACAA TGCCAGGGATTGAACACACA TCCTCATGGATACTGCTCAG GTTCCTAACCTGCTGAGCCA CAGTTGGAACTCCAAAGCAG ACTTTATTCTGATGGCTCTG CTGATCTCTAACACGTTATT TTGTGCCATGGTGTTTATCT TCACTTTACTCAAGTCAGGG AAACACGAAGAGTCTCATAC AGGATAAACCCAAGGAGAAA TGTGCAAAGTCACATACAAA TCAAACTGACAAAAATCAAA TACAAGGAAAAAATATCTTC ACTTTCAAAATCACCTACTG ATGATGAGTTTATATTTCCT TGGATATTTGAATATTAGCT ATTTTTTTCCTTTCATGAGT TTTGTGTTCAACCAACTACA GTCGTTTACTTTGATCACAG AATAATGCATTTAAGCCTTA AATAGATTAATATTTATTTT CACCATTTCATAAACCTAAG TACAATTTTCCATCCAGGTC TGTTAAATGCACAAAACACA ACTGGAAGTTAGATGTAAGC AGCATGAAGTATATCAATCC TCCTGGAAGCTTCT (-715 bp) SEQ ID NO:102 | (-9 bp) SEQ ID NO:110 | (-12bp) SEQ ID NO:117 |
| | | | | |
| | | | del AAAGT + in GAAGT (Q29R) | WT |
| | | | | SEQ ID NO:31 |
| | | | SEQ ID NO:111 | |
| | | | | |
| Fetus-1 (colony #2E1) **D-KO** | del | del | in TG (+2 bp) | del C + in TT (+1 bp) |
| | CAACTTTTCCTT TTAGAAAAGATA TTGGTATAAGCA CTTCTTAAAAAA CCATATTCCACT CTGGGTGTATTT AATCTAATTTTC CCTTCTCCTTTT CTTTTCCCAGGA GAAAATAATGAA TGTCAAAGGAAG AGTGGTTCTGTC AATGCTGCTTGT CTCAACTGTAAT GGTTGTGTTTTG GGAATACATCAA | TAAATGCACAAAACACAACT GGAAGTTAGATGTAAGCAGC ATGAAGTATATCAATCCTCC TGGAAGCTTCTGT (-73 bp) SEQ ID NO:103 | SEQ ID NO:112 | SEQ ID NO:118 |
| | | | | |
| | | | WT | |
| | | | SEQ ID NO:30 | |
| | | | | |
| | | del AGCTT + in G (-4 bp) | | |
| | | SEQ ID NO:104 | | |
| | CAGGTAATTATG AAACATGATGAA ATGATGTTGATG AAAGTCTCCTCT AATCTCC + in A (-246bp) SEQ ID NO:95 | | | |
| | del | | | |
| | TTTCCTTTTAGA AAAGATATTGGT ATAAGCACTTCT TAAAAAACCATA TTCCACTCTGGG TGTATTTAATCT AATTTTCCCTTC TCCTTTTCTTTT CCCAGGAGAAAA TAATGAATGTCA AAGGAAGAGTGG TTCTGTCAATGC T (-145 bp) SEQ ID NO:96 | | | |
| Fetus-2 (colony #3C8) T-KO | del T (-1 bp) | del TT (-2 bp) | del AAAGCGTGTTCC (-12 bp) | del |
| | SEQ ID NO:97 | SEQ ID NO:105 | | GAAGCGTGTTCCTCGA TACAGACTTCAGACTT CAGTCTCCTCAACTCA CCC (-44 bp) SEQ ID NO:119 |
| | | | SEQ ID NO:113 | |
| | del CTGTC (-5 bp) | | | |
| | SEQ ID NO:98 | | | |
| | | | | WT |
| | | | | SEQ ID NO:31 |

**Table 3. Summary of mutations (Insertions/deletions) detected in cloned D-KO calves.**

| **ID N°** | **GGTA1** | **CMAH** |
|---|---|---|
| 9161 | del AGACCCTGGGCGAGTCGGTGG (-21 bp) | del GGCAAGTGAGGGAGGCA (-17 bp) |
| | SEQ ID NO:120 | SEQ ID NO:126 |
| | del | |
| | GTGTTTAAGATCAAGCCTGAGAAGAGGTGGCAGG ACATCAGCATGATGCGCATGAAGACTATCGGGGA GCACATTGTGGCCCACATCCAGCATGAGGTTGACT TCCTTTTCTGCATGGATGTGGACCAGGTCTTCCAA GACAAGTTTGGGGTGGAGACCCTGGGCGAGTCG (-171 bp) SEQ ID NO:121 | |
| 9162 | del AGTCGGTG (-8 bp) SEQ ID NO:122 | Substitution: ATG (START) to TAA (STOP) SEQ ID NO:127 |
| | | |
| | | del AGTGAGGGAGGCA (-13 bp) SEQ ID NO:128 |
| Fetus 2180 | del GCGAGTCGGTGGCCCAGCTACAAGCCTGG (- 29 bp) | Substitution: ATG (START) to TAA (STOP) SEQ ID NO:129 |
| | SEQ ID NO:123 | |
| | | in CCATTCTTGTGAAATACCCAGGGAGAGGCAAC GACGGACTTAAG (+44 bp) SEQ ID NO:130 |
| Fetus 2197 | del GAGTCGGTGGCCCAGC (-16 bp) | del AGTGAGGGAGGCA (-13 bp) |
| | SEQ ID NO:124 | SEQ ID NO:131 |
| | | |
| | | in T (+1 bp) |
| | del TCGGTGGCCCAG (-12 bp) | SEQ ID NO:132 |
| | SEQ ID NO:125 | |

**Table 4. List of oligos used for the assembly of desired CRISPR/Cas9 expression vectors.**

| **Oligo name** | **Sequence (5'-3')** | **sgRNAs targeting sequence (5'-3'; without PAM)** | **Gene (specie)** |
|---|---|---|---|
| **sB4GNT2cr1 FW** | CACCGCTGTATCGAGGAACACGCTT SEQ ID NO:133 | CTGTATCGAGGAACACGCTT | B4GalNT2 and B4GalNT2-like (*Sus scrofa*) |
| **sB4GNT2cr1 RV** | AAACAAGCGTGTTCCTCGATACAGC SEQ ID NO:134 | | |
| **sB4GNT2cr2 FW** | CACCGACATAAAGAGTCCAACGCTC SEQ ID NO:135 | ACATAAAGAGTCCAACGCTC | B4GalNT2 and B4GalNT2-like (*Sus scrofa*) |
| **sB4GNT2cr2 RV** | AAACGAGCGTTGGACTCTTTATGTC SEQ ID NO:136 | | |
| **sB4GNT2cr3 FW** | CACCGCTTCAGCGTCTGCGCATCCA SEQ ID NO:137 | CTTCAGCGTCTGCGCATCCA | B4GalNT2 and B4GalNT2-like (*Sus scrofa*) |
| **sB4GNT2cr3 RV** | AAACTGGATGCGCAGACGCTGAAGC SEQ ID NO:138 | | |
| **btGGTA1cr1 FW** | CACCGGTGGAGACCCTGGGCGAGT SEQ ID NO:139 | GGTGGAGACCCTGGGCGAGT | GGTA1 (*Bos taurus*) |
| **btGGTA1cr1 RV** | AAACACTCGCCCAGGGTCTCCACC SEQ ID NO:140 | | |
| **btGGTA1cr2 FW** | CACCGCTACAAGCCTGGTGGTACA SEQ ID NO:141 | GCTACAAGCCTGGTGGTACA | GGTA1 (*Bos taurus*) |
| **btGGTA1cr2 RV** | AAACTGTACCACCAGGCTTGTAGC SEQ ID NO:142 | | |
| **btGGTA1cr3 FW** | CACCGTACCACCAGGCTTGTAGCT SEQ ID NO:143 | GTACCACCAGGCTTGTAGCT | GGTA1 (*Bos taurus*) |
| **btGGTA1cr3 RV** | AAACAGCTACAAGCCTGGTGGTAC SEQ ID NO:144 | | |
| **btGGTA1cr4 FW** | CACCGACACTTCTTTTCTTACAAT SEQ ID NO:145 | GACACTTCTTTTCTTACAAT | GGTA1 (*Bos taurus*) |
| **btGGTA1cr4 RV** | AAACATTGTAAGAAAAGAAGTGTC SEQ ID NO:146 | | |
| **btGGTA1cr5 FW** | CACCGGTTTTCGCCGTCGGAAGGT SEQ ID NO:147 | GGTTTTCGCCGTCGGAAGGT | GGTA1 (*Bos taurus*) |
| **btGGTA1cr5 RV** | AAACACCTTCCGACGGCGAAAACC SEQ ID NO:148 | | |
| **btGGTA1cr6 FW** | CACCGGAGACCCTGGGCGAGTCGG SEQ ID NO:149 | GGAGACCCTGGGCGAGTCGG | GGTA1 (*Bos taurus*) |
| **btGGTA1cr6 RV** | AAACCCGACTCGCCCAGGGTCTCC SEQ ID NO:150 | | |
| **btGGTA1cr7 FW** | CACCGCTGGGCCACCGACTCGCCC SEQ ID NO:151 | GCTGGGCCACCGACTCGCCC | GGTA1 (*Bos taurus*) |
| **btGGTA1cr7 RV** | AAACGGGCGAGTCGGTGGCCCAGC SEQ ID NO:152 | | |
| **btGGTA1cr8 FW** | CACCGGTCTTCCAAGACAAGTTTG SEQ ID NO:153 | GGTCTTCCAAGACAAGTTTG | GGTA1 (*Bos taurus*) |
| **btGGTA1cr8 RV** | AAACCAAACTTGTCTTGGAAGACC SEQ ID NO:154 | | |
| **btGGTA1cr9 FW** | CACCGGGATCTGCCTTGTACCACC SEQ ID NO:155 | GGGATCTGCCTTGTACCACC | GGTA1 (*Bos taurus*) |
| **btGGTA1cr9 RV** | AAACGGTGGTACAAGGCAGATCCC SEQ ID NO:156 | | |
| **btGGTA1cr10 FW** | CACCGGTCCACATCCATGCAGAAA SEQ ID NO:157 | GGTCCACATCCATGCAGAAA | GGTA1 (*Bos taurus*) |
| **btGGTA1cr10 RV** | AAACTTTCTGCATGGATGTGGACC SEQ ID NO:158 | | |
| **btGGTA1cr11 FW** | CACCGGTTGACTTCCTTTTCTGCA SEQ ID NO:159 | GGTTGACTTCCTTTTCTGCA | GGTA1 (*Bos taurus*) |
| **btGGTA1cr11 RV** | AAACTGCAGAAAAGGAAGTCAACC SEQ ID NO:160 | | |
| **btGGTA1cr12 FW** | CACCGTCAACCTCATGCTGGATGT SEQ ID NO:161 | GTCAACCTCATGCTGGATGT | GGTA1 (*Bos taurus*) |
| **btGGTA1cr12 RV** | AAACACATCCAGCATGAGGTTGAC SEQ ID NO:162 | | |
| **btGGTA1cr13 FW** | CACCGACTATCGGGGAGCACATTG SEQ ID NO:163 | GACTATCGGGGAGCACATTG | GGTA1 (*Bos taurus*) |
| **btGGTA1cr13 RV** | AAACCAATGTGCTCCCCGATAGTC SEQ ID NO:164 | | |
| **btGGTA1cr14 FW** | CACCGATGCGCATGAAGACTATCG SEQ ID NO:165 | GATGCGCATGAAGACTATCG | GGTA1 (*Bos taurus*) |
| **btGGTA1cr14 RV** | AAACCGATAGTCTTCATGCGCATC SEQ ID NO:166 | | |
| **btGGTA1cr15 FW** | CACCGCATACATTCCCTTCGGCGAA SEQ ID NO:167 | CATACATTCCCTTCGGCGAA | GGTA1 (*Bos taurus*) |
| **btGGTA1cr15 RV** | AAACTTCGCCGAAGGGAATGTATGC SEQ ID NO:168 | | |
| **btGGTA1cr16 FW** | CACCGATAAAAATCCCCTTCGCCGA SEQ ID NO:169 | ATAAAAATCCCCTTCGCCGA | GGTA1 (*Bos taurus*) |
| **btGGTA1cr16 RV** | AAACTCGGCGAAGGGGATTTTTATC SEQ ID NO:170 | | |
| **btGGTA1cr17 FW** | CACCGATACATTCCCTTCGGCGAAG SEQ ID NO:171 | ATACATTCCCTTCGGCGAAG | GGTA1 (*Bos taurus*) |
| **btGGTA1cr17 RV** | AAACCTTCGCCGAAGGGAATGTATC SEQ ID NO:172 | | |
| **btGGTA1cr18 FW** | CACCGCATACATTCCCTTCGGCGA SEQ ID NO:173 | GCATACATTCCCTTCGGCGA | GGTA1 (*Bos taurus*) |
| **btGGTA1cr18 RV** | AAACTCGCCGAAGGGAATGTATGC SEQ ID NO:174 | | |
| **btGGTA1cr19 FW** | CACCGTAAAAATCCCCTTCGCCGAA SEQ ID NO:175 | TAAAAATCCCCTTCGCCGAA | GGTA1 (*Bos taurus*) |
| **btGGTA1cr19 RV** | AAACTTCGGCGAAGGGGATTTTTAC SEQ ID NO:176 | | |
| **btGGTA1cr20 FW** | CACCGCAACTGTCATTGTTGTGTTT SEQ ID NO:177 | CAACTGTCATTGTTGTGTTT | GGTA1 (*Bos taurus*) |
| **btGGTA1cr20 RV** | AAACAAACACAACAATGACAGTTGC SEQ ID NO:178 | | |
| **btGGTA1cr21 FW** | CACCGCTTCCGAGATGGTTTAACAA SEQ ID NO:179 | CTTCCGAGATGGTTTAACAA | GGTA1 (*Bos taurus*) |
| **btGGTA1cr21 RV** | AAACTTGTTAAACCATCTCGGAAGC SEQ ID NO:180 | | |
| **btGGTA1cr22 FW** | CACCGCAAGCTTAAGCTATCGGAC SEQ ID NO:181 | GCAAGCTTAAGCTATCGGAC | GGTA1 (*Bos taurus*) |
| **btGGTA1cr22 RV** | AAACGTCCGATAGCTTAAGCTTGC SEQ ID NO:182 | | |
| **btGGTA1cr23 FW** | CACCGAGAAAATAATGAATGTCAA SEQ ID NO:183 | GAGAAAATAATGAATGTCAA | GGTA1 (*Bos taurus*) |
| **btGGTA1cr23 RV** | AAACTTGACATTCATTATTTTCTC SEQ ID NO:184 | | |
| **btCMAHcr1 FW** | CACCGGCAACGACAGACTATGGGC SEQ ID NO:185 | GGCAACGACAGACTATGGGC | CMAH (*Bos taurus*) |
| **btCMAHcr1 RV** | AAACGCCCATAGTCTGTCGTTGCC SEQ ID NO:186 | | |
| **btCMAHcr2 FW** | CACCGCCAAATCTTCAGGAGATCT SEQ ID NO:187 | GCCAAATCTTCAGGAGATCT | CMAH (*Bos taurus*) |
| **btCMAHcr2 RV** | AAACAGATCTCCTGAAGATTTGGC SEQ ID NO:188 | | |
| **btCMAHcr3 FW** | CACCGGTGTTTGACCCTTGGTTAA SEQ ID NO:189 | GGTGTTTGACCCTTGGTTAA | CMAH (*Bos taurus*) |
| **btCMAHcr3 RV** | AAACTTAACCAAGGGTCAAACACC SEQ ID NO:190 | | |
| **btCMAHcr4 FW** | CACCGCCATTCTTCTGAAATACCC SEQ ID NO:191 | GCCATTCTTCTGAAATACCC | CMAH (*Bos taurus*) |
| **btCMAHcr4 RV** | AAACGGGTATTTCAGAAGAATGGC SEQ ID NO:192 | | |
| **btCMAHcr5 FW** | CACCGACTATGGGCAGGCAAGTGA SEQ ID NO:193 | GACTATGGGCAGGCAAGTGA | CMAH (*Bos taurus*) |
| **btCMAHcr5 RV** | AAACTCACTTGCCTGCCCATAGTC SEQ ID NO:194 | | |
| **btCMAHcr6 FW** | CACCGATCTGGGCTCTGAATCCCA SEQ ID NO:195 | GATCTGGGCTCTGAATCCCA | CMAH (*Bos taurus*) |
| **btCMAHcr6 RV** | AAACTGGGATTCAGAGCCCAGATC SEQ ID NO:196 | | |
| **btCMAHcr7 FW** | CACCGCCCAGATCTCCTGAAGATT SEQ ID NO:197 | GCCCAGATCTCCTGAAGATT | CMAH (*Bos taurus*) |
| **btCMAHcr7 RV** | AAACAATCTTCAGGAGATCTGGGC SEQ ID NO:198 | | |
| **btCMAHcr8 FW** | CACCGAGAGGCAACGACAGACTAT SEQ ID NO:199 | GAGAGGCAACGACAGACTAT | CMAH (Bos *taurus*) |
| **btCMAHcr8 RV** | AAACATAGTCTGTCGTTGCCTCTC SEQ ID NO:200 | | |
| **btCMAHcr9 FW** | CACCGTTAACATGGACACCGCGACC SEQ in ID NO:201 | GTTAACATGGACACCGCGACC | CMAH (*Bos taurus*) |
| **btCMAHcr9 RV** | AAACGGTCGCGGTGTCCATGTTAAC SEQ ID NO:202 | | |
| **btCMAHcr10 FW** | CACCGACATATTCTTGCATGCTCGC SEQ ID NO:203 | ACATATTCTTGCATGCTCGC | CMAH (Bos *taurus*) |
| **btCMAHcr10 RV** | AAACGCGAGCATGCAAGAATATGTC SEQ ID NO:204 | | |
| **btB4GNT2cr1 FW** | CACCGCCCAGGGCCAAGAATAAGA SEQ ID NO:205 | GCCCAGGGCCAAGAATAAGA | B4GalNT2 (*Bos taurus*) |
| **btB4GNT2cr1 RV** | AAACTCTTATTCTTGGCCCTGGGC SEQ ID NO:206 | | |
| **btB4GNT2cr2 FW** | CACCGCCGGACTAGGCTGAAGGAG SEQ ID NO:207 | GCCGGACTAGGCTGAAGGAG | B4GalNT2 (*Bos taurus*) |
| **btB4GNT2cr2 RV** | AAACCTCCTTCAGCCTAGTCCGGC SEQ ID NO:208 | | |
| **btB4GNT2cr3 FW** | CACCGGGTCTTGATATGTTCCTCA SEQ ID NO:209 | GGGTCTTGATATGTTCCTCA | B4GalNT2 (*Bos taurus*) |
| **btB4GNT2cr3 RV** | AAACTGAGGAACATATCAAGACCC SEQ ID NO:210 | | |
| **btB4GNT2cr4 FW** | CACCGATTCCATCGTGGGTAAAGA SEQ ID NO:211 | GATTCCATCGTGGGTAAAGA | B4GalNT2 (*Bos taurus*) |
| **btB4GNT2cr4 RV** | AAACTCTTTACCCACGATGGAATC SEQ ID NO:212 | | |
| **btB4GNT2cr5 FW** | CACCGTCCGTCTTATTCTTGGCCC SEQ ID NO:213 | GTCCGTCTTATTCTTGGCCC | B4GalNT2 (*Bos taurus*) |
| **btB4GNT2cr5 RV** | AAACGGGCCAAGAATAAGACGGAC SEQ ID NO:214 | | |
| **btB4GNT2cr6 FW** | CACCGAACATTCCGGCAGCCGGACT SEQ ID NO:215 | AACATTCCGGCAGCCGGACT | B4GalNT2 (*Bos taurus*) |
| **btB4GNT2cr6 RV** | AAACAGTCCGGCTGCCGGAATGTTC SEQ ID NO:216 | | |
| **btB4GNT2cr7 FW** | CACCGTCTCACCAGATTCCATCGT SEQ ID NO:217 | GTCTCACCAGATTCCATCGT | B4GalNT2 (*Bos taurus*) |
| **btB4GNT2cr7 RV** | AAACACGATGGAATCTGGTGAGAC SEQ ID NO:218 | | |
| **btB4GNT2cr8 FW** | CACCGAGCTTCAACATTCCGGCAGC SEQ ID NO:219 | AGCTTCAACATTCCGGCAGC | B4GalNT2 (*Bos taurus*) |
| **btB4GNT2cr8 RV** | AAACGCTGCCGGAATGTTGAAGCTC SEQ ID NO:220 | | |
| **btB4GNT2cr9 FW** | CACCGCTTCAGCCTAGTCCGGCTGC SEQ ID NO:221 | CTTCAGCCTAGTCCGGCTGC | B4GalNT2 (*Bos taurus*) |
| **btB4GNT2cr9 RV** | AAACGCAGCCGGACTAGGCTGAAGC SEQ ID NO:222 | | |
| **ecGGTA1cr1 FW** | CACCGAGCGTATATACCTATTGGTC SEQ ID NO:223 | AGCGTATATACCTATTGGTC | GGTA1 (*Equus caballus*) |
| **ecGGTA1cr1 RV** | AAACGACCAATAGGTATATACGCTC SEQ ID NO:224 | | |
| **ecGGTA1cr2 FW** | CACCGTCGGAAGCGTATATACCTAT SEQ ID NO:225 | TCGGAAGCGTATATACCTAT | GGTA1 (*Equus caballus*) |
| **ecGGTA1cr2 RV** | AAACATAGGTATATACGCTTCCGAC SEQ ID NO:226 | | |
| **ecGGTA1cr3 FW** | CACCGCGTATATACCTATTGGTCA SEQ ID NO:227 | GCGTATATACCTATTGGTCA | GGTA1 (*Equus caballus*) |
| **ecGGTA1cr3 RV** | AAACTGACCAATAGGTATATACGC SEQ ID NO:228 | | |
| **ecGGTA1cr4 FW** | CACCGATATGACACTTTCTATGTA SEQ ID NO:229 | GATATGACACTTTCTATGTA | GGTA1 (*Equus caballus*) |
| **ecGGTA1cr4 RV** | AAACTACATAGAAAGTGTCATATC SEQ ID NO:230 | | |
| **ecCMAHcr1 FW** | CACCGTATTCTTGCACGCTCGCAGG SEQ ID NO:231 | TATTCTTGCACGCTCGCAGG | CMAH (*Equus caballus*) |
| **ecCMAHcr1 RV** | AAACCCTGCGAGCGTGCAAGAATAC SEQ ID NO:232 | | |
| **ecCMAHcr2 FW** | CACCGCGGAGGGTTGATATACTTCA SEQ ID NO:233 | CGGAGGGTTGATATACTTCA | CMAH (*Equus caballus*) |
| **ecCNAHcr2 RV** | AAACTGAAGTATATCAACCCTCCGC SEQ ID NO:234 | | |
| **ecCMAHcr3 FW** | CACCGTCCCTCCAAATACCTAGGCT SEQ ID NO:235 | TCCCTCCAAATACCTAGGCT | CMAH (*Equus caballus*) |
| **ecCMAHcr3 RV** | AAACAGCCTAGGTATTTGGAGGGAC SEQ ID NO:236 | | |
| **ecB4GNT2cr1 FW** | CACCGGCTATTGAACTGTGCGAGA SEQ ID NO:237 | GGCTATTGAACTGTGCGAGA | B4GNT2 (*Equus caballus*) |
| **ecB4GNT2cr1 RV** | AAACTCTCGCACAGTTCAATAGCC SEQ ID NO:238 | | |
| **ecB4GNT2cr2 FW** | CACCGAAGTTGTCAGTCTTAATCGC SEQ ID NO:239 | AAGTTGTCAGTCTTAATCGC | B4GNT2 |
| **ecB4GNT2cr2 RV** | AAACGCGATTAAGACTGACAACTTC SEQ ID NO:240 | | |
| **ecB4GNT2cr3 FW** | CACCGCTATTGAACTGTGCGAGAAG SEQ ID NO:241 | CTATTGAACTGTGCGAGAAG | B4GNT2 (*Equus caballus)* |
| **ecB4GNT2cr3 RV** | AAACCTTCTCGCACAGTTCAATAGC SEQ ID NO:242 | | |
| **ecB4GNT2cr4 FW** | CACCGCTATTGAACTGTGCGAGAA SEQ ID NO:243 | GCTATTGAACTGTGCGAGAA | B4GNT2 (*Equus caballus)* |
| **ecB4GNT2cr4 RV** | AAACTTCTCGCACAGTTCAATAGC SEQ ID NO:244 | | |
| **ecB4GNT2cr5 FW** | CACCGTCAGTCTTAATCGCAGGACT SEQ ID NO:245 | TCAGTCTTAATCGCAGGACT | B4GNT2 (*Equus caballus)* |
| **ecB4GNT2cr5 RV** | AAACAGTCCTGCGATTAAGACTGAC SEQ ID NO:246 | | |

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

### Example 1. DNA Sequencing Analysis of selected target genomic regions for porcine (GGTA1, CMAH. B4GalNT2 and B4GalNT2-like) and bovine (GGTA1 and CMAH) genome editing.

Primary fibroblasts and tissues biopsies were lysed at 55°C for 3 hours using the lysis buffer (100 mM Tris HCl pH 8.3, 5 mM EDTA pH 8.1, 0.2% SDS, 200 mM NaCl) supplemented with Proteinase K (300 µg/ml Macherey-Nagel, Germany). Genomic DNA was extracted (Sambrook et al., 1989) and finally resuspended with 1X TE buffer (200 µl). PCR amplifications of the GGTA1, CMAH and B4GalNT2 target regions were performed using gene specific primers (Table 1). All the amplifications were performed in 12.5 µL reaction volume (0.4 mM d NTPs, 0.8 µM oligonucleotides, 0.05 U/µl LA-Taq in GCI Buffer, Nuclease-free H₂O to volume) using the LA-Taq enzyme (Takara, Japan).

In Table 1 the sequences of used primers and the expected PCR products for the target genes are summarised.

PCR cycle conditions for porcine *GGTA1 gene* were as follows. For PCR-ssGGTA1 FW+RV (492 bp): 94°C 2 min; 94°C 30 sec, 60°C 30 sec, 72°C 30 sec for 40 cycles; and a final extension step at 72°C for 7 min. For PCR-ssGGTA1 FW2+RV2 (894bp) 94°C 2 min; 94°C 30 sec, 55°C 30 sec, 72°C 1 min for 35 cycles; and a final extension step at 72°C for 7 min.

For the porcine *CMAH gene* PCR cycle conditions were as follows. For PCR-ssCMAH FW+RV (327 bp): 94°C 2 min; 94°C 30 sec, 60°C 30 sec, 72°C 30 sec for 40 cycles; and a final extension step at 72°C for 7 min; using PCR-ssCMAH FW2+RV2 (2104bp) 94°C 2 min; 94°C 30 sec, 63°C 30 sec, 72°C, 2 min for 35 cycles; and a final extension step at 72°C for 7 min.

PCR cycle condition for the porcine *B4GalNT2 gene* were as follows (PCR-ssB4GNT2 FW+RV = 465 bp): 94°C 2 min; 94°C 30 sec, 60°C 30 sec, 72°C 30 sec for 35 cycles; and a final extension step at 72°C for 7 min.

PCR cycle condition for the porcine *B4GalNT2-like gene* were as follows (PCR-ssB4GNT2 FW2+RV2 = 471 bp): 94°C 2 min; 94°C 30 sec, 60°C 30 sec, 72°C 30 sec for 35 cycles; and a final extension step at 72°C for 7 min.

PCR cycle conditions for the bovine *GGTA1* were as follows. For PCR-btGGTA1 FW+RV (440 bp): 94°C 2 min; 94°C 30 sec, 72°C (-1°C/cycle) 30 sec, 72°C 15 sec for 8 cycles; 94°C 30 sec, 58°C 30 sec, 72°C 15 sec for 35 cycles; and a final extension step at 72°C for 7 min. For PCR-btGGTA1 FW2+RV2 (739 bp): 94°C 2 min; 94°C 30 sec, 60°C 30 sec, 72°C 30 sec for 35 cycles; and a final extension step at 72°C for 7 min.

For the bovine *CMAH gene,* PCR cycle conditions were as follows (PCR-btCMAH FW+RV = 225 bp): 94°C 2 min; 94°C 30 sec, 58°C 30 sec, 72°C 30 sec for 40 cycles; and a final extension step at 72°C for 7 min.

To guarantee the absence of any genomic polymorphisms resulting PCR products were loaded onto a 2% TBE agarose gel to confirm presence and purity, cloned in *E.coli* using the Topo-TA cloning kit (Topo TA, Thermo Fisher Scientific, Waltham, MA, USA) and subjected for Sanger sequencing analyses (Eurofin Genomics, Ebersberg, Germany).

Examples of the sequencing data obtained for the porcine and for the bovine wild type targets, can be found in the Figures 1 and 2.

### Example 2 RT-PCR analyses for B4GalNT2-like gene

In this example the experimental reasons that obliged us to analyse two different genomic sequences coding for the porcine β1,4N-acetylgalactosaminyltransferase in order to guarantee its complete inactivation are described.

Starting from literature study we understood that in pig B4GalNT2 gene has a genomic organization different from the other mammals like human, mice.

In 2013 Byrne described and completely characterized the porcine B4GalNT2 gene, its transcript identifying (AK34612) and its genomic localization into the chromosome 12 (ENSSSCG00000040942). Two years after Estrada and colleagues successfully inactivated this gene with the CRISPR/Cas9 genome editing system. They registered as reference the transcript NM_001244330.1 updating and replacing the previous transcript (AK34612 - Byrne et al., 2014). But during the characterization of the *InDels* affecting their triple knockout clones, Estrada and colleagues introduced and used as reference gene a different genomic sequence that is registered as the B4GalNT2-like gene (ENSSSCG00000030269). This gene is included into the chromosome 12 as the B4GalNT2 gene, but it is shorter, and reverse-oriented to the B4GalNT2. It is about 65000 bp far from the B4GalNT2, and, even if it shares with the B4GalNT2 gene some regions with high homology, it's not an exact duplication, because finally its homology rate with the B4GNT2 gene is only 64.7%. The exact name of this sequence was never reported and none transcriptional analyses were presented for the B4GalNT2-like gene, omitting why it was decided to use this new genomic sequence. It was only mentioned that a B4GalNT2 gene duplication could happened in pig, finding a reliable explanation to the three different mutated B4GalNT2 allelic sequences detected in one of their alive triple KO pigs.

Analyzing the pig genes surrounding our targets and looking to their genomic disposition in other mammals (human, mice, goat, sheep, equine, bovine), we found that always the IGF2bp1 gene is positioned before the B4GalNT2 gene, with the same orientation, as happened in pig for the B4GalNT2-like gene. By the way, further gene specific PCR-analyses demonstrated us that both pig genes can be detected and sequenced, indeed in pig genome there is not an assembly mistake of two different genomic contiguous.

*In-silico* transcriptional analyses predicted different transcript variants for each gene (5 for the B4GalNT2 and 9 for the B4GalNT2-like), describing for the B4GalNT2-like transcripts a situation similar to the human homologs where different isoforms were described (short form, long form, soluble form - Dall'Olio et al. 2014).

Alignments of B4GalNT2 and B4GalNT2-like transcripts revealed a very high sequence homology with a single evident difference at the 3' terminus of the exon2. This exon is 189 bp long for the B4GalNT2 gene but it is 201 bp long for the B4GalnT2-like gene, as reported for the human homolog. Following alignments analyses of the proteins resulting from *in-silico* translations of the two different pig transcripts, that are homologs to the human transcript variant 2, revealed that the B4GalNT2-like isoform is more similar to the human one (76.7 % Vs 75.1%) but both pig isoforms conserved intact the aminoacidic domain (SQVTTKYVLWVDDDF = divalent cation binding domain) responsible for the transferase activity of the beta 1-4 galactosyltransferase enzyme (Byrne et al., 2013).

For all these reasons we decide to retrotranscribe the RNA extracted from a culture of PAEC where it is possible to detect the transcript coding for the Sd^{a}. Following RT-PCR analyses, focused on the exon2, confirmed us that two different gene-specific transcripts could be detected. Using the oligonucleotides RT-sB4GNT2long FW (SEQ ID NO: 25) and RT-sB4GNT2long RV (SEQ ID NO: 26) we were finally able to amplify and to sequence one complete mRNA (SEQ ID NO:32 MG675060) corresponding to the B4GalNT2-like transcript variant X5.

Considering all these data we thought that genomic characterization of *Indels* occurred into the B4GalNT2 and B4GalNT2-like genes and their consequential inactivation is needed to guarantee the complete enzyme inactivation to obtain the desired Sd^{a} negative phenotype.

### Example . 3 Design and test of Talens expression vectors for the porcine GGTA1 and CMAH genes.

Editing of the porcine GGTA1 gene was achieved using the Transcription activator-like effector nucleases (Talens). For each target region 3 different sets of Talens were studied and tested for their cutting efficiency. Desired expressing vectors for GGTA1 gene (3 sets of Talens specific for the exon 3, 5 and 7) were purchased from Genecopoeia (http://www.genecopoeia.com), instead for the CMAH gene, the Talens expressing vectors were generated in our laboratories considering the exons 4 and 5 as possible targets.

Expressing vectors were transiently transfected (Nucleofector, program V024 Amaxa, Lonza, basel, Switzerlad) into the porcine primary fibroblasts (1x10⁶), and, after 48 hours, resulting cells were lysed extracting their genomic DNA for following PCR analyses, whose operating conditions were previously described by the Example 1. Obtained PCR products were loaded onto an 3% TBE 1X agarose gel for confirming the presence and purity before to use 400 ng of each sample for following mutational assays (IDT Surveyor® Mutation Detection Kit, IA, USA), following manufacturer's protocol. Treated samples were finally loaded on an high melting/high resolution 3% TBE 1X agarose gel for cutting efficiency determination of each Talens set. We finally selected for the GGTA1 gene a set of Talens specific for the exon 3; for the CMAH gene a set of Talens specific for the exon 4.

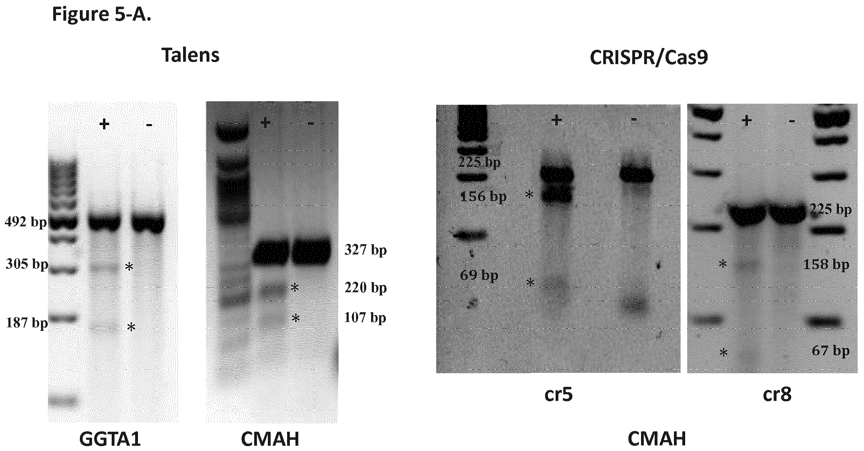

Figure 5-A presents the endonucleases activity results obtained for the final selection of Talens used for editing our porcine cell lines.

RVDs sequences of selected Talens are respectively described - to edit the GGTA1 gene - SEQ ID NO: 6, and SEQ ID NO: 8, and the CMAH gene - SEQ ID NO:16 and SEQ ID NO:18.

### Example. 4 Design and testing of CRISPR expression vectors for the porcine (B4GalNT2 and B4GalNT2-like), bovine (GGTA1, CMAH and B4GalNT2) and equine (GGTA1, CMAH and B4GalNT2) genes.

Expression of desired sgRNAs specific for the porcine (B4GalNT2 and B4GalNT2-like), bovine (GGTA1, CMAH and B4GalNT2) and equine (GGTA1, CMAH and B4GalNT2) genes_was achieved using the pX330-U6-Chimeric_BB-CBh-hSpCas9 expression vector that was a gift from Feng Zhang (Addgene plasmid # 42230). Annealing and cloning of gene specific oligos (see Table 4) for the expression of sgRNA recognizing their relative target sequences (SEQ ID NO: 27, SEQ ID NO: 28, SEQ ID NO: 29, SEQ ID NO: 37, SEQ ID NO: 38, SEQ ID NO: 39, SEQ ID NO: 44, SEQ ID NO: 45, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 64, SEQ ID NO: 65, SEQ ID NO: 66, SEQ ID NO: 67, SEQ ID NO: 75, SEQ ID NO: 76, SEQ ID NO: 77, SEQ ID NO: 85, SEQ ID NO: 86, SEQ ID NO: 87, SEQ ID NO: 88, SEQ ID NO: 89) were done following the protocol described by Cong et colleagues (Cong et al., 2013). Finally we generated 54 different expression vectors. After their endonuclease activity tests (see Example 2), finally 14 of them we selected and used in our editing experiments: 1 sgRNA for porcine B4GalNT2 and B4GalNT2-like genes (SEQ ID NO: 27); 3 sgRNAs for the bovine GGTA1 gene (SEQ ID NO: 37; SEQ ID NO: 38 and SEQ ID NO: 39); 2 sgRNA for the bovine CMAH gene (SEQ ID NO: 44 and SEQ ID NO: 45); 2 sgRNA for the bovine B4GNT2 gene (SEQ ID NO: 50 and SEQ ID NO: 51); 3 sgRNA for the equine GGTA1 gene (SEQ ID NO: 64, SEQ ID NO: 65 and SEQ ID NO: 66); 1 sgRNA for the equine CMAH gene (SEQ ID NO: 76); 2 sgRNAs for the equine B4GalNT2 gene (SEQ ID NO: 85 and SEQ ID NO: 86).

As example Figures 5-A and 5-B show the results obtained during the selection of sgRNAs targeting the bovine CMAH (6-A), the equine GGTA1 and the equine CMAH (6-B).

### Example 5. Production of Knockout Pigs (Triple edited pigs)

Primary fibroblast cell lines and colonies were cultured in TCM199/DMEM-F12, 1:1, + 20% FCS, + bFGF 82 ng/ml at 38.5°C in humidified atmosphere of 90% N₂, 5% O₂, and 5% CO₂. Pig adult fibroblasts at passage 1 to 3 thawed 2 days before transfection, were trypsinized, 1x10⁶ cells were resuspended in 100 µl of Nucleofector solution (Basic Nucleofector Kit, Prim. Fibroblasts; Amaxa, Lonza, Basel, Switzerland) and were Nucleofected (Nucleofector program V024, Amaxa, Lonza, Basel, Switzerland) contaiing: Talens expression vectors for the GGTA1 (2µg + 2µg); Talens expression vectors for the CMAH gene (2µg + 2µg); CRISPR expression vector for the B4GalNT2/B4GalNT2-like genes (1µg). Transfected cells were plated in 60 mm Petri dish and cultured for 6-7 days before IB4-selection of the Gal-KO cells with paramagnetic beads (Li et al., 2013). Resulting beads-selected cells (Gal-KO cells) were plated in 20 Petri dishes (ø = 150 mm; 100 cell/plate) and cultured for additional 8 days when the well growing colonies were picked-up and divided in two aliquots for genotyping and cryo-preservation.

Genotyping of colonies was done using the same materials and methods described in the Example 1. CMAH gene was analyses for all the colonies. Resulting PCR product were digested with *Hind*III and loaded onto a 2% TBE agarose gel for colony selection.. Undigested colonies after the RFLP analyses and characterized by an CMAH electrophoretic pattern different from wild type control were selected. At the end for these selected colonies the GGTA1, CMAH and B4GalNT2/B4GalNT2-like genes were genotyped by cloning the obtained PCR product in *E.coli* (Topo TA, Thermo Fisher Scientific, Waltham, MA, USA) and subsequent Sanger-sequencing analyses (Eurofin Genomics, Ebersberg, Germany). Only colonies that resulted knocked out at least for the GGTA1 and CMAH and that had inactivated at least one of the B4GalNT2/B4GalNT2-like genes were used as nuclear donors for the somatic cell nuclear transfer procedure, described in the Example 8. Two pregnancies went to term delivering 4 alive (Pig 746, Pig 747, Pig 748 and Pig 750) and 2 stillborn piglets (Pig 745 and Pig 749) and one pregnancy was lost. Following analyses demonstrated that 3 piglets are clones of the colony #O2 (Pig746, Pig748 and Pig750) and that Pig747 and Pig749 (stillborn) derived from colony #N1. From colony #U3 we obtained only the stillborn animal Pig745.

Figures 1 (from A to D) shows DNA sequencing results generated for Pig 746, where target genes were disrupted by mutations described as follows: GT insertion (SEQ ID NO:92) for the GGTA1 gene (Figure 1-A); CTTCTG deletion (SEQ ID NO:100)/TCT deletion (SEQ ID NO:101) for the CMAH gene (Figure 1-B); AAAGCGTGTTCC deletion (SEQ ID NO:108)/CGTGTTCCTCGATA deletion + GATC insertion (SEQ ID NO:109) for the B4GalNT2 gene (Figure 1-C); GAAGCGTGTTCC deletion (SEQ ID NO:115)/TGTGTTCCTCGATA deletion + GATC insertion (SEQ ID NO:116)for the B4GalNT2-like (Figure 1-D). Table 2 summaries the sequencing results obtained on all piglets, including also the *Indels* characterization of two aborted fetuses.

### Example 6. Production of Knockout Bovines (Double edited bovines)

Primary fibroblasts cell lines and colonies were cultured in TCM199/DMEM-F12, 1:1, + 20% FCS, at 38.5°C in humidified atmosphere of 90% N₂, 5% O₂, and 5% CO₂. Bovine adult fibroblasts at passage 1 to 3 thawed 2 days before transfection, were trypsinized, 2x10⁶ cells were resuspended in 100 µl of Nucleofector solution (Basic Nucleofector Kit, Prim. Fibroblasts; Amaxa, Lonza, Basel, Switzerland) contaiing: CRISPR expression vectors for the GGTA1 gene (2µg+2µg); CRISPR expression vector for the CMAH gene (2µg); btCMAH-STOP ssODN (400 pmol) and Nucleofected (Nucleofector program V024, Amaxa, Lonza, Basel, Switzerland). Transfected cells were plated in 60 mm Petri dish and cultured for 7 days before IB4-selection of the Gal-KO cells with paramagnetic beads (Li et al., 2013). Resulting beads-selected cells (Gal-KO cells) were plated in 20 Petri dishes (ø = 150 mm; 100 cell/plate) and cultured for additional 8 days when the well growing colonies were picked-up and divided in two aliquots for genotyping and cryo-preservation.

Genotyping of colonies was done using the same materials and methods described in the Example 1. Firstly we analysed the CMAH gene in all the lysed colonies. PCR products were loaded onto a 2% TBE agarose gel for detection of bands different from the wild type pattern. After all the colonies were RFLP analysed (*Af*/II) to detect colonies where at least one allele was targeted with our single strand oligo. The colonies that resulted digested to the RFLP analyses or that presented a modified electrophoresis pattern, were genotyped to characterize the mutations occurred also into their CMAH and GGTA1 genes, following the same procedure described into the Example2. Only colonies that resulted knocked out for both GGTA1 and CMAH genes were used as nuclear donors for the somatic cell nuclear transfer procedure, described in the Example 8. Four different pregnancies went to term delivering 2 alive (9161 and 9162) and 2 stillborn (Fetus 2180 and 2197) calves. Following sequences analyses, summarized in Table 3, confirmed data of colonies used for cloning.

Figures 2-A and 2-B shows DNA sequence analyses (Sanger sequencing) done for calf 9161, cloned from colony #A4. In this case we obtained the disruption of target genes with mutations described as follows: AGACCCTGGGCGAGTCGGTGG deletion (SEQIDNO:120) GTGTTTAAGATCAAGCCTGAGAAGAGGTGGCAGGACATCAGCATGATGCGCATGA AGACTATCGGGGAGCACATTGTGGCCCACATCCAGCATGAGGTTGACTTCCTTTTC TGCATGGATGTGGACCAGGTCTTCCAAGACAAGTTTGGGGTGGAGACCCTGGGCG

AGTCG deletion (SEQ ID NO:121) for the GGTA1 gene (Figure 2-A); GGCAAGTGAGGGAGGCA deletion (SEQ ID NO:126) for the CMAH gene (Figure 2-B). Table 3 summaries the sequencing results obtained on the GGTA1 and CMAH gene sequences of the born cloned calves.

### Example 7. Phenotype Analysis for αGal, Neu5Gc and Sd^{a} antigens in TKO porcine and in DKO bovine primary cells. A-CELL CULTURE

Cells (pig or bovine fibroblasts) are thawed and cultured in DMEM medium (GIBCO), with 10% FBS, 1% Peni Strepto and bFGF (Sigma, 1ng/ml). Once confluent, cells are trypsinized, and split into two culture dishes, one with the complete medium as above and the other with DMEM medium, 5% human serum (SIGMA), 1% Peni/strepto and bFGF (SIGMA, 1ng/ml).

### B-CELL STAINING

### α-GAL

Cells (cultured in complete medium with FBS) are trypsinized, resuspended and washed in PBS + BSA 0,1%. The cells are pelleted (2500rpm 1minute 4°C), and resuspended in PBS + BSA 0,1% containing the FITC coupled lectine (BS-I Isolectin B4, SIGMA) diluted 1 :50, Incubated at 4°C for 30 minute. After 3 washes in PBS BSA 0,1% cells are ready for FACS analysis.

### Neu5Gc

Cells are cultured for at least 2 weeks in DMEM + human serum. Cells are put in 96 well plates (1.106 cells/well) washed once with PBS with 0,5% fish gelatin, then incubated in 200µl PBS 0,5% fish gelatin with anti-Neu5Gc antibody or control isotype (Biolegend, chicken polyclonal IgY, dilution 1 :1000) at 4°C for 1h., washed 4 times in PBS 0,5% fish gelatin incubated in 100µl PBS 0,5% fish gelatin with Alexa647-coupled anti-lgY antibody (Jackson ImmunoResearch, F(ab')₂ fragment donkey anti chicken 1 :500). at 4°C for 1h, washed 4 times in PBS 0,5% fish gelatin and transfered into FACS tubes.

### B4GalNT2 (Sd^{a})

Cells (cultured in complete medium with FBS) are trypsinized, resuspended and washed in PBS + BSA 0,1%. The cells are pelleted (2500rpm 1minute 4°C), and resuspended in PBS + BSA 2% containing the biotynilated-DBA lectine (USBIO D8085 1ug/ul), incubated 30 minutes at 4°C, followed by incubation with Streptavidin-PE (Becton Disckinson, 1 :500) for 30 minute at 4°C. After 3 washes in PBS BSA 2% cells are ready for FACS analysis.

### C-FACS ANALYSIS

FACS analysis was conducted using a BD Pharmingen LSR-II flow cytometer and FlowJo software (TreeStar).

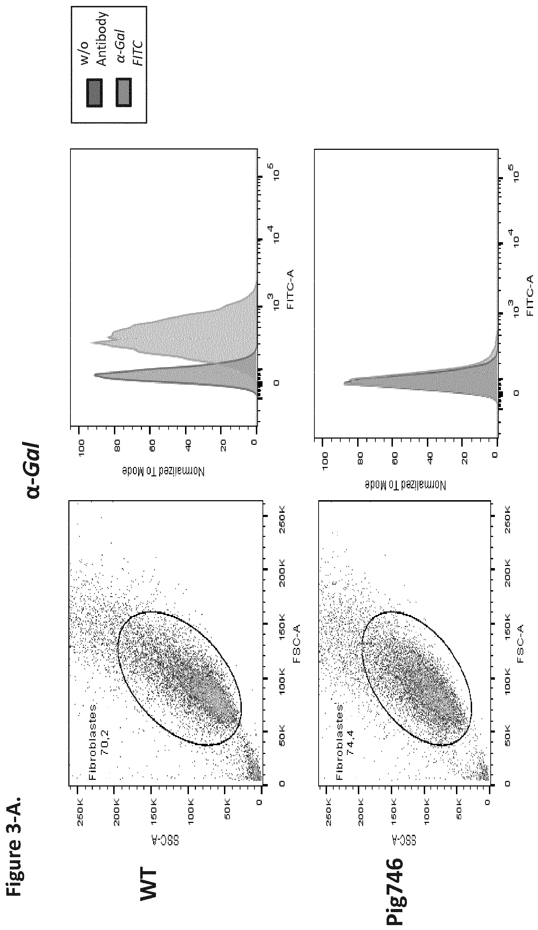

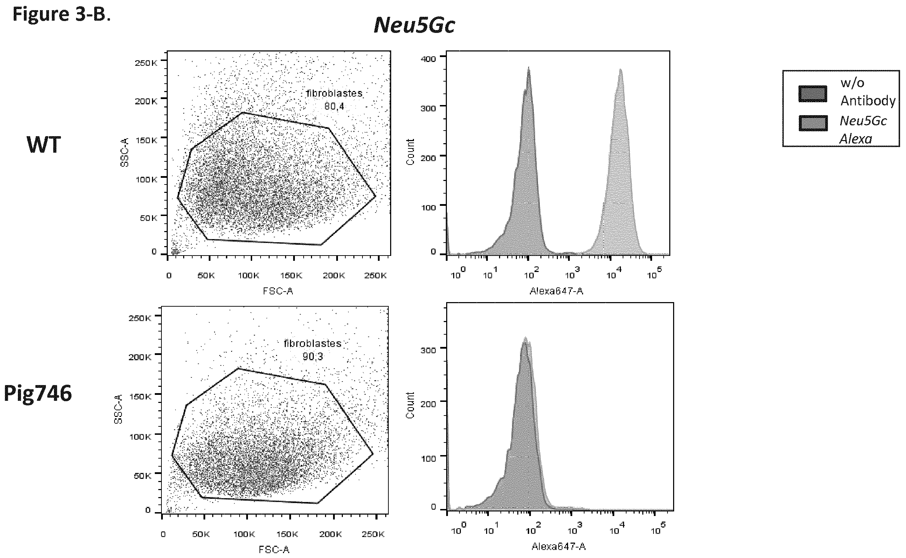

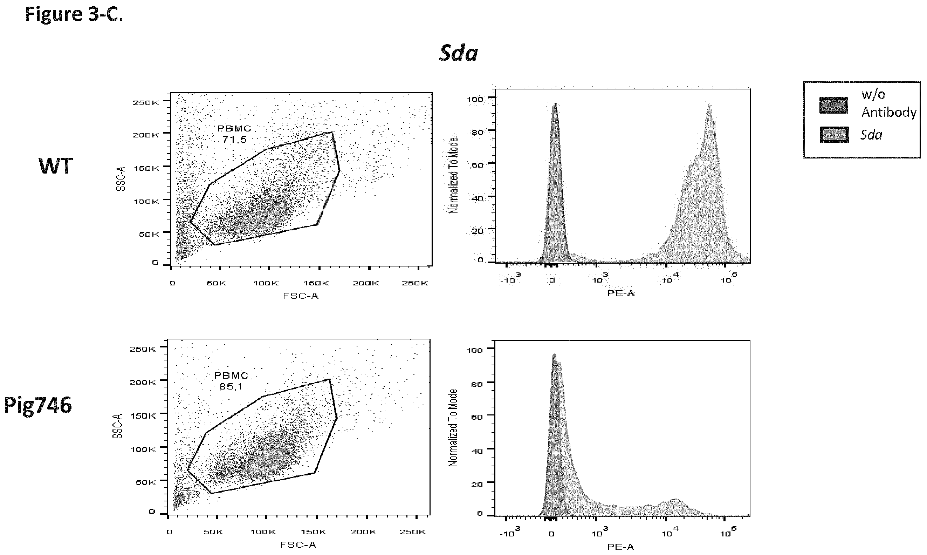

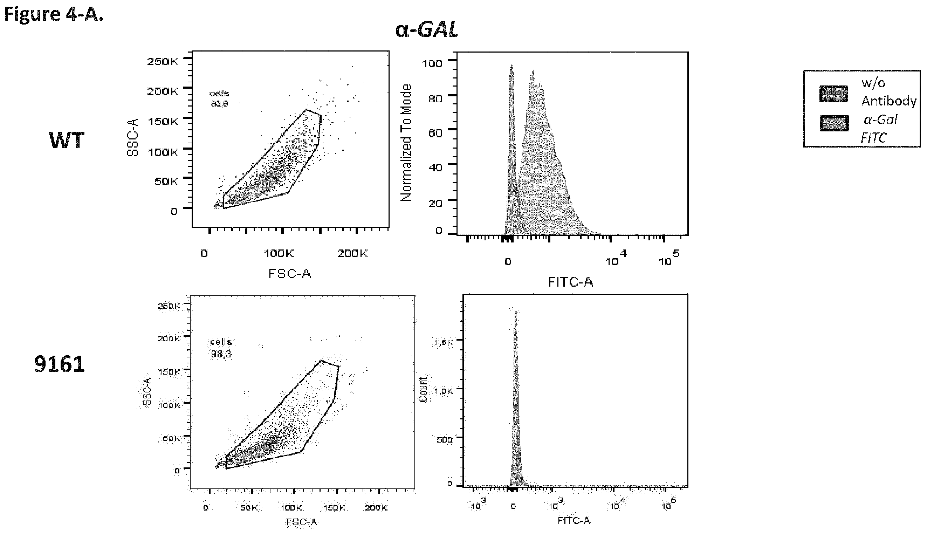

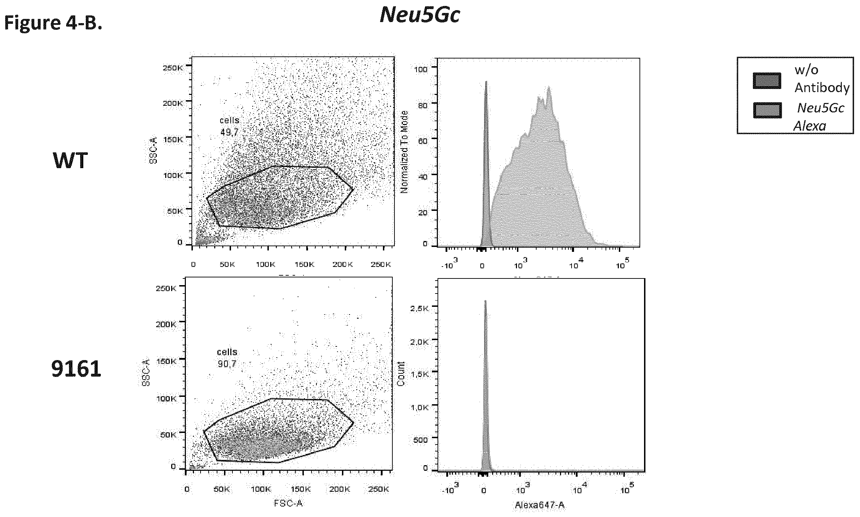

As example Figure 3 presents the results obtained for the phenotypical characterization by FACS of primary cells from the cloned TKO Pig46 respectively for the aGal (Figure 3-A), for the Neu5Gc (Figure 3-B) and for the Sd^{a} (Figure 3-C) antigens. Figure 4 presents the results obtained for the phenotypical characterization of primary cells from the cloned DKO 9161 calf respectively for the aGal (Figure 4-A) and for the Neu5Gc (Figure 3-B) antigens.

### Example 8. Reactivity test on α-Gal specific IgE and on Neu5Gc specific IgE from sera of patients affected by Read Meat allergy.

Firstly, to biochemically characterize the Neu5Gc and α-Gal in meat samples (porcine and bovine), meat homogenates were coated onto a 96-well plate in triplicates (1 µg/well) and analyzed with anti-Neu5Gc IgY (Neu5Gc) **(****Fig. 6-A****)**, or monoclonal anti αGal IgM **(****Fig. 6-B****),** washed then detected with HRP-donkey-anti-chicken IgY or HRP-goat-anti-mouse IgM, respectively. In **Figure 6-A** is shown that meat samples from swine wild-type (WT) and GGTA1-KO (GKO), and bovine WT express Neu5Gc, while swine and bovine with CMAH-KO and GGTA1-KO (DKO) lack Neu5Gc. Instead in **Figure 6-B** in shown that meat samples from WT swine and WT bovine express aGal , while swine DKO or GKO, and bovine DKO lack aGal (mean ± SD of triplicates; representative of at least two independent experiments;).

Subsequently sera from patients affected by red meat allergy were used to assess the low antigenicity of meat extracts derived from single (GGTA1-KO), double (GGTA1/CMAH-KO) and triple (GGTA1/CMAH/B4GalNT2-KO) edited pigs. To this end, an inhibition assay was developed based on the ImmunoCAP platform (ImmunoCAP technique, Thermo Fisher, Sweden). The results of the study demonstrate that the binding α-gal antigen specific IgE to their epitope was prevented by co-incubation with red meat extracts from wild-type non-engineered animals. In contrast, co-incubation with red meat extracts from GGTA1-KO animals did not inhibit such a binding. This data confirmed our hypothesis regarding the validity of using low allergenic animal products for treatment and prevention of xeno-allergies related to usage and consumption of animal-derived meat products.

As additional validation of previous data collected using 3-KO porcine tissues, we evaluated the human serum reactivity against bovine meat samples. Meat homogenates were coated onto a 96-well plate in duplicates (1µg/well), then reactivity of human serum IgE (1:100) was examined by ELISA in samples of donors with no allergy, birch-pollen allergy or red meat allergy. As shown in **Figure 6-C****,** sera samples reactivity examined against bovine meat samples showed increased IgE response in red meat allergy sera against WT compared to the DKO meat samples (mean ± SEM; Two-Way ANOVA with Bonferroni multiple comparison test).

Finally, we tested the patients human serum IgE reactivity against glycans microarrays. Reactivity of human serum IgE (1:100) was examined by glycan microarrays using samples of donors with no allergy, birch-pollen allergy or red meat allergy allergy. Glycan microarray profiles of human sera samples donors are presented in Relative Fluorescent Units (RFU; representing fluorescence at 532 nm after local background subtraction) as a heatmap in **Figure 6-D****.**

In **Figure 6-E****,** the reactivity of sera samples against Neu5Gc-glycans was compared between the groups showing that red meat allergy sera have a higher anti-Neu5Gc IgE reactivity (mean ± SEM; each spot is a different glycan per serum; Kruskal Wallis test with Dunn's multiple comparison test; P < 0.0001). In **Figure 6-F****,** the reactivity of sera samples against Neu5Ac-glycans was compared between the groups showing that red meat allergy sera have a higher reactivity (mean ± SEM; each spot is a different glycan per serum; Kruskal Wallis test with Dunn's multiple comparison test; P = 0.0031). However, further analysis revealed strong matching between pairs of glycans that differ only by their terminal sialic acid, Neu5Ac versus Neu5Gc, suggesting that the observed reactivity against Neu5Ac-glycans likely represent cross-reactive anti-Neu5Gc antibodies (Wilcoxon matched pairs test; r Spearman = 0.2901, ***, P = 0.0007). In **Figure 6-G****,** the reactivity of sera samples against aGal glycan on the microarray was compared between the groups showing that red meat allergy sera have a higher anti-Gal IgE reactivity (mean ± SEM; one spot per serum; Kruskal Wallis test with Dunn's multiple comparison test; P = 0.0012).

### Example 9. Somatic Cell Nuclear Transfer

The protocol used was the one described by Lagutina et al. (2006) with minor modifications. Briefly, ovaries of pubertal gilts were collected at a local abattoir and transported to the laboratory at 31°- 33°C. Follicles larger than 3 mm were aspirated and cumulus-oocyte complexes were selected and *in vitro* matured in defined maturation medium at 38.5 °C in 5% CO₂ in humidified air for 42 hours. At the end of maturation process, only matured oocytes were selected (presence of an extruded polar body). The day before SCNT, selected nuclear donor cells were induced into quiescence by serum starvation (0.5% FCS). The day of SCNT, cells were trypsinized, washed and resuspended in SOF (Tervit et al., 1972) supplemented with 25mM Hepes (SOF-Hepes). NT-embryos were reconstructed following a zona-free method (Vajta et al., 2010), where the zona pellucida was digested with 0.5% pronase in PBS. All manipulations were performed in SOF-Hepes with 10% FCS. Enucleation was performed following 10 minutes exposure to cytochalasin B. Enucleated cytoplasts were then rinsed in phytohemagglutinin P in PBS and quickly dropped over a single nuclear donor cell. Donor cell-cytoplast couples were washed in 0.3 M mannitol solution and fused by double DC-pulse (1.2 KVolt / cm) and returned in to maturation medium. At about 48 hours of maturation, NT embryos were activated by double DC-pulses (Lagutina et al., 2006), followed by two hours in maturation medium supplemented with cytochalasin B, reconstructed embryos were cultured in mSOF supplemented with essential and non-essential aminoacids and with 4 mg/mL BSA, in a modification of the Well Of the Well system (Vajta et al., 2000) and the culture was continued as described by Lagutina et al. (2006).

### Example 10. Recipient sows synchronization, Embryo Transfer (ET) and post-implantation development

Recipient sows estrus was synchronized by feeding 12 mg of altrenogest (Regumate, Intervet, Peschiera Borromeo, Italy) per sow for 15 days, followed by an injection of 0.15 mg of PgF2 of Dalmazin, Fatro, Ozzano Emilia, Italy) at the 13th day of altrenogest treatment, and one of 1000 IU of hCG (Chorulon, Intervet, Peschiera Borromeo, Italy) 96 hours after the last altrenogest treatment. SCNT embryos were surgically transferred to the uterus of synchronized sows on day 6 of development (compacted morula and early blastocyst stages) through a mid-ventral laparotomy procedure (24), five days after standing estrus. Recipients were checked for pregnancy by trans-abdominal ultrasound examination 30 days after ET and pregnancy were confirmed around day 60 post-ET. Farrowing was induced by a PgF2α injection on day 119 and 120 of gestation.

From the above description and the above-noted examples, the advantage attained by the product described and obtained according to the present invention are apparent.

The present invention therefore resolves the above-lamented problem with reference to the mentioned prior art, offering at the same time numerous other advantages, including making possible the development of genetically modified non-human mammals as a source of raw biological materials free of αGal, Neu5GC and Sd^{a}-like antigens, avoiding formation/reaction of (new) specific human xeno-antibodies.

## Claims

1. A genetically modified non-human mammal chosen from the group consisting of a pig, a bovine, an equine, a sheep, a goat, a buffalo, a camel and a rabbit, said non-human mammal comprising mutated α(1,3)-galactosyltransferase (GGTA1), cytidine monophosphate-N-acetylneuraminic acid hydroxylase (CMAH) and beta1-4N-acetylgalactosaminyltransferase (B4GalNT2) genes in at least one cell,
wherein:
when said non-human mammal is a pig:
- the α(1,3)-galactosyltransferase gene has a sequence chosen from the group consisting of SEQ ID NO:91, SEQ ID NO:92, SEQ ID NO:93, SEQ ID NO:94, SEQ ID NO:95, SEQ ID NO:96, SEQ ID NO:97 and SEQ ID NO:98;
- the CMAH gene has a sequence chosen from the group consisting of SEQ ID NO:99, SEQ ID NO:100, SEQ ID NO:101, SEQ ID NO:102, SEQ ID NO:103, SEQ ID NO:104 and SEQ ID NO:105; and
- the B4GalNT2 gene has a sequence chosen from the group consisting of SEQ ID NO:106, SEQ ID NO:107, SEQ ID NO:108, SEQ ID NO:109, SEQ ID NO:110, SEQ ID NO:111, SEQ ID NO:112, SEQ ID NO:113, SEQ ID NO:114, SEQ ID NO:115, SEQ ID NO:116, SEQ ID NO:117, SEQ ID NO:30, SEQ ID NO:31, SEQ ID NO:118 and SEQ ID NO:119;
when said non-human mammal is a bovine:
- the α(1,3)-galactosyltransferase gene has a sequence chosen from the group consisting of SEQ ID NO:120, SEQ ID NO:121, SEQ ID NO:122, SEQ ID NO:123, SEQ ID NO:124 and SEQ ID NO:125; and
- the CMAH gene has a sequence chosen from the group consisting of SEQ ID NO:126, SEQ ID NO:127, SEQ ID NO:128, SEQ ID NO:129, SEQ ID NO:130, SEQ ID NO:131 and SEQ ID NO:132; and
when said non-human mammal is an equine:
- the α(1,3)-galactosyltransferase gene having the sequence of SEQ ID NO:68;
- the CMAH gene having a sequence chosen from the group consisting of SEQ ID NO:78, SEQ ID NO:79 and SEQ ID NO:80; and
- the B4GalNT2 gene having the sequence of SEQ ID NO:90 are mutated.

2. The genetically modified non-human mammal according to claim 1, said genetically modified non-human mammal being a pig, wherein
- the α(1,3)-galactosyltransferase gene has the sequence of SEQ ID NO:91;
- the CMAH gene has the sequence of SEQ ID NO:99; and
- the B4GalNT2 gene has the sequences of SEQ ID NO:106 in allele A and SEQ ID NO:107 in allele B or SEQ ID NO:114 in allele A and SEQ ID NO:31 in allele B;
or
- the α(1,3)-galactosyltransferase gene has the sequences of SEQ ID NO:92 in allele A and SEQ ID NO:93 in allele B;
- the CMAH gene has the sequences of SEQ ID NO:100 in allele A and SEQ ID NO:101 in allele B; and
- the B4GalNT2 gene has the sequences of SEQ ID NO:108 in allele A and SEQ ID NO:109 in allele B or SEQ ID NO:115 in allele A and SEQ ID NO:116 in allele B;
or
- the α(1,3)-galactosyltransferase gene has the sequence of SEQ ID NO:94;
- the CMAH gene has the sequence of SEQ ID NO:102; and
- the B4GalNT2 gene has the sequences of SEQ ID NO:110 in allele A and SEQ ID NO:111 in allele B or SEQ ID NO:117 in allele A and SEQ ID NO:31 in allele B;
or
- the α(1,3)-galactosyltransferase gene has the sequence of SEQ ID NO:95 in allele A and SEQ ID NO: 96 in allele B;
- the CMAH gene has the sequence of SEQ ID NO:103 in allele A and SEQ ID NO: 104 in allele B; and
- the B4GalNT2 gene has the sequences of SEQ ID NO:112 in allele A and SEQ ID NO:30 in allele B or SEQ ID NO:118.
or
- the α(1,3)-galactosyltransferase gene has the sequence of SEQ ID NO:97 in allele A and SEQ ID NO: 98 in allele B;
- the CMAH gene has the sequence of SEQ ID NO:105; and
- the B4GalNT2 gene has the sequences of SEQ ID NO:113 in allele A and SEQ ID NO:113 in allele B or SEQ ID NO:119 in allele A and SEQ ID NO:31 in allele B.

3. The genetically modified non-human mammal according to claim 1, said genetically modified non-human mammal being a bovine, wherein
- the α(1,3)-galactosyltransferase gene has the sequences of SEQ ID NO:120 in allele A and SEQ ID NO:121 in allele B;
- the CMAH gene has the sequence of SEQ ID NO:126
or
- the α(1,3)-galactosyltransferase gene has the sequences of SEQ ID NO:122;
- the CMAH gene has the sequences of SEQ ID NO:127 in allele A and SEQ ID NO:128 in allele B
or
- the α(1,3)-galactosyltransferase gene has the sequences of SEQ ID NO: 123;
- the CMAH gene has the sequences of SEQ ID NO:129 in allele A and SEQ ID NO:130 in allele B
or
the α(1,3)-galactosyltransferase gene has the sequences of SEQ ID NO:124 in allele A and SEQ ID NO:125 in allele B;
- the CMAH gene has the sequences of SEQ ID NO:131 in allele A and SEQ ID NO:132 in allele B.

4. A hypoallergenic product isolated from the genetically modified non-human mammal according to anyone of claims 1 to 3, wherein said product is chosen from the group consisting of a carcass, meat, milk, milk derived products, organ, tissue, transfusion product or a cell.

5. The product according to claim 4, wherein said organ is selected from the group consisting of skin, muscle, heart, liver, kidneys, lung, pancreas, thyroid, small bowel, udder, blood and components thereof.

6. Use of the product isolated from the genetically modified non-human mammal according to anyone of claims 4 or 5 for the preparation of animal derived products to prevent specific xeno-allergies, food born allergies and autoimmune and degenerative diseases.

7. Use of the product isolated from the genetically modified non-human mammal according to anyone of claims 4 or 5 for the direct consumption or for the preparation of animal derived products (foodstuffs) for human consumption.

8. Use of the product isolated from the genetically modified non-human mammal according to anyone of claims 4 or 5 for the preparation of animal derived medical products.

9. Use of the product isolated from the genetically modified non-human mammal according to anyone of claims 4 or 5 for the preparation of animal derived products for human drug production.

10. Use of the product isolated from the genetically modified non-human mammal according to anyone of claims 4 or 5 for the preparation of animal derived products for human medical devices production.

11. Use of the product isolated from the genetically modified non-human mammal according to anyone of claims 4 or 5 for the preparation of animal derived products for antisera or antibody production.

12. Use of the product isolated from the genetically modified non-human mammal according to anyone of claims 4 or 5 for the preparation of animal derived products for human cosmetics production.

13. Use of the product isolated from the genetically modified non-human mammal according to anyone of claims 4 or 5 for the preparation of animal derived products for human detergents production.


SEQUENCE LISTING

<110> AVANTEA S.R.L.

<120> Safer, healthier food and medical products from genome edited livestock

<130> P019396EP-01

<150> IT102017000149385
<151> 2017-12-22

<160> 246

<170> BiSSAP 1.3.6

<210> 1
<211> 20
<212> DNA
<213> Sus scrofa

<400> 1
ggaccttggg atcagtcagg                                                20

<210> 2
<211> 19
<212> DNA
<213> Sus scrofa

<400> 2
cggcaactct ctggaatgc                                                 19

<210> 3
<211> 21
<212> DNA
<213> Sus scrofa

<400> 3
gtagacctag gaaacggatg c                                              21

<210> 4
<211> 19
<212> DNA
<213> Sus scrofa

<400> 4
cggtatttaa gggctcagg                                                 19

<210> 5
<211> 18
<212> DNA
<213> Sus scrofa

<400> 5
taatgaatgt caaaggaa                                                  18

<210> 6
<211> 34
<212> PRT
<213> Sus scrofa

<400> 6
Asn Ile Asn Ile Asn Gly Asn Asn Asn Ile Asn Ile Asn Gly Asn Asn
1               5                   10                  15
Asn Gly His Asp Asn Ile Asn Ile Asn Ile Asn Asn Asn Asn Asn Ile
            20                  25                  30
Asn Ile

<210> 7
<211> 18
<212> DNA
<213> Sus scrofa

<400> 7
ttacagttga gacaagca                                                    18

<210> 8
<211> 34
<212> PRT
<213> Sus scrofa

<400> 8
Asn Gly Asn Ile His Asp Asn Ile Asn Asn Asn Gly Asn Gly Asn Asn
1               5                   10                  15
Asn Ile Asn Asn Asn Ile His Asp Asn Ile Asn Ile Asn Asn His Asp
            20                  25                  30
Asn Ile

<210> 9
<211> 18
<212> DNA
<213> Sus scrofa

<400> 9
gagtggttct gtcaatgc                                                    18

<210> 10
<211> 894
<212> DNA
<213> Sus scrofa

<220>
<223> (derived from NC_010443.5 Reference - Sscrofa11.1 -
      ENSSSCG00000005518)

<400> 10
gtagacctag gaaacggatg cttcctctag tctgtgatgc gaggtggggc atctgagttg      60

ggggcggctg gagcccttag ggaccattaa ctaaacccgt cactctccca catctcggtg 120

gaccttggga tcagtcagga tgcttcccct ttgagcctca aaatggcctt agtatccttc 180

ccaacccaga cggccctgtc agttcattga cttggctaat ttgccagtgt aggcctatgc 240

aaattaaggt agaacgcact ccttagcgct cgttgactat tcatcaactt ttccttttag 300

aaaagatatt ggtataagca cttcttaaaa aaccatattc cactctgggt gtatttaatc 360

taattttccc ttctcctttt cttttcccag gagaaaataa tgaatgtcaa aggaagagtg 420

gttctgtcaa tgctgcttgt ctcaactgta atggttgtgt tttgggaata catcaacagg 480

taattatgaa acatgatgaa atgatgttga tgaaagtctc ctctaatctc ctagttatca 540

gccaagtcac cagcttgcat taaaagtagg attcactgac accgtaaaga aagcattcca 600

gagagttgcc gttgtggctc aggggcagca aacccaatta ggatccaaga ggaggtgggt 660

ttgatccctg gccttgctct ttggcttaag gatccggcat tgccgtgacc tgtggtgtag 720

gttgcagatg cagctcggat ctggcattgc tgtggctgtg gcgtaggctg gtggcttcag 780

ctccagtttg acccctagcc tgggaacttc catatcccac acttgcggcc ctaaaaagca 840

aagaaagaaa gaaaatattc tacccttcct gtatccctga gcccttaaat accg 894

<210> 11
<211> 25
<212> DNA
<213> Sus scrofa

<400> 11
cctttcatga gttttgtgtt caacc 25

<210> 12
<211> 23
<212> DNA
<213> Sus scrofa

<400> 12
aatcagaatg catccaaggc ttc 23

<210> 13
<211> 23
<212> DNA
<213> Sus scrofa

<400> 13
ccctatctcc aactatcaca tcc 23

<210> 14
<211> 26
<212> DNA
<213> Sus scrofa

<400> 14
tggtcaattg tcctctcacc tacaca                                          26

<210> 15
<211> 18
<212> DNA
<213> Sus scrofa

<400> 15
tgaagtatat caatcctc                                                   18

<210> 16
<211> 36
<212> PRT
<213> Sus scrofa

<400> 16
Asn Gly Asn Asn Asn Ile Asn Ile Asn Asn Asn Gly Asn Ile Asn Gly
1               5                   10                  15
Asn Ile Asn Gly His Asp Asn Ile Asn Ile Asn Gly His Asp His Asp
            20                  25                  30
Asn Gly His Asp
        35

<210> 17
<211> 18
<212> DNA
<213> Sus scrofa

<400> 17
tatttaccca gttcgtct                                                   18

<210> 18
<211> 36
<212> PRT
<213> Sus scrofa

<400> 18
Asn Gly Asn Ile Asn Gly Asn Gly Asn Gly Asn Ile His Asp His Asp
1               5                   10                  15
His Asp Asn Ile Asn Asn Asn Gly Asn Gly His Asp Asn Asn Asn Gly
            20                  25                  30
His Asp Asn Gly
        35

<210> 19
<211> 16
<212> DNA
<213> Sus scrofa

<400> 19
ctggaagctt ctgtca                                                     16

<210> 20
<211> 2105
<212> DNA
<213> Sus scrofa

<220>
<223> (derived from NC_010449.5 Reference - Sscrofa11.1 -
      ENSSSCG00000001099)

<400> 20
ccctatctcc aactatcaca tccctatcac cgtctggtaa gaaatggaaa aaatattact      60

aagcctcctt tgttgctaca attaatctga ttctcattct gaagcagtgt tgccagagtt     120

aacaaataaa aatgcaaagc tgagtagtta aatttgaatt acagataaac aaatttccaa     180

tatatgttca atattgtgta agacatttta aaattatttt ttatttatct gaaatttata     240

tttttcctgt attttatctg gcaaccatga tcagaaatct ttaaacaatc aggaagtctt     300

ttttcttaga caaatgaaaa tttgagttga tcttaggttt agtacactat actaggggcc     360

aagggttata gtgtgactat taaatcacag ataatcttta ttactacatt atttccttat     420

actggcccca cttggatctt actcagctta gcttttgtat gagagtcatc cttaaagatg     480

actttattct ttaaaaaaaa aaacaaattt taagggctgc acccatagca tatagaagtt     540

cctaggctag cggtcaaatt agagctgcag ctgccagcct atgccacagc cacagcaatg     600

ccagatctga gctgcatctg tgacctacac tgcagcttgc agcaatgctg gatccttaac     660

ccattgaaca atgccaggga ttgaacacac atcctcatgg atactgctca ggttcctaac     720

ctgctgagcc acagttggaa ctccaaagca gactttattc tgatggctct gctgatctct     780

aacacgttat tttgtgccat ggtgtttatc ttcactttac tcaagtcagg gaaacacgaa     840

gagtctcata caggataaac ccaaggagaa atgtgcaaag tcacatacaa atcaaactga     900

caaaaatcaa atacaaggaa aaaatatctt cactttcaaa atcacctact gatgatgagt     960

ttatatttcc ttggatattt gaatattagc tatttttttc ctttcatgag ttttgtgttc    1020

aaccaactac agtcgtttac tttgatcaca gaataatgca tttaagcctt aaatagatta    1080

atatttattt tcaccatttc ataaacctaa gtacaatttt ccatccaggt ctgttaaatg    1140

cacaaaacac aactggaagt tagatgtaag cagcatgaag tatatcaatc ctcctggaag    1200

cttctgtcaa gacgaactgg gtaaatacca tcaatactga tcaatgtttt ctgctgttac    1260

tgtcattggg gtccctcttg tcaacttgtt tccaatctca ttagaagcct tggatgcatt    1320

ctgattttaa actgaggtat tttaaaagta accatcactg aaaattctag gcaagttttc    1380

tctaaaaaat cccttcattc attcatttgt tcagtaagta tttgatgaga ccttaccatg    1440

tgtaaacatt gcactaggta ttaagaaata caaagatgga taagatagag tcggcgtaaa    1500

tgagatgata taatgagacg ttataatgaa actcacaatt ccagttggga aataaagtcc    1560

ttcaaattcc atgactcttt ctggcacacg ttagaggcta cagcttctgt gtgattctca 1620

tgctggctcc acttctactt tttccttctt cctactcaag aaagcctata gaaatatgag 1680

taagaagggc ttaatcatag gaataaattt gtctctgttc taagtgatta aaaatgtctt 1740

tatcagtata aaaagttact tgggaagatt cttaaaactg cttttacaca ctgttttaga 1800

atgactgtta tataaataaa aaagtagatt tgatctaaca caattaaatg acttttggaa 1860

atattgacta attctcacct tgcccctcaa agggatgcct gaaccatttc cttcttttgc 1920

cagaaagccc ccaccctttg tctgttgacc tagcctagga aatcttcaga tcacattgtt 1980

agcaggaact ggttacatgt gctgtacaaa tactatttaa ttcatctgat taaaaaaaaa 2040

aagagataag aagcaaaagt ttgactgtct taaactgttt gtgtaggtga gaggacaatt 2100

gacca 2105

<210> 21
<211> 24
<212> DNA
<213> Sus scrofa

<400> 21
cgcaagtgac cagacatcgt tccc 24

<210> 22
<211> 25
<212> DNA
<213> Sus scrofa

<400> 22
caccgtagaa atcgggtttc tcagg 25

<210> 23
<211> 22
<212> DNA
<213> Sus scrofa

<400> 23
cgcaagtgac cagacatcgt tc 22

<210> 24
<211> 22
<212> DNA
<213> Sus scrofa

<400> 24
aggtgaagag gctcctgaca tg 22

<210> 25

<211> 22
<212> DNA
<213> Sus scrofa

<400> 25
atgacttcgt acagccctag at                                                22

<210> 26
<211> 22
<212> DNA
<213> Sus scrofa

<400> 26
ttaggtggca cattgaagat gg                                                22

<210> 27
<211> 20
<212> DNA
<213> Sus scrofa

<400> 27
ctgtatcgag gaacacgctt                                                   20

<210> 28
<211> 20
<212> DNA
<213> Sus scrofa

<400> 28
acataaagag tccaacgctc                                                   20

<210> 29
<211> 20
<212> DNA
<213> Sus scrofa

<400> 29
cttcagcgtc tgcgcatcca                                                   20

<210> 30
<211> 465
<212> DNA
<213> Sus scrofa

<220>
<223> (derived from NC_010454.4 Reference - Sscrofa11.1 -
      ENSSSCG00000040942)

<400> 30
cgcaagtgac cagacatcgt tcccagtgga tcccagcagg atctttttgg gttattttat      60

ggtttccttt gctgtgcaaa agcttgtcag tttgcttagg tttatttttg cttttctttc     120

tcttttgctt tgggaagaaa tcaattttga tacgtagtgt acgaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat atcgatggtg cttttggtcc tgagcgttgg actctttatg ttccaaagcg 360

tgttcctcga tacagacttc agtctcctca actcacccat cccgtccccc accctggatg 420

cgcagacgct gaagcttcta cctgagaaac ccgatttcta cggtg 465

<210> 31
<211> 471
<212> DNA
<213> Sus scrofa

<220>
<223> (derived from Sscrofa11.1 - ENSSSCG00000030269)

<400> 31
cgcaagtgac cagacatcgt tcccagtgga tcccagcagg atctttttgg gttattttat 60

ggtttccttt gctgtgcaaa agcttgtcag tttgcttagg tttatttttg cttttctttc 120

tcttttgctt tgggaagaaa tcaattttga tacgtagtgt acgaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat gtcgatggtg cttttggtcc tgagcgttgg actctttatg ttccgaagtg 360

tgttcctcga tacagacttc agtctcctca actcacccat cccgtccccc accctggatg 420

cgcagacgct gaagcttcta cctgagaaac atgtcaggag cctcttcacc t 471

<210> 32
<211> 1521
<212> DNA
<213> Sus scrofa

<220>
<223> GenBank accession number: MG675060

<400> 32
atgacttcgt acagccctag atgtctgtcg atcctcaaga tgtcgatggt gcttttggtc 60

ctgagcgttg ggctctttat gttccgaagc gtgttcctcg atacagactt cagtctcctc 120

aactcaccca tcccgtcccc caccctggat gcgcagacgc tgaagcttct acctgagaaa 180

catgtcagga gcctcttcac ctacagtgga atctggctgt tcccgaaaaa ccagtgccaa 240

tgtgacgcct tcgggcatca gggaagctat aacttggagg atgcctacga cccgcgggac 300

ctccccgcag tgaacctgag gagacaggct gagctcgaac actttcagag gagagaaggg 360

ctccctcgcc caccgcccct gctggctcag cccaacctcc cctttgggta cccggtccac 420

ggggtggaag tgatgcctct acacaccatc cccatcccag gcctccggtt tgaaggacct 480

gatgctccca tctatgaggt caccctgaca gcttctctgg ggacactgaa cacccttgct 540

gacgtcccag acaatgtggt gaagggcaga ggccagaagc agctgaacat tttgaccagt 600

agccgggagc ttttgaattt catcctccag catgtgacat acacgagcac agagtaccac 660

ctccacagag tggatgtggt gagtctggag tccaagtcct cagtggccaa gtttccggtg 720

gccatccgct atcctgtcat gcccaagtta tatgaccctg gaccagagag gaagctccga 780

gacctggtga ctattgccac caaaaccttc ctccgtcccc acaagctcat gaccatgctc 840

cggagtgttc gtgagtacta cccagacctg acggtgatcg tggccgatga cagcaaggag 900

cccctgaaaa tcactgacag ccacgtggag tattacgcca tgccatttgg gaagggctgg 960

tttgctggca ggaacctggc catatctcag gtcaccacca aatatgtgct ctgggtggac 1020

gatgacttca tcttcaacag caagaccagg atcgaggcgc tggtggacgt cctagagaaa 1080

acggaactgg acgtggtagg tggcagggtg attgaaaaca cattccagtt caagctgttg 1140

ctggagcagg ggaagaatgg cgactgtctc caccggcagt caggattttt ccggcccgtg 1200

gatggcttcc ccggttgcgt ggttaccagt ggcgttgtca acttcttcct ggctcacaca 1260

gagcgactcc aaagagttgg cttcgacccc cggctgcagc gagtggctca ctcagagttc 1320

tttattgatg ggctcgggag cctgctcgtg gggtcctgcc cacaggtgat cataggtcac 1380

cagccccatt taccagtgat ggacccagag ctggccgccc tggaggggaa ctacagcagg 1440

tatcgggcca acaccaaagc ccagatcaaa ttcaagttgg ctctccacta cttcaagaac 1500

catcttcaat gtgccaccta a 1521

<210> 33
<211> 21
<212> DNA
<213> Bos taurus

<400> 33
ggatgccttt gatagagttg g 21

<210> 34
<211> 19
<212> DNA
<213> Bos taurus

<400> 34
gctttcatca tgccattgg 19

<210> 35
<211> 20

<212> DNA
<213> Bos taurus

<400> 35
agcatctttc acaactcagg                                                    20

<210> 36
<211> 20
<212> DNA
<213> Bos taurus

<400> 36
tgagacatta ggaacatggc                                                    20

<210> 37
<211> 23
<212> DNA
<213> Bos taurus

<400> 37
ggagaccctg ggcgagtcgg tgg                                                23

<210> 38
<211> 23
<212> DNA
<213> Bos taurus

<400> 38
gctgggccac cgactcgccc agg                                                23

<210> 39
<211> 23
<212> DNA
<213> Bos taurus

<400> 39
gagaaaataa tgaatgtcaa agg                                                23

<210> 40
<211> 440
<212> DNA
<213> Bos taurus

<400> 40
ggatgccttt gatagagttg ggtcctctgc gctccttcaa agtgtttaag atcaagcctg      60

agaagaggtg gcaggacatc agcatgatgc gcatgaagac tatcggggag cacattgtgg     120

cccacatcca gcatgaggtt gacttccttt tctgcatgga tgtggaccag gtcttccaag     180

acaagtttgg ggtggagacc ctgggcgagt cggtggccca gctacaagcc tggtggtaca     240

aggcagatcc caatgacttc acctacgaga ggcggaagga gtctgcagca tacattccct 300

tcggcgaagg ggatttttat taccatgcag ccatttttgg gggaacaccc actcaggtcc 360

ttaacatcac ccaggaatgc ttcaaaggaa tcctcaagga caagaaaaat gacatagaag 420

cccaatggca tgatgaaagc 440

<210> 41
<211> 739
<212> DNA
<213> Bos taurus

<220>
<223> (derived from AC_000168.1 Reference Bos_taurus_UMD_3.1.1 \226
      ENSBTAG00000012090)

<400> 41
agcatctttc acaactcagg cagccccatc tgctcattga attggctaat ttgccattgc 60

agacccatgc aatttaaggt agagcctgct ccttgcacgt gtgtgtgccc agttgcttca 120

gtcgtgtcca gctgtttgtg acactatgga ctatagccca ccagacactt ctgtccatgg 180

gattctccag gcaagagtac tggagtgggt tgccatgccc tccttcaggg gatcttcccc 240

atccagggat cgaacttgtg tctcctgcat tgcaggcaga ttcttcactg ctgagccact 300

ggggaagccc agaacatgcc cctcagtgct tattaaatgt tcatcacctt ttccttttag 360

aaaggatatg ggtagaaata tttcttaaaa aaaatcatat cccactcttg ctatatttaa 420

tctatttccc cccctcttct tttcttttcc caggagaaaa taatgaatgt caaaggaaaa 480

gtgattctgt caatgctggt tgtctcaact gtcattgttg tgttttggga atatatccac 540

aggtaattat ggaacatgat aaagtgatgt taatgaacgt ctccatcagc caagtcacca 600

tgttgaatta aaattaggat ttcttccttc ctgtttccct gagccctaga ctttaacatc 660

tgtctttaaa atcattagat cttcaagcac tttctaagga attaattatc tttcttcctg 720

ccatgttcct aatgtctca 739

<210> 42
<211> 23
<212> DNA
<213> Bos taurus

<400> 42
tcaggaggag acatcaccaa cgg 23

<210> 43
<211> 22
<212> DNA
<213> Bos taurus

<400> 43
tgcccatcct acttgtcgag gg                                                22

<210> 44
<211> 23
<212> DNA
<213> Bos taurus

<400> 44
gactatgggc aggcaagtga ggg                                               23

<210> 45
<211> 23
<212> DNA
<213> Bos taurus

<400> 45
gagaggcaac gacagactat ggg                                               23

<210> 46
<211> 225
<212> DNA
<213> Bos taurus

<220>
<223> (derived from NC_007324.6 Alternate Btau_5.0.1 -
      ENSBTAG00000003892)

<400> 46
tcaggaggag acatcaccaa cggaaatact gagctgagtg tacatcctct ttctccacgc      60

agacttctgc cttgggggga gtgaatgagc cacagaagct gaagtgacag ctgccattct     120

tctgaaatac ccagggagag gcaacgacag actatgggca ggcaagtgag ggaggcatta     180

ctttgctggg aaggtggggt caaccctcga caagtaggat gggca                     225

<210> 47
<211> 100
<212> DNA
<213> Bos taurus

<220>
<223> (START) codon with TAA (STOP) codon into the Bos taurus CMAH gene

<400> 47
gtgacagctg ccattcttct gaaataccca gggagaggca acgacagact taaggcaggc      60

aagtgaggga ggcattactt tgctgggaag gtggggtcaa                           100

<210> 48
<211> 22
<212> DNA
<213> Bos taurus

<400> 48
cagcatttgc tcttttgtgt cc                                                22

<210> 49
<211> 20
<212> DNA
<213> Bos taurus

<400> 49
tgtaaggtgg gcacaatagg                                                   20

<210> 50
<211> 23
<212> DNA
<213> Bos taurus

<400> 50
gcccagggcc aagaataaga cgg                                               23

<210> 51
<211> 23
<212> DNA
<213> Bos taurus

<400> 51
gccggactag gctgaaggag tgg                                               23

<210> 52
<211> 23
<212> DNA
<213> Bos taurus

<400> 52
gggtcttgat atgttcctca ggg                                               23

<210> 53
<211> 23
<212> DNA
<213> Bos taurus

<400> 53
gattccatcg tgggtaaaga ggg                                               23

<210> 54
<211> 23
<212> DNA
<213> Bos taurus

<400> 54

gtccgtctta ttcttggccc tgg                                                23

<210> 55
<211> 23
<212> DNA
<213> Bos taurus

<400> 55
aacattccgg cagccggact agg                                                23

<210> 56
<211> 23
<212> DNA
<213> Bos taurus

<400> 56
gtctcaccag attccatcgt ggg                                                23

<210> 57
<211> 23
<212> DNA
<213> Bos taurus

<400> 57
agcttcaaca ttccggcagc cgg                                                23

<210> 58
<211> 23
<212> DNA
<213> Bos taurus

<400> 58
cttcagccta gtccggctgc cgg                                                23

<210> 59
<211> 312
<212> DNA
<213> Bos taurus

<220>
<223> (derived from NC_007317.6 Alternate Btau_5.0.1 -
      ENSBTAG00000009864)

<400> 59
cagcatttgc tcttttgtgt cctaacagct ctagatatct gtggatcctc aagatgtccg      60

tcttattctt ggccctgggc attgtgttat ttatgttcag aagtgtgtac ctgtattcag     120

aattcagcag ctacaaagca ccactccttc agcctagtcc ggctgccgga atgttgaagc     180

ttctccctga ggaacatatc aagaccctct ttacccacga tggaatctgg tgagacagcc     240

agttattccc ccttggtctg acctctccct ccctcctcct gtggctgagt gtcctattgt     300

gcccacctta ca                                                          312

<210> 60
<211> 20
<212> DNA
<213> Equus caballus

<400> 60
acttctccca catgccatgg                                                  20

<210> 61
<211> 20
<212> DNA
<213> Equus caballus

<400> 61
gaacctgaat gggagtgcca                                                  20

<210> 62
<211> 20
<212> DNA
<213> Equus caballus

<400> 62
ttctcctcac acacatgggc                                                  20

<210> 63
<211> 20
<212> DNA
<213> Equus caballus

<400> 63
ttctcctcac acacatgggc                                                  20

<210> 64
<211> 23
<212> DNA
<213> Equus caballus

<400> 64
agcgtatata cctattggtc agg                                              23

<210> 65
<211> 23
<212> DNA
<213> Equus caballus

<400> 65
tcggaagcgt atatacctat tgg                                              23

<210> 66
<211> 23
<212> DNA
<213> Equus caballus

<400> 66
gcgtatatac ctattggtca ggg                                              23

<210> 67
<211> 23
<212> DNA
<213> Equus caballus

<400> 67
gatatgacac tttctatgta agg                                              23

<210> 68
<211> 369
<212> DNA
<213> Equus caballus

<220>
<223> (derived from NC_009168.2 Reference EquCab2.0 -
      ENSECAG00000022868)

<400> 68
acttctccca catgccatgg atacagctga gtcccctcca taccatgaaa gtttttgaaa      60

ttaagcgaga gaggagatgg caagatgtca gtatgatgcg catgaagact atcagtgaac     120

atgttgtaaa tcacatccaa tatgaagttg acttcctctt ctgcatggac gtggatcaaa     180

tcttcttaag aaaatatgga ttggagactc tgggggagtc agtagctcaa ttacatgctt     240

attggtataa tgcaaatcct atggaggtac cttatgagag aagtcagtta tcggaagcgt     300

atatacctat tggtcaggga gatttttatt accatgctgc tgtatttggt ggcactccca     360

ttcaggttc                                                             369

<210> 69
<211> 20
<212> DNA
<213> Equus caballus

<400> 69
atttggaggg aggggctttg                                                  20

<210> 70
<211> 20
<212> DNA
<213> Equus caballus

<400> 70
gagataccct cagcgagcac                                                    20

<210> 71
<211> 20
<212> DNA
<213> Equus caballus

<400> 71
ttctggtggc tccgttgatc                                                    20

<210> 72
<211> 20
<212> DNA
<213> Equus caballus

<400> 72
accaacccta atgcccacag                                                    20

<210> 73
<211> 20
<212> DNA
<213> Equus caballus

<400> 73
ttctggtggc tccgttgatc                                                    20

<210> 74
<211> 20
<212> DNA
<213> Equus caballus

<400> 74
accaacccta atgcccacag                                                    20

<210> 75
<211> 23
<212> DNA
<213> Equus caballus

<400> 75
tattcttgca cgctcgcagg cgg                                                23

<210> 76
<211> 23
<212> DNA
<213> Equus caballus

<400> 76
cggagggttg atatacttca tgg                                                23

<210> 77
<211> 23
<212> DNA
<213> Equus caballus

<400> 77
tccctccaaa tacctaggct tgg                                              23

<210> 78
<211> 391
<212> DNA
<213> Equus caballus

<220>
<223> (derived from NC_009163.2 Reference EquCab2.0 -
      ENSECAG00000009766)

<400> 78
gggggaaagc tgagatggac gttcaaccag tagaatgtat gtttaagaga agagataagg      60

tgcaccataa ggtagccacc aagcctaggt atttggaggg aggggctttg aaagtatttc     120

cataaaaaga ggtttttgaa ctgtcagttc caaatgaata tctcttaaag ccttaacatg     180

attttctttc ctctccaaat gtttgccttg tcatagcatc gaacaaacaa ctgagatcct     240

gttgcatctg tcacctgatg aagctgccaa tctgaaggaa gggatcaatt ttcttcgaaa     300

taagaagacg ggcaaagatt acatcttata taagaacaag agccgcctgc gagcgtgcaa     360

gaatatgtgc aagcatcaag gaggcctgtt c                                    391

<210> 79
<211> 481
<212> DNA
<213> Equus caballus

<220>
<223> (derived from NC_009163.2 Reference EquCab2.0 -
      ENSECAG00000009766)

<400> 79
atttggaggg aggggctttg aaagtatttc cataaaaaga ggtttttgaa ctgtcagttc      60

caaatgaata tctcttaaag ccttaacatg attttctttc ctctccaaat gtttgccttg     120

tcatagcatc gaacaaacaa ctgagatcct gttgcatctg tcacctgatg aagctgccaa     180

tctgaaggaa gggatcaatt ttcttcgaaa taagaagacg ggcaaagatt acatcttata     240

taagaacaag agccgcctgc gagcgtgcaa gaatatgtgc aagcatcaag gaggcctgtt     300

cataaaagat attgaggatt tcgacggaag gtaccaagag tcctttgctt tctcaccagc     360

gacacttctt aaggggattt ggcagaaaat gtgctcattt aaaaatttca aagggaccaa      420

tcaacagatt atttccccta gtcaatccat cactttcact tgtgctcgct gagggtatct     480

c                                                                   481

<210> 80
<211> 472
<212> DNA
<213> Equus caballus

<220>
<223> (derived from NC_009163.2 Reference EquCab2.0 -
      ENSECAG00000009766)

<400> 80
ttctggtggc tccgttgatc tctcacttgt catttcatga cgtgaagttt atcttcattt      60

tttaaatcac ctactgataa ttagtctata tctccttggg tatttgaatg ttagctatta     120

atagttttct tctcatgtat tttgtgttca atcaactagt gtaattttgt cacaaaaata     180

atacgtttta gcacgataag attaattttt attttcgccg tcataaacct aagcacaatt     240

tttcatccag gtctgttagg tgcacaaaac acaactggaa gttagacgtg agctccatga     300

agtatatcaa ccctccgggc agcttctgtc aagatgaact gggtaaatac catcaagttg     360

ataaatgtat tttgctatta tcgttattgg ggttcttctt gtcaacttgt ttccagtctc     420

acttgaagct ttggatacat tatgatttta agctgtgggc attagggttg gt             472

<210> 81
<211> 21
<212> DNA
<213> Equus caballus

<400> 81
gctcttttgt gtcccaacag c                                                21

<210> 82
<211> 20
<212> DNA
<213> Equus caballus

<400> 82
aaaccacagg aggagcaagg                                                  20

<210> 83
<211> 20
<212> DNA
<213> Equus caballus

<400> 83
cctgccattc tctgctcagt                                                  20

<210> 84

<211> 20
<212> DNA
<213> Equus caballus

<400> 84
aagcctcagc atctggatgg                                                    20

<210> 85
<211> 23
<212> DNA
<213> Equus caballus

<400> 85
ggctattgaa ctgtgcgaga agg                                                23

<210> 86
<211> 23
<212> DNA
<213> Equus caballus

<400> 86
aagttgtcag tcttaatcgc agg                                                23

<210> 87
<211> 23
<212> DNA
<213> Equus caballus

<400> 87
ctattgaact gtgcgagaag ggg                                                23

<210> 88
<211> 23
<212> DNA
<213> Equus caballus

<400> 88
gctattgaac tgtgcgagaa ggg                                                23

<210> 89
<211> 23
<212> DNA
<213> Equus caballus

<400> 89
tcagtcttaa tcgcaggact tgg                                                23

<210> 90
<211> 689
<212> DNA
<213> Equus caballus

<220>
<223> (derived from NC_009154.2 Reference EquCab2.0)

<400> 90
cctgccattc tctgctcagt gtgcttttat gcgattattt agtggataaa gttcatgacc      60

actacctctg ttttgtggat catgcacttg ctcattataa gtgatcattt ctgtcctatt     120

taatactttt ttttgtcttt aattcaacta tctgacatta atattgcaaa ccttgctctc     180

cttttttttc tcttgggatg tttttgccca ttcttttatt tgtgactttt ctgagtcttt     240

ttgttgcaat gtgattcttt actgttttat ttatcaaatc aattttagta aataggataa     300

gatataggtt taacttatat tttccctcaa cttagtattt ccgtatgtat gacacaatca     360

agtaatttat tccataatca agaaaaaaga ttttaacaga atttgctctt ttgtgtccca     420

acagctctag atttccatgg ctcctcaagt tgtcagtctt aatcgcagga cttggcattg     480

ttttatttac attcagaagt gtgccccttc tcgcacagtt caatagcctc ataccagggc     540

tcccacgtcc tgctccagcc atccagatgc tgaggcttct gcctgaggaa cgtctcagga     600

acctctttac ctacaatgga atctggtgag atagcaatgt cccctctctt ccccttggtc     660

caacacctcc cttgctcctc ctgtggttt                                       689


<210> 91
<211> 383
<212> DNA
<213> Sus scrofa


<220>
<223> Resulting edited sequence - Deletion 109 bp.

<400> 91
ggaccttggg atcagtcagg atgcttcccc tttgagcctc aaaatggcct tagtatcctt      60

cccaacccag acggccctgt cagttcattg acttggctaa tttgccagtg taggcctatg     120

caaattaagg tagaacgcac tccttagcgc tcgttgacta ttcatcaact tttcctttta     180

gaaaagatat tggtataagc acttcttaaa aaaccaactg taatggttgt gttttgggaa     240

tacatcaaca ggtaattatg aaacatgatg aaatgatgtt gatgaaagtc tcctctaatc     300

tcctagttat cagccaagtc accagcttgc attaaaagta ggattcactg acaccgtaaa     360

gaaagcattc cagagagttg ccg                                             383


<210> 92
<211> 494
<212> DNA
<213> Sus scrofa


<220>

<223> Resulting edited sequence \226 Allele A - Insertion 2 bp.

<400> 92
ggaccttggg atcagtcagg atgcttcccc tttgagcctc aaaatggcct tagtatcctt      60

cccaacccag acggccctgt cagttcattg acttggctaa tttgccagtg taggcctatg     120

caaattaagg tagaacgcac tccttagcgc tcgttgacta ttcatcaact tttcctttta     180

gaaaagatat tggtataagc acttcttaaa aaaccatatt ccactctggg tgtatttaat     240

ctaattttcc cttctccttt tcttttccca ggagaaaata atgaatgtca aaggaagagt     300

ggttctgtgt caatgctgct tgtctcaact gtaatggttg tgttttggga atacatcaac     360

aggtaattat gaaacatgat gaaatgatgt tgatgaaagt ctcctctaat ctcctagtta     420

tcagccaagt caccagcttg cattaaaagt aggattcact gacaccgtaa agaaagcatt     480

ccagagagtt gccg                                                       494


<210> 93
<211> 864
<212> DNA
<213> Sus scrofa


<220>
<223> Resulting edited sequence - Allele B - Insertion 373bp + del 1
      bp.

<400> 93
ggaccttggg atcagtcagg atgcttcccc tttgagcctc aaaatggcct tagtatcctt      60

cccaacccag acggccctgt cagttcattg acttggctaa tttgccagtg taggcctatg     120

caaattaagg tagaacgcac tccttagcgc tcgttgacta ttcatcaact tttcctttta     180

gaaaagatat tggtataagc acttcttaaa aaaccatatt ccactctggg tgtatttaat     240

ctaattttcc cttctccttt tcttttccca ggagaaaata atgaatgtca aaggaagagt     300

ggttcttagg ggttccgcgc acatttcccc gaaaagtgcc acctaaattg taagcgttaa     360

tattttgtta aaattcgcgt taaatttttg ttaaatcagc tcatttttta accaataggc     420

cgaaatcggc aaaatccctt ataaatcaaa agaatagacc gagatagggc tggccccagc     480

gctgcgatga taccgcgaga accacgctca ccggctccag atttatcagc aataaaccag     540

ccagccggaa gggccgagcg cagaagtggt cctgcaactt tatccgcctc catccagtct     600

attaattgtt gccgggaagc tagagtaagt agttcgccag ttaatagttt gcgcaacgtt     660

gttgcccatc gctacaggct caatgctgct tgtctcaact gtaatggttg tgttttggga     720

atacatcaac aggtaattat gaaacatgat gaaatgatgt tgatgaaagt ctcctctaat     780

ctcctagtta tcagccaagt caccagcttg cattaaaagt aggattcact gacaccgtaa     840

agaaagcatt ccagagagtt gccg                                            864

<210> 94
<211> 491
<212> DNA
<213> Sus scrofa

<220>
<223> Resulting edited sequence - Deletion 1 bp

<400> 94
ggaccttggg atcagtcagg atgcttcccc tttgagcctc aaaatggcct tagtatcctt 60

cccaacccag acggccctgt cagttcattg acttggctaa tttgccagtg taggcctatg 120

caaattaagg tagaacgcac tccttagcgc tcgttgacta ttcatcaact tttcctttta 180

gaaaagatat tggtataagc acttcttaaa aaaccatatt ccactctggg tgtatttaat 240

ctaattttcc cttctccttt tcttttccca ggagaaaata atgaatgtca aaggaagagt 300

ggtctgtcaa tgctgcttgt ctcaactgta atggttgtgt tttgggaata catcaacagg 360

taattatgaa acatgatgaa atgatgttga tgaaagtctc ctctaatctc ctagttatca 420

gccaagtcac cagcttgcat taaaagtagg attcactgac accgtaaaga aagcattcca 480

gagagttgcc g 491

<210> 95
<211> 246
<212> DNA
<213> Sus scrofa

<220>
<223> Resulting edited sequence \226 Allele A - Deletion 246 bp (del 247
      bp + in A

<400> 95
ggaccttggg atcagtcagg atgcttcccc tttgagcctc aaaatggcct tagtatcctt 60

cccaacccag acggccctgt cagttcattg acttggctaa tttgccagtg taggcctatg 120

caaattaagg tagaacgcac tccttagcgc tcgttgacta ttcatatagt tatcagccaa 180

gtcaccagct tgcattaaaa gtaggattca ctgacaccgt aaagaaagca ttccagagag 240

ttgccg 246

<210> 96
<211> 347
<212> DNA
<213> Sus scrofa

<220>
<223> Resulting edited sequence \226 Allele B - Deletion 145 bp

<400> 96
ggaccttggg atcagtcagg atgcttcccc tttgagcctc aaaatggcct tagtatcctt 60

cccaacccag acggccctgt cagttcattg acttggctaa tttgccagtg taggcctatg 120

caaattaagg tagaacgcac tccttagcgc tcgttgacta ttcatcaact gcttgtctca 180

actgtaatgg ttgtgttttg ggaatacatc aacaggtaat tatgaaacat gatgaaatga 240

tgttgatgaa agtctcctct aatctcctag ttatcagcca agtcaccagc ttgcattaaa 300

agtaggattc actgacaccg taaagaaagc attccagaga gttgccg 347

<210> 97
<211> 491
<212> DNA
<213> Sus scrofa

<220>
<223> Resulting edited sequence \226 Allele A \226 Deletion 1 bp

<400> 97
ggaccttggg atcagtcagg atgcttcccc tttgagcctc aaaatggcct tagtatcctt 60

cccaacccag acggccctgt cagttcattg acttggctaa tttgccagtg taggcctatg 120

caaattaagg tagaacgcac tccttagcgc tcgttgacta ttcatcaact tttcctttta 180

gaaaagatat tggtataagc acttcttaaa aaaccatatt ccactctggg tgtatttaat 240

ctaattttcc cttctccttt tcttttccca ggagaaaata atgaatgtca aaggaagagt 300

ggtctgtcaa tgctgcttgt ctcaactgta atggttgtgt tttgggaata catcaacagg 360

taattatgaa acatgatgaa atgatgttga tgaaagtctc ctctaatctc ctagttatca 420

gccaagtcac cagcttgcat taaaagtagg attcactgac accgtaaaga aagcattcca 480

gagagttgcc g 491

<210> 98
<211> 487
<212> DNA
<213> Sus scrofa

<220>
<223> Resulting edited sequence \226 Allele B - Deletion 5 bp

<400> 98
ggaccttggg atcagtcagg atgcttcccc tttgagcctc aaaatggcct tagtatcctt 60

cccaacccag acggccctgt cagttcattg acttggctaa tttgccagtg taggcctatg 120

caaattaagg tagaacgcac tccttagcgc tcgttgacta ttcatcaact tttcctttta 180

gaaaagatat tggtataagc acttcttaaa aaaccatatt ccactctggg tgtatttaat 240

ctaattttcc cttctccttt tcttttccca ggagaaaata atgaatgtca aaggaagagt 300

ggttaatgct gcttgtctca actgtaatgg ttgtgttttg ggaatacatc aacaggtaat 360

tatgaaacat gatgaaatga tgttgatgaa agtctcctct aatctcctag ttatcagcca 420

agtcaccagc ttgcattaaa agtaggattc actgacaccg taaagaaagc attccagaga 480

gttgccg 487

<210> 99
<211> 305
<212> DNA
<213> Sus scrofa

<220>
<223> Resulting edited sequence - Deletion 22 bp.

<400> 99
cctttcatga gttttgtgtt caaccaacta cagtcgttta ctttgatcac agaataatgc 60

atttaagcct taaatagatt aatatttatt ttcaccattt cataaaccta agtacaattt 120

tccatccagg tctgttaaat gcacaaaaca caactggaag ttagatgtaa gcagcatgaa 180

gtatatcaag acgaactggg taaataccat caatactgat caatgttttc tgctgttact 240

gtcattgggg tccctcttgt caacttgttt ccaatctcat tagaagcctt ggatgcattc 300

tgatt 305

<210> 100
<211> 321
<212> DNA
<213> Sus scrofa

<220>
<223> Resulting edited sequence - Allele A - Deletion 6 bp.

<400> 100
cctttcatga gttttgtgtt caaccaacta cagtcgttta ctttgatcac agaataatgc 60

atttaagcct taaatagatt aatatttatt ttcaccattt cataaaccta agtacaattt 120

tccatccagg tctgttaaat gcacaaaaca caactggaag ttagatgtaa gcagcatgaa 180

gtatatcaat cctcctggaa gtcaagacga actgggtaaa taccatcaat actgatcaat 240

gttttctgct gttactgtca ttggggtccc tcttgtcaac ttgtttccaa tctcattaga 300

agccttggat gcattctgat t 321

<210> 101
<211> 324
<212> DNA
<213> Sus scrofa

<220>
<223> Resulting edited sequence - Allele B - Deletion 3 bp.

<400> 101

cctttcatga gttttgtgtt caaccaacta cagtcgttta ctttgatcac agaataatgc 60

atttaagcct taaatagatt aatatttatt ttcaccattt cataaaccta agtacaattt 120

tccatccagg tctgttaaat gcacaaaaca caactggaag ttagatgtaa gcagcatgaa 180

gtatatcaat cctcctggaa gctgtcaaga cgaactgggt aaataccatc aatactgatc 240

aatgttttct gctgttactg tcattggggt ccctcttgtc aacttgtttc caatctcatt 300

agaagccttg gatgcattct gatt 324

<210> 102
<211> 1390
<212> DNA
<213> Sus scrofa

<220>
<223> Resulting edited sequence - Deletion 715 bp.

<400> 102
ccctatctcc aactatcaca tccctatcac cgtctggtaa gaaatggaaa aaatattact 60

aagcctcctt tgttgctaca attaatctga ttctcattct gaagcagtgt tgccagagtt 120

aacaaataaa aatgcaaagc tgagtagtta aatttgaatt acagataaac aaatttccaa 180

tatatgttca atattgtgta agacatttta aaattatttt ttatttatct gaaatttata 240

tttttcctgt attttatctg gcaaccatga tcagaaatct ttaaacaatc aggaagtctt 300

ttttcttaga caaatgaaaa tttgagttga tcttaggttt agtacactat actaggggcc 360

aagggttata gtgtgactat taaatcacag ataatcttta ttactacatt atttccttat 420

actggcccca cttggatctt actcagctta gcttttgtat gagagtcatc cttaaagatg 480

actttattct gtcaagacga actgggtaaa taccatcaat actgatcaat gttttctgct 540

gttactgtca ttggggtccc tcttgtcaac ttgtttccaa tctcattaga agccttggat 600

gcattctgat tttaaactga ggtattttaa aagtaaccat cactgaaaat tctaggcaag 660

ttttctctaa aaaatccctt cattcattca tttgttcagt aagtatttga tgagacctta 720

ccatgtgtaa acattgcact aggtattaag aaatacaaag atggataaga tagagtcggc 780

gtaaatgaga tgatataatg agacgttata atgaaactca caattccagt tgggaaataa 840

agtccttcaa attccatgac tctttctggc acacgttaga ggctacagct tctgtgtgat 900

tctcatgctg gctccacttc tactttttcc ttcttcctac tcaagaaagc ctatagaaat 960

atgagtaaga agggcttaat cataggaata aatttgtctc tgttctaagt gattaaaaat 1020

gtctttatca gtataaaaag ttacttggga agattcttaa aactgctttt acacactgtt 1080

ttagaatgac tgttatataa ataaaaaagt agatttgatc taacacaatt aaatgacttt 1140

tggaaatatt gactaattct caccttgccc ctcaaaggga tgcctgaacc atttccttct 1200

tttgccagaa agcccccacc ctttgtctgt tgacctagcc taggaaatct tcagatcaca 1260

ttgttagcag gaactggtta catgtgctgt acaaatacta tttaattcat ctgattaaaa 1320

aaaaaaagag ataagaagca aaagtttgac tgtcttaaac tgtttgtgta ggtgagagga 1380

caattgacca 1390

<210> 103
<211> 254
<212> DNA
<213> Sus scrofa

<220>
<223> Resulting edited sequence \226 Allele A - Deletion 73 bp.

<400> 103
cctttcatga gttttgtgtt caaccaacta cagtcgttta ctttgatcac agaataatgc 60

atttaagcct taaatagatt aatatttatt ttcaccattt cataaaccta agtacaattt 120

tccatccagg tctgtcaaga cgaactgggt aaataccatc aatactgatc aatgttttct 180

gctgttactg tcattggggt ccctcttgtc aacttgtttc caatctcatt agaagccttg 240

gatgcattct gatt 254

<210> 104
<211> 323
<212> DNA
<213> Sus scrofa

<220>
<223> Resulting edited sequence \226 Allele B - Deletion 4 bp (del AGCTT +
      in G)

<400> 104
cctttcatga gttttgtgtt caaccaacta cagtcgttta ctttgatcac agaataatgc 60

atttaagcct taaatagatt aatatttatt ttcaccattt cataaaccta agtacaattt 120

tccatccagg tctgttaaat gcacaaaaca caactggaag ttagatgtaa gcagcatgaa 180

gtatatcaat cctcctggag ctgtcaagac gaactgggta aataccatca atactgatca 240

atgttttctg ctgttactgt cattggggtc cctcttgtca acttgtttcc aatctcatta 300

gaagccttgg atgcattctg att 323

<210> 105
<211> 325
<212> DNA
<213> Sus scrofa

<220>
<223> Resulting edited sequence \226 Deletion 2bp.

<400> 105
cctttcatga gttttgtgtt caaccaacta cagtcgttta ctttgatcac agaataatgc      60

atttaagcct taaatagatt aatatttatt ttcaccattt cataaaccta agtacaattt     120

tccatccagg tctgttaaat gcacaaaaca caactggaag ttagatgtaa gcagcatgaa     180

gtatatcaat cctcctggaa gcctgtcaag acgaactggg taaataccat caatactgat     240

caatgttttc tgctgttact gtcattgggg tccctcttgt caacttgttt ccaatctcat     300

tagaagcctt ggatgcattc tgatt                                           325


<210> 106
<211> 454
<212> DNA
<213> Sus scrofa


<220>
<223> Resulting edited sequence \226 Allele A - Deletion 12 bp.

<400> 106
cgcaagtgac cagacatcgt tcccagtgga tcccagcagg atctttttgg gttattttat      60

ggtttccttt gctgtgcaaa agcttgtcag tttgcttagg tttatttttg cttttctttc      120

tcttttgcct tgggaagaaa tcaattttga tacgtagtgt acaaaacagg tttgacttgc     180

atttttgctc aagttgctat tcccatctat gtcgcacaat gaaatctttt ttaacatact     240

cagaaaagat tttaacagcg tttgctctct tgtgtcccaa cagccctaga tgtctgtcga     300

tcctcaagat attgatggtg cttttggtcc tgagcgttgg actctttatg ttccatcgat     360

acagacttca gtctcctcaa ctcacccatc ccgtccccca ccctggatgc gcagacgctg     420

aagcttctac ctgagaaacc cgatttctac ggtg                                 454


<210> 107
<211> 455
<212> DNA
<213> Sus scrofa


<220>
<223> Resulting edited sequence \226 Allele B - Deletion 14 bp + insertion
      4 bp.

<400> 107
cgcaagtgac cagacatcgt tcccagtgga tcccagcagg atctttttgg gttattttat      60

ggtttccttt gctgtgcaaa agcttgtcag tttgcttagg tttatttttg cttttctttc      120

tcttttgcct tgggaagaaa tcaattttga tacgtagtgt acaaaacagg tttgacttgc     180

atttttgctc aagttgctat tcccatctat gtcgcacaat gaaatctttt ttaacatact     240

cagaaaagat tttaacagcg tttgctctct tgtgtcccaa cagccctaga tgtctgtcga     300

tcctcaagat attgatggtg cttttggtcc tgagcgttgg actctttatg ttccgaagga     360

tacagacttc agtctcctca actcacccat cccgtccccc accctggatg cgcagacgct 420

gaagcttcta cctgagaaac ccgatttcta cggtg 455

<210> 108
<211> 454
<212> DNA
<213> Sus scrofa

<220>
<223> Resulting edited sequence - Allele A - Deletion 12 bp.

<400> 108
cgcaagtgac cagacatcgt tcccagtgga tcccagcagg atctttttgg gttattttat 60

ggtttccttt gctgtgcaaa agcttgtcag tttgcttagg tttatttttg cttttctttc 120

tcttttgcct tgggaagaaa tcaattttga tacgtagtgt acaaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat attgatggtg cttttggtcc tgagcgttgg actctttatg ttccatcgat 360

acagacttca gtctcctcaa ctcacccatc ccgtccccca ccctggatgc gcagacgctg 420

aagcttctac ctgagaaacc cgatttctac ggtg 454

<210> 109
<211> 455
<212> DNA
<213> Sus scrofa

<220>
<223> Resulting edited sequence \226 Allele B - Deletion 14 bp + insertion
      4 bp.

<400> 109
cgcaagtgac cagacatcgt tcccagtgga tcccagcagg atctttttgg gttattttat 60

ggtttccttt gctgtgcaaa agcttgtcag tttgcttagg tttatttttg cttttctttc 120

tcttttgcct tgggaagaaa tcaattttga tacgtagtgt acaaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat attgatggtg cttttggtcc tgagcgttgg actctttatg ttccaaagga 360

tccagacttc agtctcctca actcacccat cccgtccccc accctggatg cgcagacgct 420

gaagcttcta cctgagaaac ccgatttcta cggtg 455

<210> 110
<211> 453

<212> DNA
<213> Sus scrofa

<220>
<223> Resulting edited sequence \226 Allele A - Deletion 12 bp.

<400> 110
cgcaagtgac cagacatcgt tcccagtgga tcccagcagg atctttttgg gttattttat      60

ggtttccttt gctgtgcaaa agcttgtcag tttgcttagg tttatttttg cttttctttc     120

tcttttgcct tgggaagaaa tcaattttga tacgtagtgt acaaaacagg tttgacttgc     180

atttttgctc aagttgctat tcccatctat gtcgcacaat gaaatctttt ttaacatact     240

cagaaaagat tttaacagcg tttgctctct tgtgtcccaa cagccctaga tgtctgtcga     300

tcctcaagat attgatggtg cttttggtcc tgagcgttgg actctttatg ttcctcgata     360

cagacttcag tctcctcaac tcacccatcc cgtcccccac cctggatgcg cagacgctga     420

agcttctacc tgagaaaccc gatttctacg gtg                                  453

<210> 111
<211> 465
<212> DNA
<213> Sus scrofa

<220>
<223> Resulting edited sequence - Allele B - Substitution AAAGC to
      GAAGT (Q29R).

<400> 111
cgcaagtgac cagacatcgt tcccagtgga tcccagcagg atctttttgg gttattttat      60

ggtttccttt gctgtgcaaa agcttgtcag tttgcttagg tttatttttg cttttctttc     120

tcttttgcct tgggaagaaa tcaattttga tacgtagtgt acaaaacagg tttgacttgc     180

atttttgctc aagttgctat tcccatctat gtcgcacaat gaaatctttt ttaacatact     240

cagaaaagat tttaacagcg tttgctctct tgtgtcccaa cagccctaga tgtctgtcga     300

tcctcaagat attgatggtg cttttggtcc tgagcgttgg actctttatg ttccgaagtg     360

tgttcctcga tacagacttc agtctcctca actcacccat cccgtccccc accctggatg     420

cgcagacgct gaagcttcta cctgagaaac ccgatttcta cggtg                     465

<210> 112
<211> 467
<212> DNA
<213> Sus scrofa

<220>
<223> Resulting edited sequence - Allele A \226 Insertion 2 bp.

<400> 112

cgcaagtgac cagacatcgt tcccagtgga tcccagcagg atctttttgg gttattttat 60

ggtttccttt gctgtgcaaa agcttgtcag tttgcttagg tttatttttg cttttctttc 120

tcttttgcct tgggaagaaa tcaattttga tacgtagtgt acaaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat attgatggtg cttttggtcc tgagcgttgg actctttatg tgttccaaag 360

cgtgttcctc gatacagact tcagtctcct caactcaccc atcccgtccc ccaccctgga 420

tgcgcagacg ctgaagcttc tacctgagaa acccgatttc tacggtg 467

<210> 113
<211> 453
<212> DNA
<213> Sus scrofa

<220>
<223> Resulting edited sequence \226 Deletion 12 bp.

<400> 113
cgcaagtgac cagacatcgt tcccagtgga tcccagcagg atctttttgg gttattttat 60

ggtttccttt gctgtgcaaa agcttgtcag tttgcttagg tttatttttg cttttctttc 120

tcttttgcct tgggaagaaa tcaattttga tacgtagtgt acaaaacagg tttgacttgc 180

atttttgctc aagttgctat tcccatctat gtcgcacaat gaaatctttt ttaacatact 240

cagaaaagat tttaacagcg tttgctctct tgtgtcccaa cagccctaga tgtctgtcga 300

tcctcaagat attgatggtg cttttggtcc tgagcgttgg actctttatg ttcctcgata 360

cagacttcag tctcctcaac tcacccatcc cgtcccccac cctggatgcg cagacgctga 420

agcttctacc tgagaaaccc gatttctacg gtg 453

<210> 114
<211> 186
<212> DNA
<213> Sus scrofa

<220>
<223> Resulting edited sequence \226 Deletion 2 bp (del GAACC + in AAA).

<400> 114
ccctagatgt ctgtcgatcc tcaagatttc gatggtgctt ttggtcctga gcgttggact 60

ctttatgttc caaagtgttc ctcgatacag acttcagtct cctcaactca cccatcccgt 120

cccccaccct ggatgcgcag acgctgaagc ttctacctga gaaacatgtc aggagcctct 180

tcacct 186

<210> 115
<211> 176
<212> DNA
<213> Sus scrofa

<220>
<223> Resulting edited sequence \226 Allele A - Deletion 12 bp.

<400> 115
ccctagatgt ctgtcgatcc tcaagatttc gatggtgctt ttggtcctga gcgttggact      60

ctttatgttc ctcgatacag acttcagtct cctcaactca cccatcccgt cccccaccct     120

ggatgcgcag acgctgaagc ttctacctga gaaacatgtc aggagcctct tcacct         176

<210> 116
<211> 178
<212> DNA
<213> Sus scrofa

<220>
<223> Resulting edited sequence \226 Allele B - Deletion 10 bp (del
      TGTGTTCCTCGATA + in GATC)

<400> 116
ccctagatgt ctgtcgatcc tcaagatttc gatggtgctt ttggtcctga gcgttggact      60

ctttatgttc cgaaggatcc agacttcagt ctcctcaact cacccatccc gtcccccacc     120

ctggatgcgc agacgctgaa gcttctacct gagaaacatg tcaggagcct cttcacct       178

<210> 117
<211> 176
<212> DNA
<213> Sus scrofa

<220>
<223> Resulting edited sequence - Deletion 12 bp.

<400> 117
ccctagatgt ctgtcgatcc tcaagatttc gatggtgctt ttggtcctga gcgttggact      60

ctttatgttc ctcgatacag acttcagtct cctcaactca cccatcccgt cccccaccct     120

ggatgcgcag acgctgaagc ttctacctga gaaacatgtc aggagcctct tcacct         176

<210> 118
<211> 189
<212> DNA
<213> Sus scrofa

<220>
<223> Resulting edited sequence - Insertion 1 bp (del C + in TT)

<400> 118
ccctagatgt ctgtcgatcc tcaagatttc gatggtgctt ttggtcctga gcgttggact      60

ctttatgttc cgaagttgtg ttcctcgata cagacttcag tctcctcaac tcacccatcc 120

cgtcccccac cctggatgcg cagacgctga agcttctacc tgagaaacat gtcaggagcc 180

tcttcacct 189

<210> 119
<211> 144
<212> DNA
<213> Sus scrofa

<220>
<223> Resulting edited sequence \226 Allele A - deletion 44 bp.

<400> 119
ccctagatgt ctgtcgatcc tcaagatttc gatggtgctt ttggtcctga gcgttggact 60

ctttatgttc catcccgtcc cccaccctgg atgcgcagac gctgaagctt ctacctgaga 120

aacatgtcag gagcctcttc acct 144

<210> 120
<211> 419
<212> DNA
<213> Bos taurus

<220>
<223> Resulting edited sequence \226 Allele A - Deletion 21 bp.

<400> 120
ggatgccttt gatagagttg ggtcctctgc gctccttcaa agtgtttaag atcaagcctg 60

agaagaggtg gcaggacatc agcatgatgc gcatgaagac tatcggggag cacattgtgg 120

cccacatcca gcatgaggtt gacttccttt tctgcatgga tgtggaccag gtcttccaag 180

acaagtttgg ggtggcccag ctacaagcct ggtggtacaa ggcagatccc aatgacttca 240

cctacgagag gcggaaggag tctgcagcat acattccctt cggcgaaggg gatttttatt 300

accatgcagc catttttggg ggaacaccca ctcaggtcct taacatcacc caggaatgct 360

tcaaaggaat cctcaaggac aagaaaaatg acatagaagc ccaatggcat gatgaaagc 419

<210> 121
<211> 269
<212> DNA
<213> Bos taurus

<220>
<223> Resulting edited sequence \226 Allele B - Deletion 171 bp.

<400> 121
ggatgccttt gatagagttg ggtcctctgc gctccttcaa agtggcccag ctacaagcct 60

ggtggtacaa ggcagatccc aatgacttca cctacgagag gcggaaggag tctgcagcat 120

acattccctt cggcgaaggg gatttttatt accatgcagc catttttggg ggaacaccca 180

ctcaggtcct taacatcacc caggaatgct tcaaaggaat cctcaaggac aagaaaaatg 240

acatagaagc ccaatggcat gatgaaagc 269

<210> 122
<211> 432
<212> DNA
<213> Bos taurus

<220>
<223> Resulting edited sequence \226 Allele A - Deletion 8 bp.

<400> 122
ggatgccttt gatagagttg ggtcctctgc gctccttcaa agtgtttaag atcaagcctg 60

agaagaggtg gcaggacatc agcatgatgc gcatgaagac tatcggggag cacattgtgg 120

cccacatcca gcatgaggtt gacttccttt tctgcatgga tgtggaccag gtcttccaag 180

acaagtttgg ggtggagacc ctgggcggcc cagctacaag cctggtggta caaggcagat 240

cccaatgact tcacctacga gaggcggaag gagtctgcag catacattcc cttcggcgaa 300

ggggattttt attaccatgc agccattttt gggggaacac ccactcaggt ccttaacatc 360

acccaggaat gcttcaaagg aatcctcaag gacaagaaaa atgacataga agcccaatgg 420

catgatgaaa gc 432

<210> 123
<211> 411
<212> DNA
<213> Bos taurus

<220>
<223> Resulting edited sequence - Deletion 29 bp.

<400> 123
ggatgccttt gatagagttg ggtcctctgc gctccttcaa agtgtttaag atcaagcctg 60

agaagaggtg gcaggacatc agcatgatgc gcatgaagac tatcggggag cacattgtgg 120

cccacatcca gcatgaggtt gacttccttt tctgcatgga tgtggaccag gtcttccaag 180

acaagtttgg ggtggagacc ctggtggtac aaggcagatc ccaatgactt cacctacgag 240

aggcggaagg agtctgcagc atacattccc ttcggcgaag gggattttta ttaccatgca 300

gccatttttg ggggaacacc cactcaggtc cttaacatca cccaggaatg cttcaaagga 360

atcctcaagg acaagaaaaa tgacatagaa gcccaatggc atgatgaaag c 411

<210> 124
<211> 424
<212> DNA

<213> Bos taurus

<220>
<223> Resulting edited sequence \226 Allele A - Deletion 16 bp.

<400> 124
ggatgccttt gatagagttg ggtcctctgc gctccttcaa agtgtttaag atcaagcctg 60

agaagaggtg gcaggacatc agcatgatgc gcatgaagac tatcggggag cacattgtgg 120

cccacatcca gcatgaggtt gacttccttt tctgcatgga tgtggaccag gtcttccaag 180

acaagtttgg ggtggagacc ctgggctaca agcctggtgg tacaaggcag atcccaatga 240

cttcacctac gagaggcgga aggagtctgc agcatacatt cccttcggcg aaggggattt 300

ttattaccat gcagccattt ttgggggaac acccactcag gtccttaaca tcacccagga 360

atgcttcaaa ggaatcctca aggacaagaa aaatgacata gaagcccaat ggcatgatga 420

aagc 424

<210> 125
<211> 428
<212> DNA
<213> Bos taurus

<220>
<223> Resulting edited sequence \226 Allele B - Deletion 12 bp.

<400> 125
ggatgccttt gatagagttg ggtcctctgc gctccttcaa agtgtttaag atcaagcctg 60

agaagaggtg gcaggacatc agcatgatgc gcatgaagac tatcggggag cacattgtgg 120

cccacatcca gcatgaggtt gacttccttt tctgcatgga tgtggaccag gtcttccaag 180

acaagtttgg ggtggagacc ctgggcgagc tacaagcctg gtggtacaag gcagatccca 240

atgacttcac ctacgagagg cggaaggagt ctgcagcata cattcccttc ggcgaagggg 300

atttttatta ccatgcagcc atttttgggg gaacacccac tcaggtcctt aacatcaccc 360

aggaatgctt caaaggaatc ctcaaggaca agaaaaatga catagaagcc caatggcatg 420

atgaaagc 428

<210> 126
<211> 208
<212> DNA
<213> Bos taurus

<220>
<223> Resulting edited sequence - Deletion 17 bp.

<400> 126
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag actatgggca ttactttgct gggaaggtgg 180

ggtcaaccct cgacaagtag gatgggca 208

<210> 127
<211> 225
<212> DNA
<213> Bos taurus

<220>
<223> Resulting edited sequence - Allele A - Substitution of ATG to
      TAA.

<400> 127
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag acttaaggca ggcaagtgag ggaggcatta 180

ctttgctggg aaggtggggt caaccctcga caagtaggat gggca 225

<210> 128
<211> 212
<212> DNA
<213> Bos taurus

<220>
<223> Resulting edited sequence - Allele B - Deletion 13 bp.

<400> 128
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag actatgggca ggcattactt tgctgggaag 180

gtggggtcaa ccctcgacaa gtaggatggg ca 212

<210> 129
<211> 208
<212> DNA
<213> Bos taurus

<220>
<223> Resulting edited sequence \226 Allele A - Substitution of ATG to
      TAA.

<400> 129
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag acttaaggca ttactttgct gggaaggtgg 180

ggtcaaccct cgacaagtag gatgggca 208

<210> 130
<211> 268
<212> DNA
<213> Bos taurus

<220>
<223> Resulting edited sequence \226 Allele B - Insertion 44 bp.

<400> 130
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag actatgccat tcttgtgaaa tacccaggga 180

gaggcaacga cggacttaag gcaggcaagt gagggaggca ttactttgct gggaaggtgg 240

ggtcaaccct cgacaagtag gatgggca 268

<210> 131
<211> 212
<212> DNA
<213> Bos taurus

<220>
<223> Resulting edited sequence - Deletion 13 bp.

<400> 131
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag actatgggca ggcattactt tgctgggaag 180

gtggggtcaa ccctcgacaa gtaggatggg ca 212

<210> 132
<211> 226
<212> DNA
<213> Bos taurus

<220>
<223> Resulting edited sequence - Insertion 1 bp.

<400> 132
tcaggaggag acatcaccaa cggaaatact gagctgagtg tacatcctct ttctccacgc 60

agacttctgc cttgggggga gtgaatgagc cacagaagct gaagtgacag ctgccattct 120

tctgaaatac ccagggagag gcaacgacag actatgggca ggcaagttga gggaggcatt 180

actttgctgg gaaggtgggg tcaaccctcg acaagtagga tgggca 226

<210> 133

<211> 25
<212> DNA
<213> Artificial Sequence

<220>
<223> Synthetic oligonucleotide

<400> 133
caccgctgta tcgaggaaca cgctt                                             25

<210> 134
<211> 25
<212> DNA
<213> Artificial Sequence

<220>
<223> Synthetic oligonucleotide

<400> 134
aaacaagcgt gttcctcgat acagc                                             25

<210> 135
<211> 25
<212> DNA
<213> Artificial Sequence

<220>
<223> Synthetic oligonucleotide

<400> 135
caccgacata aagagtccaa cgctc                                             25

<210> 136
<211> 25
<212> DNA
<213> Artificial Sequence

<220>
<223> Synthetic oligonucleotide

<400> 136
aaacgagcgt tggactcttt atgtc                                             25

<210> 137
<211> 25
<212> DNA
<213> Artificial Sequence

<220>
<223> Synthetic oligonucleotide

<400> 137
caccgcttca gcgtctgcgc atcca                                             25

<210> 138
<211> 25
<212> DNA
<213> Artificial Sequence

<220>
<223> Synthetic oligonucleotide

<400> 138
aaactggatg cgcagacgct gaagc                                              25

<210> 139
<211> 24
<212> DNA
<213> Artificial Sequence

<220>
<223> Synthetic oligonucleotide

<400> 139
caccggtgga gaccctgggc gagt                                               24

<210> 140
<211> 24
<212> DNA
<213> Artificial Sequence

<220>
<223> Synthetic oligonucleotide

<400> 140
aaacactcgc ccagggtctc cacc                                               24

<210> 141
<211> 24
<212> DNA
<213> Artificial Sequence

<220>
<223> Synthetic oligonucleotide

<400> 141
caccgctaca agcctggtgg taca                                               24

<210> 142
<211> 24
<212> DNA
<213> Artificial Sequence

<220>
<223> Synthetic oligonucleotide

<400> 142

aaactgtacc accaggcttg tagc                                                24

<210> 143
<211> 24
<212> DNA
<213> Artificial Sequence

<220>
<223> Synthetic oligonucleotide

<400> 143
caccgtacca ccaggcttgt agct                                                24

<210> 144
<211> 24
<212> DNA
<213> Artificial Sequence

<220>
<223> Synthetic oligonucleotide

<400> 144
aaacagctac aagcctggtg gtac                                                24

<210> 145
<211> 24
<212> DNA
<213> Artificial Sequence

<220>
<223> Synthetic oligonucleotide

<400> 145
caccgacact tcttttctta caat                                                24

<210> 146
<211> 24
<212> DNA
<213> Artificial Sequence

<220>
<223> Synthetic oligonucleotide

<400> 146
aaacattgta agaaaagaag tgtc                                                24

<210> 147
<211> 24
<212> DNA
<213> Artificial Sequence

<220>
<223> Synthetic oligonucleotide

<400> 147
caccggtttt cgccgtcgga aggt                                              24


<210> 148
<211> 24
<212> DNA
<213> Artificial Sequence


<220>
<223> Synthetic oligonucleotide

<400> 148
aaacaccttc cgacggcgaa aacc                                              24


<210> 149
<211> 24
<212> DNA
<213> Artificial Sequence


<220>
<223> Synthetic oligonucleotide

<400> 149
caccggagac cctgggcgag tcgg                                              24


<210> 150
<211> 24
<212> DNA
<213> Artificial Sequence


<220>
<223> Synthetic oligonucleotide

<400> 150
aaacccgact cgcccagggt ctcc                                              24


<210> 151
<211> 24
<212> DNA
<213> Artificial Sequence


<220>
<223> Synthetic oligonucleotide

<400> 151
caccgctggg ccaccgactc gccc                                              24


<210> 152
<211> 24
<212> DNA
<213> Artificial Sequence

<220>
<223> Synthetic oligonucleotide

<400> 152
aaacgggcga gtcggtggcc cagc                                              24


<210> 153
<211> 24
<212> DNA
<213> Artificial Sequence


<220>
<223> Synthetic oligonucleotide

<400> 153
caccggtctt ccaagacaag tttg                                              24


<210> 154
<211> 24
<212> DNA
<213> Artificial Sequence


<220>
<223> Synthetic oligonucleotide

<400> 154
aaaccaaact tgtcttggaa gacc                                              24


<210> 155
<211> 24
<212> DNA
<213> Artificial Sequence


<220>
<223> Synthetic oligonucleotide

<400> 155
caccgggatc tgccttgtac cacc                                              24


<210> 156
<211> 24
<212> DNA
<213> Artificial Sequence


<220>
<223> Synthetic oligonucleotide

<400> 156
aaacggtggt acaaggcaga tccc                                              24


<210> 157
<211> 24
<212> DNA
<213> Artificial Sequence

<220>
<223> Synthetic oligonucleotide

<400> 157
caccggtcca catccatgca gaaa                                              24


<210> 158
<211> 24
<212> DNA
<213> Artificial Sequence


<220>
<223> Synthetic oligonucleotide

<400> 158
aaactttctg catggatgtg gacc                                              24


<210> 159
<211> 24
<212> DNA
<213> Artificial Sequence


<220>
<223> Synthetic oligonucleotide

<400> 159
caccggttga cttccttttc tgca                                              24


<210> 160
<211> 24
<212> DNA
<213> Artificial Sequence


<220>
<223> Synthetic oligonucleotide

<400> 160
aaactgcaga aaaggaagtc aacc                                              24


<210> 161
<211> 24
<212> DNA
<213> Artificial Sequence


<220>
<223> Synthetic oligonucleotide

<400> 161
caccgtcaac ctcatgctgg atgt                                              24


<210> 162
<211> 24

<212> DNA
<213> Artificial Sequence

<220>
<223> Synthetic oligonucleotide

<400> 162
aaacacatcc agcatgaggt tgac                                              24

<210> 163
<211> 24
<212> DNA
<213> Artificial Sequence

<220>
<223> Synthetic oligonucleotide

<400> 163
caccgactat cggggagcac attg                                              24

<210> 164
<211> 24
<212> DNA
<213> Artificial Sequence

<220>
<223> Synthetic oligonucleotide

<400> 164
aaaccaatgt gctccccgat agtc                                              24

<210> 165
<211> 24
<212> DNA
<213> Artificial Sequence

<220>
<223> Synthetic oligonucleotide

<400> 165
caccgatgcg catgaagact atcg                                              24

<210> 166
<211> 24
<212> DNA
<213> Artificial Sequence

<220>
<223> Synthetic oligonucleotide

<400> 166
aaaccgatag tcttcatgcg catc                                              24

<210> 167
<211> 25
<212> DNA
<213> Artificial Sequence

<220>
<223> Synthetic oligonucleotide

<400> 167
caccgcatac attcccttcg gcgaa                                          25

<210> 168
<211> 25
<212> DNA
<213> Artificial Sequence

<220>
<223> Synthetic oligonucleotide

<400> 168
aaacttcgcc gaagggaatg tatgc                                          25

<210> 169
<211> 25
<212> DNA
<213> Artificial Sequence

<220>
<223> Synthetic oligonucleotide

<400> 169
caccgataaa aatccccttc gccga                                          25

<210> 170
<211> 25
<212> DNA
<213> Artificial Sequence

<220>
<223> Synthetic oligonucleotide

<400> 170
aaactcggcg aaggggattt ttatc                                          25

<210> 171
<211> 25
<212> DNA
<213> Artificial Sequence

<220>
<223> Synthetic oligonucleotide

<400> 171
caccgataca ttcccttcgg cgaag                                          25

<210> 172
<211> 25
<212> DNA
<213> Artificial Sequence

<220>
<223> Synthetic oligonucleotide

<400> 172
aaaccttcgc cgaagggaat gtatc                                              25

<210> 173
<211> 24
<212> DNA
<213> Artificial Sequence

<220>
<223> Synthetic oligonucleotide

<400> 173
caccgcatac attcccttcg gcga                                               24

<210> 174
<211> 24
<212> DNA
<213> Artificial Sequence

<220>
<223> Synthetic oligonucleotide

<400> 174
aaactcgccg aagggaatgt atgc                                               24

<210> 175
<211> 25
<212> DNA
<213> Artificial Sequence

<220>
<223> Synthetic oligonucleotide

<400> 175
caccgtaaaa atccccttcg ccgaa                                              25

<210> 176
<211> 25
<212> DNA
<213> Artificial Sequence

<220>
<223> Synthetic oligonucleotide

<400> 176
aaacttcggc gaaggggatt tttac                                          25

<210> 177
<211> 25
<212> DNA
<213> Artificial Sequence

<220>
<223> Synthetic oligonucleotide

<400> 177
caccgcaact gtcattgttg tgttt                                          25

<210> 178
<211> 25
<212> DNA
<213> Artificial Sequence

<220>
<223> Synthetic oligonucleotide

<400> 178
aaacaaacac aacaatgaca gttgc                                          25

<210> 179
<211> 25
<212> DNA
<213> Artificial Sequence

<220>
<223> Synthetic oligonucleotide

<400> 179
caccgcttcc gagatggttt aacaa                                          25

<210> 180
<211> 25
<212> DNA
<213> Artificial Sequence

<220>
<223> Synthetic oligonucleotide

<400> 180
aaacttgtta aaccatctcg gaagc                                          25

<210> 181
<211> 24
<212> DNA
<213> Artificial Sequence

<220>

<223> Synthetic oligonucleotide

<400> 181
caccgcaagc ttaagctatc ggac                                              24


<210> 182
<211> 24
<212> DNA
<213> Artificial Sequence


<220>
<223> Synthetic oligonucleotide

<400> 182
aaacgtccga tagcttaagc ttgc                                              24


<210> 183
<211> 24
<212> DNA
<213> Artificial Sequence


<220>
<223> Synthetic oligonucleotide

<400> 183
caccgagaaa ataatgaatg tcaa                                              24


<210> 184
<211> 24
<212> DNA
<213> Artificial Sequence


<220>
<223> Synthetic oligonucleotide

<400> 184
aaacttgaca ttcattattt tctc                                              24


<210> 185
<211> 24
<212> DNA
<213> Artificial Sequence


<220>
<223> Synthetic oligonucleotide

<400> 185
caccggcaac gacagactat gggc                                              24


<210> 186
<211> 24
<212> DNA
<213> Artificial Sequence

<220>
<223> Synthetic oligonucleotide

<400> 186
aaacgcccat agtctgtcgt tgcc                                              24


<210> 187
<211> 24
<212> DNA
<213> Artificial Sequence


<220>
<223> Synthetic oligonucleotide

<400> 187
caccgccaaa tcttcaggag atct                                              24


<210> 188
<211> 24
<212> DNA
<213> Artificial Sequence


<220>
<223> Synthetic oligonucleotide

<400> 188
aaacagatct cctgaagatt tggc                                              24


<210> 189
<211> 24
<212> DNA
<213> Artificial Sequence


<220>
<223> Synthetic oligonucleotide

<400> 189
caccggtgtt tgacccttgg ttaa                                              24


<210> 190
<211> 24
<212> DNA
<213> Artificial Sequence


<220>
<223> Synthetic oligonucleotide

<400> 190
aaacttaacc aagggtcaaa cacc                                              24


<210> 191
<211> 24
<212> DNA

<213> Artificial Sequence

<220>
<223> Synthetic oligonucleotide

<400> 191
caccgccatt cttctgaaat accc                                              24

<210> 192
<211> 24
<212> DNA
<213> Artificial Sequence

<220>
<223> Synthetic oligonucleotide

<400> 192
aaacgggtat ttcagaagaa tggc                                              24

<210> 193
<211> 24
<212> DNA
<213> Artificial Sequence

<220>
<223> Synthetic oligonucleotide

<400> 193
caccgactat gggcaggcaa gtga                                              24

<210> 194
<211> 24
<212> DNA
<213> Artificial Sequence

<220>
<223> Synthetic oligonucleotide

<400> 194
aaactcactt gcctgcccat agtc                                              24

<210> 195
<211> 24
<212> DNA
<213> Artificial Sequence

<220>
<223> Synthetic oligonucleotide

<400> 195
caccgatctg ggctctgaat ccca                                              24

<210> 196

<211> 24
<212> DNA
<213> Artificial Sequence

<220>
<223> Synthetic oligonucleotide

<400> 196
aaactgggat tcagagccca gatc                                              24

<210> 197
<211> 24
<212> DNA
<213> Artificial Sequence

<220>
<223> Synthetic oligonucleotide

<400> 197
caccgcccag atctcctgaa gatt                                              24

<210> 198
<211> 24
<212> DNA
<213> Artificial Sequence

<220>
<223> Synthetic oligonucleotide

<400> 198
aaacaatctt caggagatct gggc                                              24

<210> 199
<211> 24
<212> DNA
<213> Artificial Sequence

<220>
<223> Synthetic oligonucleotide

<400> 199
caccgagagg caacgacaga ctat                                              24

<210> 200
<211> 24
<212> DNA
<213> Artificial Sequence

<220>
<223> Synthetic oligonucleotide

<400> 200
aaacatagtc tgtcgttgcc tctc                                              24

<210> 201
<211> 25
<212> DNA
<213> Artificial Sequence

<220>
<223> Synthetic oligonucleotide

<400> 201
caccgttaac atggacaccg cgacc                                          25

<210> 202
<211> 25
<212> DNA
<213> Artificial Sequence

<220>
<223> Synthetic oligonucleotide

<400> 202
aaacggtcgc ggtgtccatg ttaac                                          25

<210> 203
<211> 25
<212> DNA
<213> Artificial Sequence

<220>
<223> Synthetic oligonucleotide

<400> 203
caccgacata ttcttgcatg ctcgc                                          25

<210> 204
<211> 25
<212> DNA
<213> Artificial Sequence

<220>
<223> Synthetic oligonucleotide

<400> 204
aaacgcgagc atgcaagaat atgtc                                          25

<210> 205
<211> 24
<212> DNA
<213> Artificial Sequence

<220>
<223> Synthetic oligonucleotide

<400> 205

caccgcccag ggccaagaat aaga                                          24

<210> 206
<211> 24
<212> DNA
<213> Artificial Sequence

<220>
<223> Synthetic oligonucleotide

<400> 206
aaactcttat tcttggccct gggc                                          24

<210> 207
<211> 24
<212> DNA
<213> Artificial Sequence

<220>
<223> Synthetic oligonucleotide

<400> 207
caccgccgga ctaggctgaa ggag                                          24

<210> 208
<211> 24
<212> DNA
<213> Artificial Sequence

<220>
<223> Synthetic oligonucleotide

<400> 208
aaacctcctt cagcctagtc cggc                                          24

<210> 209
<211> 24
<212> DNA
<213> Artificial Sequence

<220>
<223> Synthetic oligonucleotide

<400> 209
caccgggtct tgatatgttc ctca                                          24

<210> 210
<211> 24
<212> DNA
<213> Artificial Sequence

<220>
<223> Synthetic oligonucleotide

<400> 210
aaactgagga acatatcaag accc                                              24


<210> 211
<211> 24
<212> DNA
<213> Artificial Sequence


<220>
<223> Synthetic oligonucleotide

<400> 211
caccgattcc atcgtgggta aaga                                              24


<210> 212
<211> 24
<212> DNA
<213> Artificial Sequence


<220>
<223> Synthetic oligonucleotide

<400> 212
aaactcttta cccacgatgg aatc                                              24


<210> 213
<211> 24
<212> DNA
<213> Artificial Sequence


<220>
<223> Synthetic oligonucleotide

<400> 213
caccgtccgt cttattcttg gccc                                              24


<210> 214
<211> 24
<212> DNA
<213> Artificial Sequence


<220>
<223> Synthetic oligonucleotide

<400> 214
aaacgggcca agaataagac ggac                                              24


<210> 215
<211> 25
<212> DNA
<213> Artificial Sequence

<220>
<223> Synthetic oligonucleotide

<400> 215
caccgaacat tccggcagcc ggact                                          25


<210> 216
<211> 25
<212> DNA
<213> Artificial Sequence


<220>
<223> Synthetic oligonucleotide

<400> 216
aaacagtccg gctgccggaa tgttc                                          25


<210> 217
<211> 24
<212> DNA
<213> Artificial Sequence


<220>
<223> Synthetic oligonucleotide

<400> 217
caccgtctca ccagattcca tcgt                                           24


<210> 218
<211> 24
<212> DNA
<213> Artificial Sequence


<220>
<223> Synthetic oligonucleotide

<400> 218
aaacacgatg gaatctggtg agac                                           24


<210> 219
<211> 25
<212> DNA
<213> Artificial Sequence


<220>
<223> Synthetic oligonucleotide

<400> 219
caccgagctt caacattccg gcagc                                          25


<210> 220
<211> 25
<212> DNA
<213> Artificial Sequence

<220>
<223> Synthetic oligonucleotide

<400> 220
aaacgctgcc ggaatgttga agctc                                          25


<210> 221
<211> 25
<212> DNA
<213> Artificial Sequence


<220>
<223> Synthetic oligonucleotide

<400> 221
caccgcttca gcctagtccg gctgc                                          25


<210> 222
<211> 25
<212> DNA
<213> Artificial Sequence


<220>
<223> Synthetic oligonucleotide

<400> 222
aaacgcagcc ggactaggct gaagc                                          25


<210> 223
<211> 25
<212> DNA
<213> Artificial Sequence


<220>
<223> Synthetic oligonucleotide

<400> 223
caccgagcgt atatacctat tggtc                                          25


<210> 224
<211> 25
<212> DNA
<213> Artificial Sequence


<220>
<223> Synthetic oligonucleotide

<400> 224
aaacgaccaa taggtatata cgctc                                          25


<210> 225
<211> 25

<212> DNA
<213> Artificial Sequence

<220>
<223> Synthetic oligonucleotide

<400> 225
caccgtcgga agcgtatata cctat                                             25

<210> 226
<211> 25
<212> DNA
<213> Artificial Sequence

<220>
<223> Synthetic oligonucleotide

<400> 226
aaacataggt atatacgctt ccgac                                             25

<210> 227
<211> 24
<212> DNA
<213> Artificial Sequence

<220>
<223> Synthetic oligonucleotide

<400> 227
caccgcgtat atacctattg gtca                                              24

<210> 228
<211> 24
<212> DNA
<213> Artificial Sequence

<220>
<223> Synthetic oligonucleotide

<400> 228
aaactgacca ataggtatat acgc                                              24

<210> 229
<211> 24
<212> DNA
<213> Artificial Sequence

<220>
<223> Synthetic oligonucleotide

<400> 229
caccgatatg acactttcta tgta                                              24

<210> 230
<211> 24
<212> DNA
<213> Artificial Sequence

<220>
<223> Synthetic oligonucleotide

<400> 230
aaactacata gaaagtgtca tatc                                              24

<210> 231
<211> 25
<212> DNA
<213> Artificial Sequence

<220>
<223> Synthetic oligonucleotide

<400> 231
caccgtattc ttgcacgctc gcagg                                             25

<210> 232
<211> 25
<212> DNA
<213> Artificial Sequence

<220>
<223> Synthetic oligonucleotide

<400> 232
aaaccctgcg agcgtgcaag aatac                                             25

<210> 233
<211> 25
<212> DNA
<213> Artificial Sequence

<220>
<223> Synthetic oligonucleotide

<400> 233
caccgcggag ggttgatata cttca                                             25

<210> 234
<211> 25
<212> DNA
<213> Artificial Sequence

<220>
<223> Synthetic oligonucleotide

<400> 234
aaactgaagt atatcaaccc tccgc                                             25

<210> 235
<211> 25
<212> DNA
<213> Artificial Sequence

<220>
<223> Synthetic oligonucleotide

<400> 235
caccgtccct ccaaatacct aggct                                             25

<210> 236
<211> 25
<212> DNA
<213> Artificial Sequence

<220>
<223> Synthetic oligonucleotide

<400> 236
aaacagccta ggtatttgga gggac                                             25

<210> 237
<211> 24
<212> DNA
<213> Artificial Sequence

<220>
<223> Synthetic oligonucleotide

<400> 237
caccggctat tgaactgtgc gaga                                              24

<210> 238
<211> 24
<212> DNA
<213> Artificial Sequence

<220>
<223> Synthetic oligonucleotide

<400> 238
aaactctcgc acagttcaat agcc                                              24

<210> 239
<211> 25
<212> DNA
<213> Artificial Sequence

<220>
<223> Synthetic oligonucleotide

<400> 239
caccgaagtt gtcagtctta atcgc                                          25

<210> 240
<211> 25
<212> DNA
<213> Artificial Sequence

<220>
<223> Synthetic oligonucleotide

<400> 240
aaacgcgatt aagactgaca acttc                                          25

<210> 241
<211> 25
<212> DNA
<213> Artificial Sequence

<220>
<223> Synthetic oligonucleotide

<400> 241
caccgctatt gaactgtgcg agaag                                          25

<210> 242
<211> 25
<212> DNA
<213> Artificial Sequence

<220>
<223> Synthetic oligonucleotide

<400> 242
aaaccttctc gcacagttca atagc                                          25

<210> 243
<211> 24
<212> DNA
<213> Artificial Sequence

<220>
<223> Synthetic oligonucleotide

<400> 243
caccgctatt gaactgtgcg agaa                                           24

<210> 244
<211> 24
<212> DNA
<213> Artificial Sequence

<220>

<223> Synthetic oligonucleotide

<400> 244
aaacttctcg cacagttcaa tagc                                              24


<210> 245
<211> 25
<212> DNA
<213> Artificial Sequence


<220>
<223> Synthetic oligonucleotide

<400> 245
caccgtcagt cttaatcgca ggact                                             25


<210> 246
<211> 25
<212> DNA
<213> Artificial Sequence


<220>
<223> Synthetic oligonucleotide

<400> 246
aaacagtcct gcgattaaga ctgac                                             25